# EUROPEAN PATENT APPLICATION

(11) **EP 2 199 298 A1**
(43) Date of publication of application: **23.06.2010**
(21) Application number: 10000396.1
(22) Date of filing: 17.11.2005
(51) Int. Cl.: C07H 21/02, C12N 15/88, A61K 9/127, A61K 48/00, A61K 47/44

(54) **Sirna silencing of Apolipoprotein B**

(30) Priority: 17.11.2004 US 629808 P; 27.07.2005 US 703226 P
(62) Divisional of application: 05810655.0
(71) Applicant: Protiva Biotherapeutics Inc., Burnaby BC V5G 4Y1 (CA)
(72) Inventor: MacLachlan, Ian, Mission British Columbia V4S 1ES (CA); Jeffs, Lloyd B., Delta British Columbia V4K 3B4 (CA); Judge, Adam, Vancouver British Columbia V6J 2C6 (CA); Lee, Amy C.H., Burnaby British Columbia V3J 7Y2 (CA); Palmer, Lorne R., Vancouver British Columbia V5S 2P2 (CA); Sood, Vandana, Vancouver British Columbia V6E 1W5 (CA)
(74) Representative: Campbell, Patrick John Henry

(57) **Abstract**

The present invention provides nucleic acid-lipid particles comprising siRNA molecules that silence apoB expression and methods of using such nucleic acid-lipid particles to silence apoB expression.

## Description

### CROSS-REFERENCES TO RELATED APPLICATIONS

This application claims the benefit of U.S. Provisional Patent Applications Nos. 60/703,226, filed July 27, 2005 and 60/629,808 filed November 17, 2004, the disclosures of each of which are hereby incorporated by reference in their entirety for all purposes.

### BACKGROUND OF THE INVENTION

Apolipoprotein B (also known as ApoB, apolipoprotein B-100; ApoB-100, apolipoprotein B-48; ApoB-48 and Ag(x) antigen), is a large glycoprotein that serves an indispensable role in the assembly and secretion of lipids and in the transport and receptor-mediated uptake and delivery of distinct classes of lipoproteins. Apolipoprotein B was cloned (Law et al., PNAS USA 82:8340-8344 (1985)) and mapped to chromosome 2p23-2p24 in 1986 (Deeb et al., PNAS USA 83, 419-422 (1986)). ApoB has a variety of functions, from the absorption and processing of dietary lipids to the regulation of circulating lipoprotein levels (Davidson and Shelness, Annu. Rev. Nutr., 20:169-193(2000)). Two forms of ApoB have been characterized: ApoB-100 and ApoB-48. ApoB-100 is the major protein component of LDL, contains the domain required for interaction of this lipoprotein species with the LDL receptor, and participates in the transport and delivery of endogenous plasma cholesterol (Davidson and Shelness, 2000, *supra*). ApoB-48 circulates in association with chylomicrons and chylomicron remnants which are cleared the LDL-receptor-related protein (Davidson and Shelness, 2000, *supra*). ApoB-48 plays a role in the delivery of dietary lipid from the small intestine to the liver.

Susceptibility to atherosclerosis is highly correlated with the ambient concentration of apolipoprotein B-containing lipoproteins (Davidson and Shelness, 2000, *supra*). Elevated plasma levels of the ApoB-100-containing lipoprotein Lp(a) are associated with increased risk for atherosclerosis and its manifestations, which may include hypercholesterolemia (Seed et al., N. Engl. J. Med. 322:1494-1499 (1990), myocardial infarction (Sandkamp et al., Clin. Chem. 36:20-23 (1990), and thrombosis (Nowak-Gottl et al., Pediatrics, 99:E11 (1997)).

Apolipoprotein B knockout mice (bearing disruptions of both ApoB-100 and ApoB-48) have been generated which are protected from developing hypercholesterolemia when fed a high-fat diet (Farese et al., PNAS USA. 92:1774-1778 (1995) and Kim and Young, J. Lipid Res., 39:703-723 (1998)). The incidence of atherosclerosis has been investigated in mice expressing exclusively ApoB-100 or ApoB-48 and susceptibility to atherosclerosis was found to be dependent on total cholesterol levels.

Methods for modulating serum cholesterol using antibodies that specifically bind to ApoB are set forth in U.S. Patent Nos. 6,156,315; 6,309,844; and 6,096,516 and WO 99/18986. Small molecules that lower plasma concentrations of apolipoprotein B or apolipoprotein B-containing lipoproteins by stimulating a pathway for apolipoprotein B degradation are set forth in WO 01/30354. However, these compositions must be administered continuously to effectively modulate serum cholesterol (*i.e.,* by modulating ApoB). None of the compositions or methods described can specifically modulate serum cholesterol on a long term basis.

Thus, there is a need for compositions and methods for specifically modulating apolipoprotein B expression. The present invention addresses these and other needs.

### SUMMARY OF THE INVENTION

The present invention provides compositions comprising siRNA molecules that target ApoB expression and methods of using such compositions to silence ApoB gene expression. In some embodiments, the compositions can also be used to modulate (*i.e*., enhance or decrease) an immune response.

One embodiment of the present invention provides a nucleic acid-lipid particle that targets ApoB expression. The nucleic acid-lipid particle comprises an siRNA molecule that silences Apolipoprotein B (ApoB) expression; a cationic lipid; and a non-cationic lipid. The nucleic acid-lipid particle can further comprise a conjugated lipid that inhibits aggregation of particles. The nucleic acid-lipid particles comprse an siRNA molecule comprising a sequence set forth in Table 1, rows A-F of Table 2, Table 3, and Table 4. In some embodiments, the nucleic acid-lipid particles comprise at least 2, 3, 4, 5, or 6 or more siPNA molecules comprising the sequences set forth in Table 1, rows A-F of Table 2, and Tables 3-7.

The cationic lipid may be, *e.g.*, N,N-dioleyl-N,N-dimethylammonium chloride (DODAC), N,N-distearyl-N,N-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), and N,N-dimethyl-2,3-dioleyloxy)propylamine (DODMA), 1,2-DiLmoleyloxy-N,N-dimethylaminopropane (DLinDMA), and 1,2-Dilinolenyloxy-N,N-dimethylaminopropane (DLendMA), or mixtures thereof. The cationic lipid may comprise from about 2 mol % to about 60 mol %, about 5 mol % to about 45 mol %, about 5 mol % to about 15 mol %, about 30 mol % to about 50 mol %or about 40 mol % to about 50 mol % of the total lipid present in the particle.

The non-cationic lipid may be an anionic lipid or a neutral lipid including, but not limited to, dioleoylphosphatidylethanolamine (DOPE), palmitoyloleoylphosphatidylcholine (POPC), egg phosphatidylcholine (EPC), distearoylphosphatidylcholine (DSPC), palmitoyloleyolphosphatidylglycerol (POPG), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, palmitoyloleoylphosphatidylethanolamine (POPE), 1-stearoyl-2-oleoyl-phosphatidyethanolamine (SOPE), 1,2-dielaidoyl-sn-glycero-3-phophoethanolamine (transDOPE), cholesterol, or mixtures thereof. The non-cationic lipid comprises from about 5 mol % to about 90 mol % or about 20 mol % to about 85 mol % of the total lipid present in the particle.

The conjugated lipid that inhibits aggregation of particles may be a polyethyleneglycol (PEG)-lipid conjugate, a polyamide (ATTA)-lipid conjugate, a cationic-polymer-lipid conjugates (CPLs), or mixtures thereof. In one preferred embodiment, the nucleic acid-lipid particules comprise either a PEG-lipid conjugate or an ATTA-lipid conjugate together with a CPL. The conjugated lipid that inhibits aggregation of particles may comprise a polyethyleneglycol-lipid including, *e.g*., a PEG-diacylglycerol (DAG), a PEG dialkyloxypropyl (DAA), a PEG-phospholipid, a PEG-ceramide (Cer), or mixtures thereof. The PEG-DAA conjugate may be PEG-dilauryloxypropyl (C12), a PEG-dimyristyloxypropyl (C14), a PEG-dipalmityloxypropyl (C16), and a PEG-distearyloxypropyl (C18). In some embodiments, the conjugated lipid that inhibits aggregation of particles has the formula: A-W-Y, wherein: A is a lipid moiety; W is a hydrophilic polymer; and Y is a polycationic moiety. W may be a polymer selected from the group consisting of polyethyleneglycol (PEG), polyamide, polylactic acid, polyglycolic acid, polylactic acid/polyglycolic acid copolymers or combinations thereof, said polymer having a molecular weight of about 250 to about 7000 daltons. In some embodiments, Y has at least 4 positive charges at a selected pH. In some embodiments, Y may be lysine, arginine, asparagine, glutamine, derivatives thereof and combinations thereof. The conjugated lipid that prevents aggregation of particles may comprise from about 0 mol % to about 20 mol %, about 0.5 mol % to about 20 mol %, about 1 mol % to about 15 mol %, about 4 mol % to about 10 mol %, or about about 2 mol % of the total lipid present in said particle.

In some embodiments, the nucleic acid-lipid particle further comprises cholesterol at, *e.g*., about 0 mol % to about 10 mol %, about 2 mol % to about 10 mol %, about 10 mol % to about 60 mol % or about 20 mol % to about 45 mol % of the total lipid present in said particle.

In some embodiments, the siRNA in the nucleic acid-lipid particle is not substantially degraded after exposure of the particle to a nuclease at 37°C for at least 20, 30, 45, or 60 minutes; or after incubation of the particle in serum at 37°C for at least 30, 45, or 60 minutes.

In some embodiments, the siRNA is fully encapsulated in the nucleic acid-lipid particle. In some embodiments, the siRNA is complexed to the lipid portion of the particle.

The present invention further provides pharmaceutical compositions comprising the nucleic acid-lipid particles described herein and a pharmaceutically acceptable carrier.

The nucleic acid-lipid particles of the present invention are useful for the therapeutic delivery of nucleic acids comprising an interfering RNA sequence (*i.e*., an siRNA sequence that targets ApoB expression). In particular, it is an object of this invention to provide in *vitro* and *in vivo* methods for treatment of a disease in a mammal by downregulating or silencing the transcription and translation of a target nucleic acid sequence of interest. In these methods, an interfering RNA is formulated into a nucleic acid-lipid particle, and the particles are administered to patients requiring such treatment. Alternatively, cells are removed from a patient, the interfering RNA delivered *in vitro,* and reinjected into the patient. In one embodiment, the present invention provides for a method of introducing a nucleic acid into a cell by contacting a cell with a nucleic acid-lipid particle comprised of a cationic lipid, a non-cationic lipid, and an interfering RNA. The nucleic acid-lipid particle may further comprise a conjugated lipid that inhibits aggregation of the particles.

In one embodiment, at least 1%, 2%, 4%, 6%, 8%, or 10% of the total injected dose of the nucleic acid-lipid particles is present in plasma about 1, 2, 4, 6, 8, 12, 16,18; or 24 hours after injection. In other embodiments, more than about 20%, 30%, 40% and as much as 60%, 70% or 80% of the total injected dose of the nucleic acid-lipid particles is present in plasma about 1, 4, 6, 8, 10, 12, 20, or 24 hours after injection. In one embodiment, the effect of an interfering RNA (*e.g*., downregulation of the target sequence) at a site proximal or distal to the site of administration is detectable at about 12, 24, 48, 72, or 96 hours, or about 6, 8, 10, 12, 14, 16, 18, 19, 20, 22, 24, 26, or 28 days after administration of the nucleic acid-lipid particles. In one embodiment, downregulation of expression of the target sequence is detectable at about 12, 24, 48, 72, or 96 hours, or about 6, 8, 10, 12, 14, 16, 18, 19, 20, 22, 24, 26, or 28 days after administration. In some embodiments, downregulation of expression of an ApoB sequence is detected by measuring ApoB mRNA levels in a biological sample from the mammal. In some embodiments, downregulation of expression of an ApoB sequence is detected by measuring ApoB protein levels in a biological sample from the mammal. In some embodiments, downregulation of expression of an ApoB sequence is measured by measuring cholesterol levels in a biological sample from the mammal.

The particles are suitable for use in intravenous nucleic acid transfer as they are stable in circulation, of a size required for pharmacodynamic behavior resulting in access to extravascular sites and target cell populations. The particles are also suitable for subcutaneous and intraperitoneal administration. The invention also provides for pharmaceutically acceptable compositions comprising a nucleic acid-lipid particle.

Another embodiment of the present invention provides methods for *in vivo* delivery of interfering RNA (*e.g.*, an siRNA that silences expression of Apolipoprotein B). A nucleic acid-lipid particle comprising a cationic lipid, a non-cationic lipid, an interfering RNA, and optionally a conjugated lipid that inhibits aggregation of particles, and is administered (*e.g.*, intravenously, intraperitoneally, intramuscularly, or subcutaneously) to a subject (*e.g.*, a mammal such as a human or a rodent).

A further embodiment of the present invention provides a method of treating a disease or disorder in a mammalian subject. A therapeutically effective amount of a nucleic acid-lipid particle comprising a cationic lipid, a non-cationic lipid, a conjugated lipid that inhibits aggregation of particles, and interfering RNA (*e.g.*, an siRNA that silences expression of Apolipoprotein B) is administered to the mammalian subject (*e.g.*, a rodent such as a mouse, a primate such as a human or a monkey). In some embodiments, the disease or disorder is a in which ApoB is expressed or overexpressed and expression of ApoB is silenced by the siRNA. In some embodiments, the disease or disorder is atherosclerosis, angina pectoris, high blood pressure, diabetes, or hypothyroidism. In some embodiments, the disease or disorder involves hypercholesterolemia (*e.g.*, atherosclerosis, angina pectoris, or high blood pressure) and serum cholesterol levels are lowered when expression of ApoB is silenced by said siRNA.

One embodiment of the invention provides a modified siRNA that is capable of silencing expression of a target sequence (*i.e*., an ApoB sequence), comprising a double-stranded region of about 15 to about 30 nucleotides in length and a non-immunostimulatory mismatch motif consisting of a 5'-XX'-3 ' dinucleotide corresponding to a 5'-GU-3' dinucleotide in an unmodified siRNA sequence that is capable of silencing expression of the target sequence, wherein X and X' are independently selected from the group consisting of A, U, C, and G, with the proviso that if X is G, X' is not U and if X' is U, X is not G. The modified siRNA is less immunogenic than an siRNA that does not comprise the non-immunostimulatory mismatch motif. In some embodiments, the siRNA comprises one, two, three, or more additional immunostimulatory mismatch motifs relative to the target sequence. The immunostimulatory mismatch motifs may be adjacent to each other or, alternatively, they may be separated by 1, 2, 4, 6, 8, 10, or 12 or more nucleotides.

Another embodiment of the invention provides a modified siRNA that is capable of silencing expression of a target sequence (*i.e.,* an ApoB sequence) comprising a double stranded sequence of about 15 to about 30 nucleotides in length and an immunostimulatory mismatch motif consisting of a 5'-GU-3' dinucleotide corresponding to a 5'-XX'-3' dinucleotide motif in an unmodified siRNA that is capable of silencing expression of a target sequence, wherein X and X' are independently selected from the group consisting of A, U, C, and G, with the proviso that if X is G, X' is not U and if X' is U, X is not G. The modified siRNA is more immunogenic than an siRNA that does not comprise the immunostimulatory mismatch motif. In some embodiments, the siRNA comprises one, two, three, or more additional immunostimulatory mismatch motifs relative to the target sequence. The immunostimulatory mismatch motifs may be adjacent to each other or, alternatively, they may be separated by 1, 2, 4, 6, 8, 10, or 12 or more nucleotides.

In some embodiments, the siRNA described herein are used in methods of silencing expression of a target sequence and/or in methods of modulating (*i.e*., enhancing or reducing) immune responses associated with the siRNA. An effective amount of the siRNA is administered to a mammalian subject, thereby silencing expression of a target sequence (*i.e*., an ApoB sequence) or modulating an immune response associated with the siRNA.

The invention also provides pharmaceutical compositions comprising the siRNA molecules (*i.e*., the siRNA sequences that target APoB) described herein.

Yet another embodiment of the invention provides a method of identifying and modifying an siRNA having immunostimulatory properties. The method comprises (a) contacting an unmodified siRNA sequence with a mammalian responder cell under conditions suitable for the responder cell to produce a detectable immune response; (b) identifying the unmodified siRNA sequence as an immunostimulatory-siRNA by the presence of a detectable immune response in the responder cell; and (c) modifying the immunostimulatory siRNA by substituting at least one nucleotide with a modified nucleotide, thereby generating a modified siRNA sequence that is less immunostimulatory than the unmodified siRNA sequence.

In some embodiments, the modified siRNA comprises the modified siRNA contains at least one 2'-O-methyl (2'OMe) purine or pyrimidine nucleotide such as a 2'OMe-guanosine, 2'OMe-uridine, 2'OMe-adenosine nucleotide, and/or 2'OMe-cytosine nucleotide. In certain instances, the unmodified siRNA sequence comprises a 5'-GU-3' motif and at least one nucleotide in the 5'-GU-3' motif is substituted with a modified nucleotide. In one embodiment, the mammalian responder cell is a peripheral blood mononuclear cell (PBMC). In another embodiment, the detectable immune response comprises production of a cytokine or growth factor such as, for example, TNF-α, TNF-β, IFN-α, IFN-γ, IL-6, IL-12, or a combination thereof.

In another embodiment, the present invention provides isolated nucleic acid molecules comprising an siRNA sequence set forth in Table 1, rows A-F of Table 2, and Tables 3-7. The siRNA sequence can be modified or unmodified and can further include its complementary strand, thereby generating an siRNA duplex.

Other features, objects, and advantages of the invention and its preferred embodiments will become apparent from the detailed description, examples, and claims that follow.

### BRIEF DESCRIPTION OF THE DRAWINGS

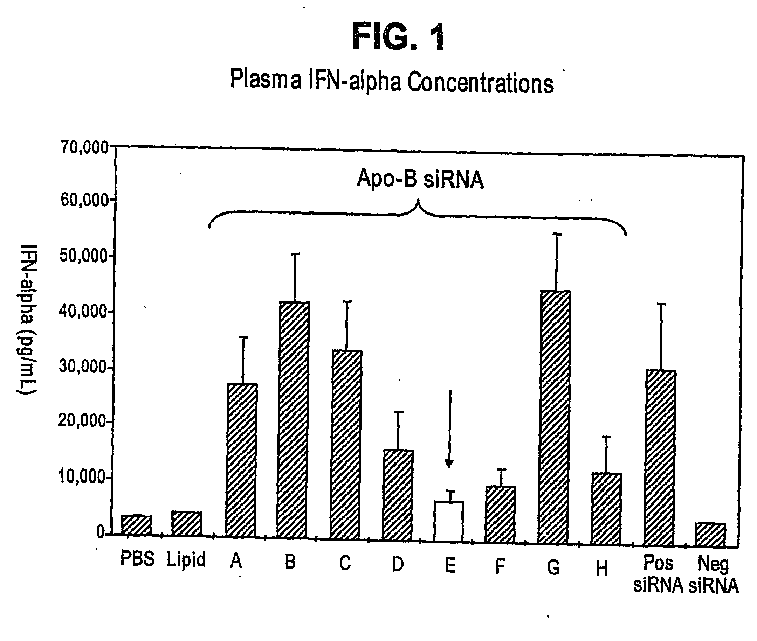

Figure 1 illustrates data showing plasma IFN-α levels six hours following administration of SNALP encapsulating siRNA targeting ApoB.

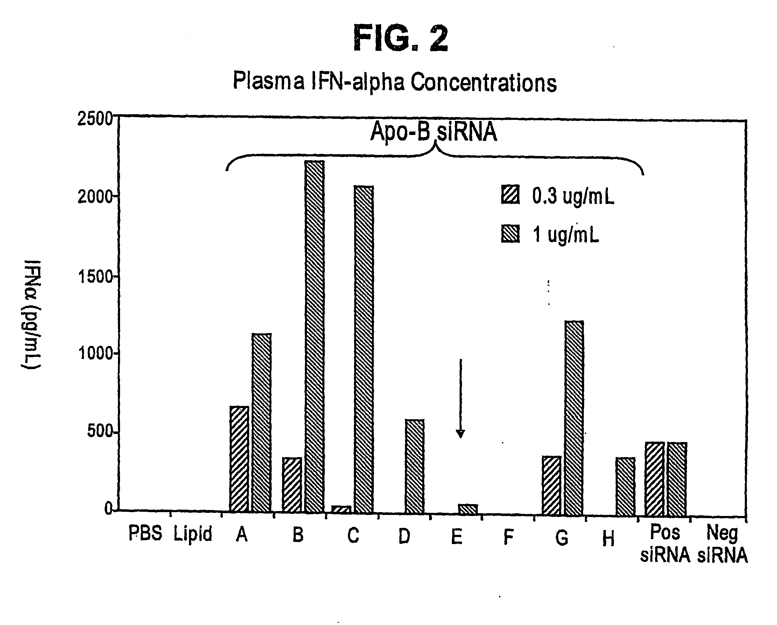

Figure 2 illustrates data showing IFN-α levels produced by human PBMC 24 hours following contacting the PBMC with SNALP encapsulating siRNA targeting ApoB.

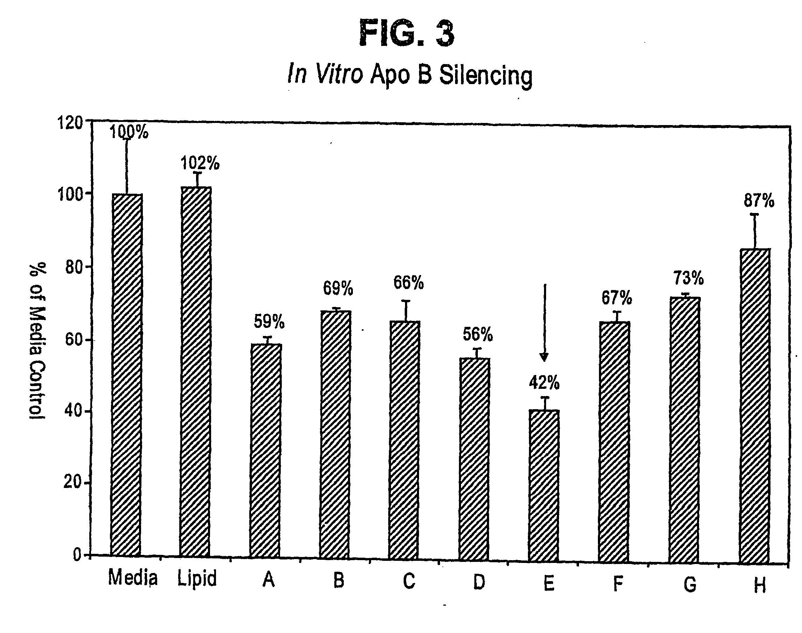

Figure 3 illustrates data showing *in vitro* silencing of ApoB in AML12 cells 40 hours after transfection with SNALP encapsulating siRNA targeting ApoB.

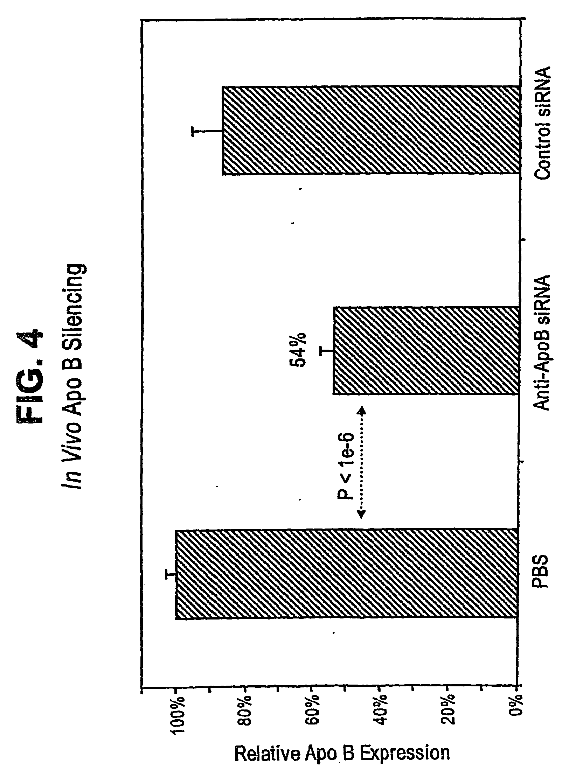

Figure 4 illustrates data showing *in vivo* silencing of ApoB in mice 48 hours following three once daily treatments of siRNA targeting ApoB (2.5 mg /kg).

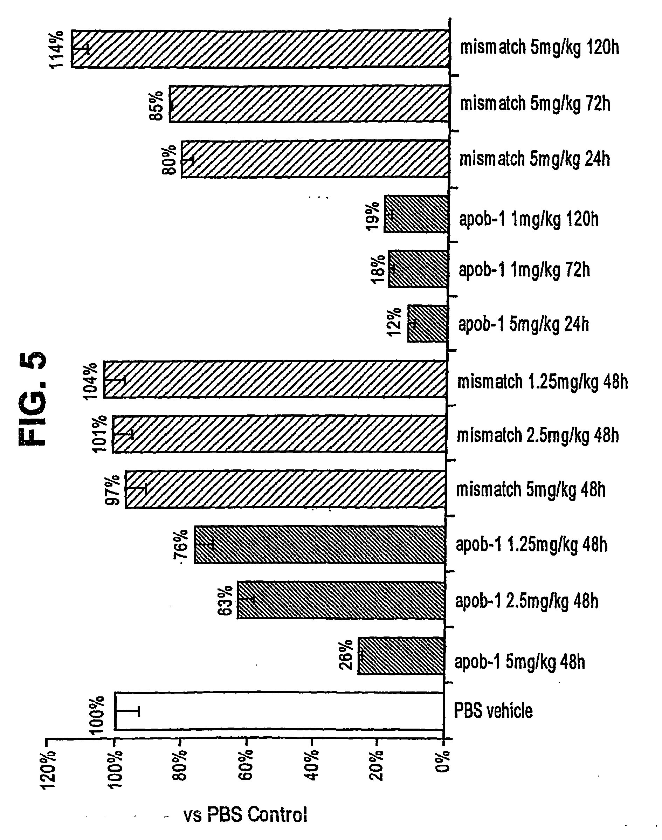

Figure 5 illustrates data showing ApoB silencing from multiple-dose IV administration of SNALP. Values describe measured ApoB:GAPDH mRNA ratios as a percentage of the ratio found in control PBS-treated liver. "Mismatch" is a shortform name of the siRNA apob-1-mismatch. Indicated dosages refer to siRNA amount per body weight. Indicated time points refer to time after the third and last daily SNALP injection. Each column represents the mean of 5 animals and error bars denote the standard error of the mean (SEM).

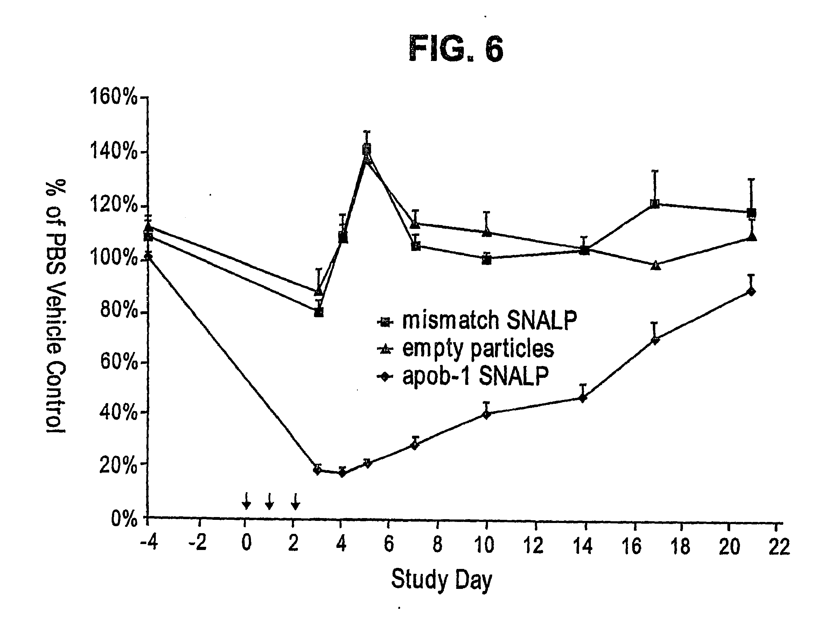

Figure 6 illustrates data showing an extended time course of ApoB silencing from multiple-dose IV administration of SNALP. Values describe measured plasma ApoB protein as a percentage of the concentration found in control PBS-treated blood. "Mismatch" is a shortform name of the siRNA apob-1-mismatch. Arrows indicate the three consecutive days of SNALP injection at a dosage of 5 mg siRNA per kg body eight. Each data point represents the mean of 5 animals and error bars denote SEM

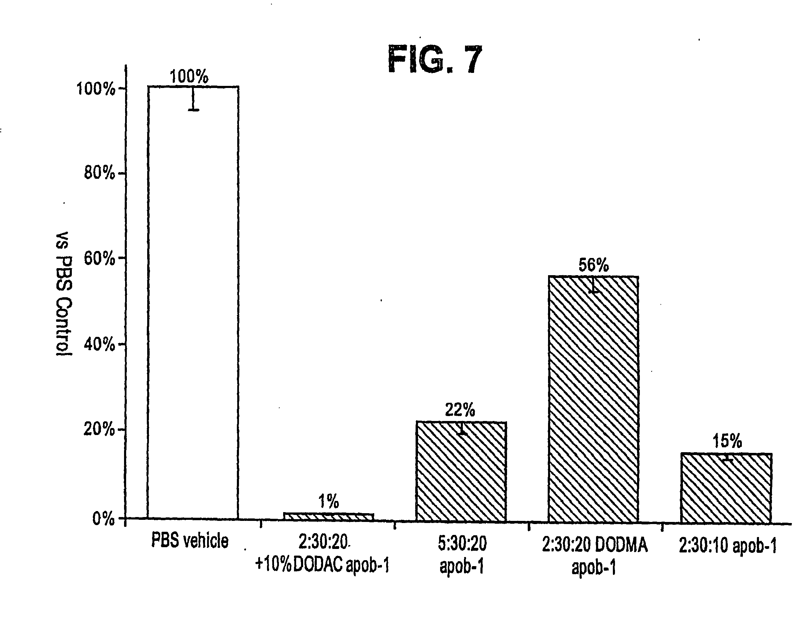

Figure 7 illustrates data showing ApoB silencing from multiple-dose IV administration of SNALP prepared via a Stepwise Dilution process. Values describe measured ApoB:GAPDH mRNA ratios as a percentage of the ratio found in control PBS-treated liver. Samples were collected 2 days after administration the third and last daily administration of SNALP at 5 mg siRNA per kg body weight. Each column represents the mean of 5 animals and error bars denote the standard error of the mean (SEM).

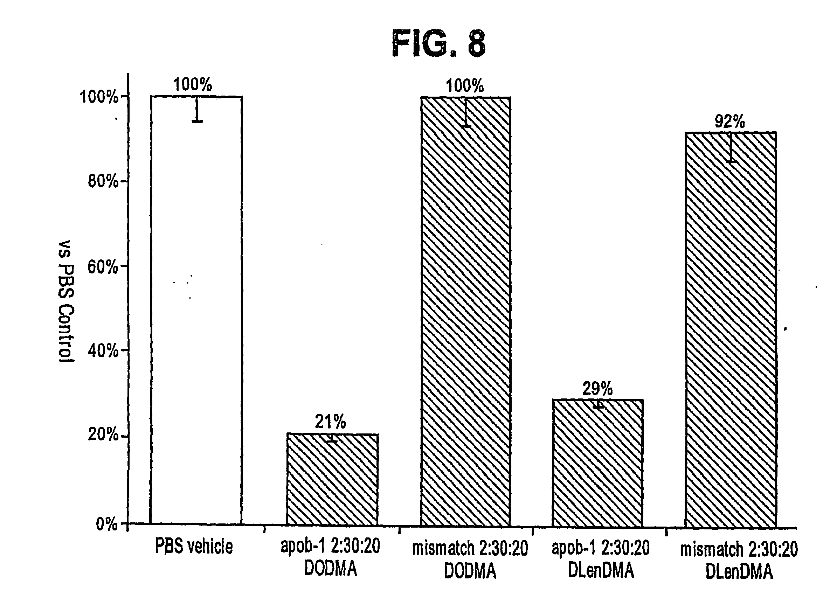

Figure 8 illustrates data showing ApoB silencing from multiple-dose IV administration of SNALP containing different cationic lipids. Values describe measured ApoB:GAPDH mRNA ratios as a percentage of the ratio found in control PBS-treated liver. "Mismatch" is a shortform name of the siRNA apob-1-mismatch. Samples were collected 2 days after administration the third and last daily administration of SNALP at 5 mg siRNA per kg body weight. Each column represents the mean of 5 animals and error bars denote the standard error of the mean (SEM).

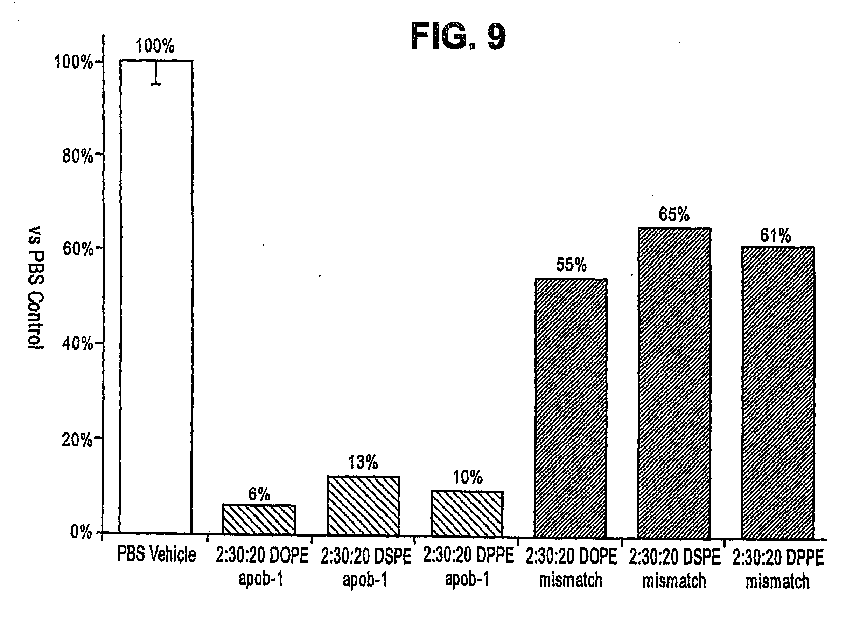

Figure 9 illustrates data showing ApoB silencing from multiple-dose IV administration of SNALP containing different phospholipids. Values describe measured ApoB:GAPDH mRNA ratios as a percentage of the ratio found in control PBS-treated liver. "Mismatch" is a shortform name of the siRNA apob-1-mismatch. Samples were collected 1 day after administration the third and last daily administration of SNALP at 3.5 mg siRNA per kg body weight. Each column represents the mean of 4 (for apob-1 SNALP, PBS) or 3 (for mismatch SNALP) animals and error bars denote the standard error of the mean (SEM).

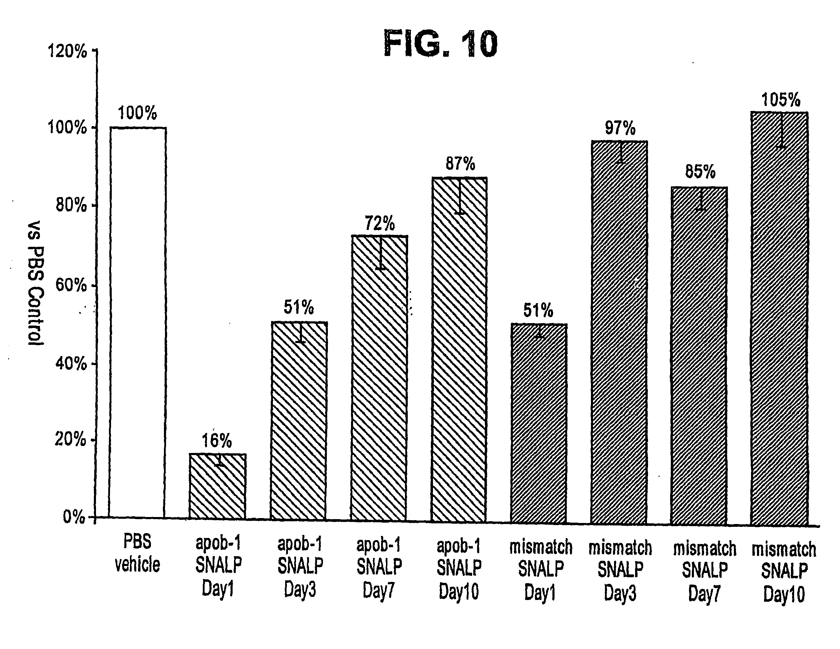

Figure 10 illustrates data showing a time course of ApoB silencing from single-dose IV administration of SNALP. Values describe measured plasma ApoB protein as a percentage of the concentration found in control PBS-treated blood. "Mismatch" is a shortform name of the siRNA apob-1-mismatch. On Study Day 0, animals were administered one SNALP injection at a dosage of 5 mg siRNA per kg body weight. Each data point represents the mean of 4 animals and error bars denote SEM.

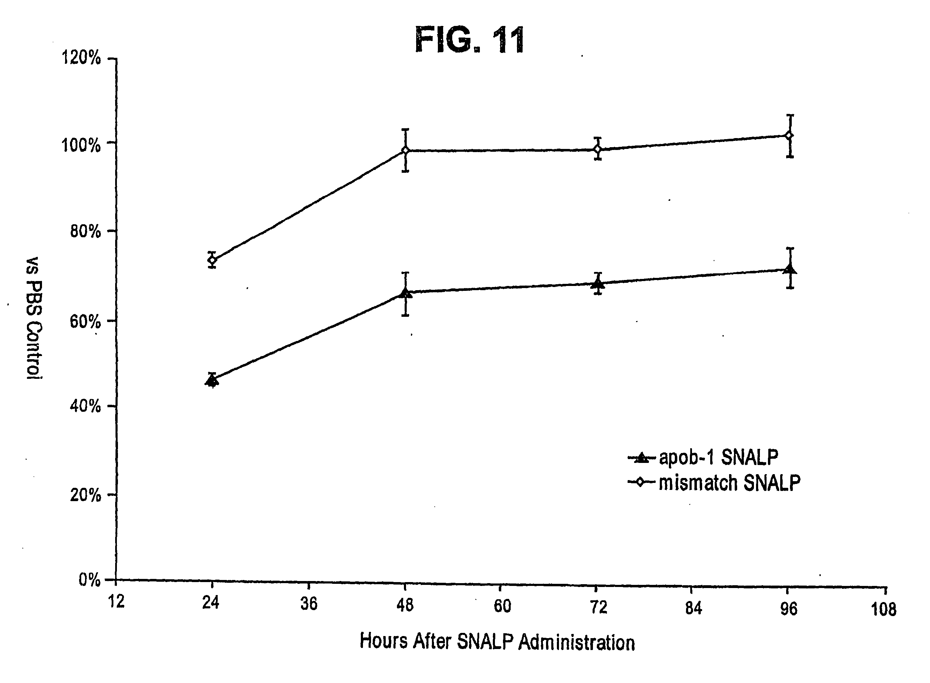

Figure 11 illustrates data showing a time course of ApoB silencing from single-dose IV administration of SNALP. Values describe measured plasma ApoB protein as a percentage of the concentration found in control PBS-treated blood. "Mismatch" is a shortform name of the siRNA apob-1-mismatch. On Study Day 0, animals were administered one SNALP injection at a dosage of 5 mg siRNA per kg body weight. Each data point represents the mean of 4 animals and error bars denote SEM.

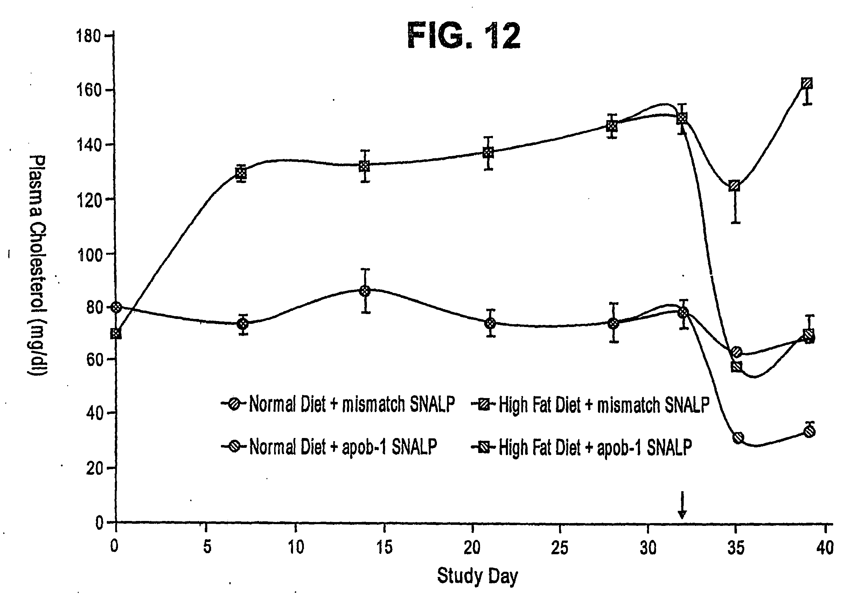

Figure 12 illustrates data showing the efficacy of anti-ApoB SNALP treatment in a hypercholesterolemia model. Total cholesterol concentration in female C57BL/6 mice was monitored in blood collected via tail nick. The red arrow indicates the day of IV SNALP administration at a dosage of 5 mg siRNA per kg body weight. Each data point between Day 0 and 32 (inclusive) represents the mean of 4 animals. Each data point from Day 35 onwards represents the mean of 2 animals. Error bars denote the standard error of the mean (SEM).

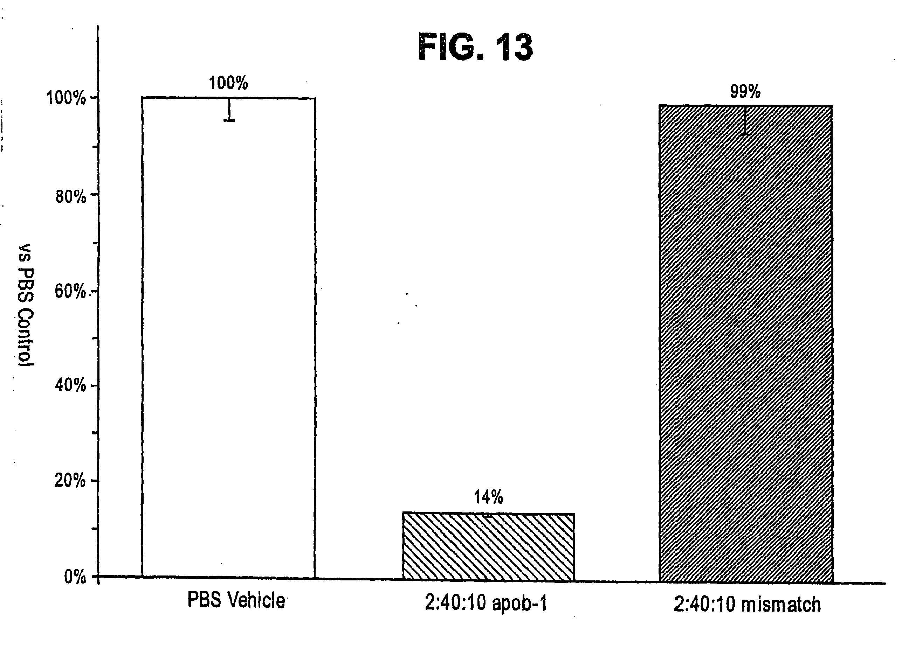

Figure 13 illustrates data showing ApoB silencing from single-dose IV administration of SNALP. Values describe measured ApoB:GAPDH mRNA ratios as a percentage of the ratio found in control PBS-treated liver. "Mismatch" is a shortform name of the siRNA apob-1-mismatch. Samples were collected four days after administration of SNALP at 2 mg siRNA per kg body weight. Each column represents the mean of 4 animals (except n=3 for mismatch) and error bars denote the standard error of the mean (SEM).

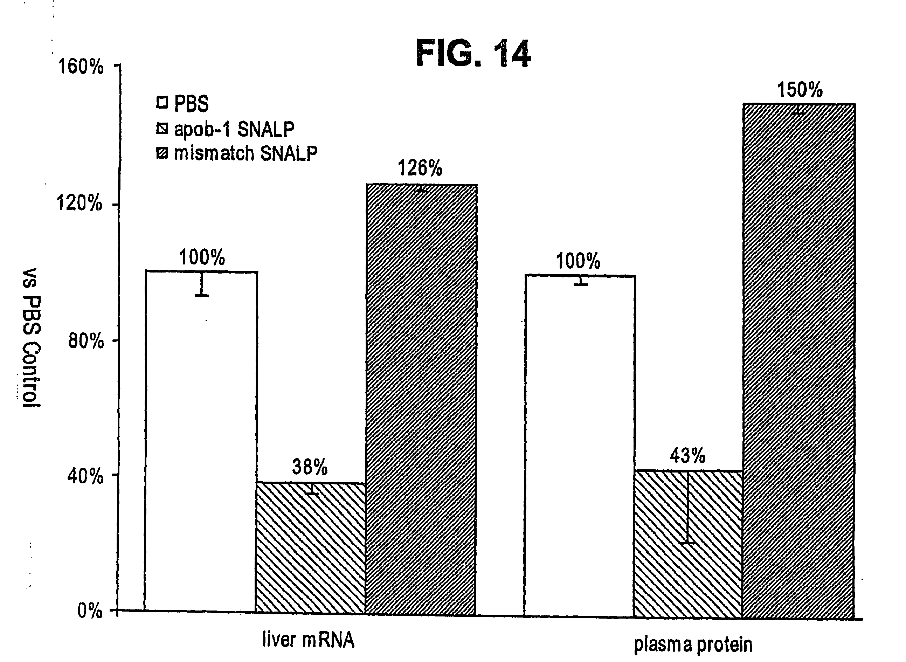

Figure 14 depicts data demonstrating *in vivo* silencing of ApoB expression following multi-dose intraperitoneal administration of SNALP encapsulating ApoB siRNA. 'Liver mRNA' values describe measured ApoB:GAPDH mRNA ratios as a percentage of the ratio found in control PBS-treated liver. 'Plasma protein' values describe ApoB protein as a percentage of the concentration found in control PBS-treated plasma. SNALP were administered to animals at 2 mg siRNA per kg body weight per injection, with injections on three consecutive days. Samples were collected 48 hours after the last administration of SNALP. Each column represents the mean of 4 animals (except 3 animals for mismatch SNALP) and error bars denote the standard error of the mean (SEM).

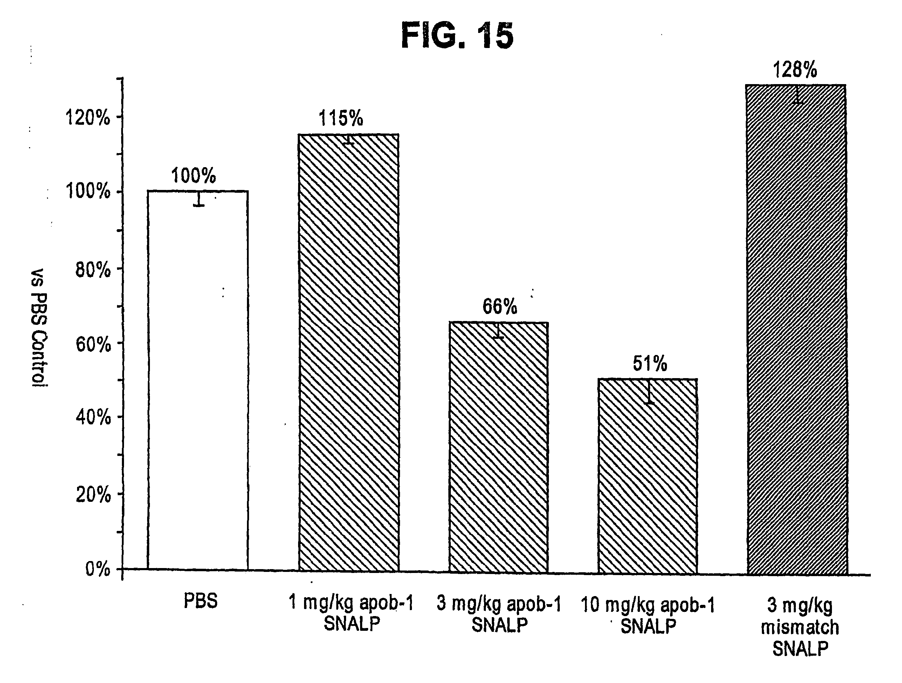

Figure 15 depicts data demonstrating *in vivo* silencing of ApoB gene expression following single-dose subcutaneous administration of SNALP encapsulating ApoB siRNA. Values describe measured ApoB:GAPDH mRNA ratios as a percentage of the ratio found in control PBS-treated liver. "mismatch" is a shortform name of the siRNA apob-1-mismatch. Samples were collected 48 hours after administration of SNALP at 1, 3 or 10 mg siRNA per kg body weight. Each column represents the mean of 4 animals and error bars denote the standard deviation (SD) of the mean.

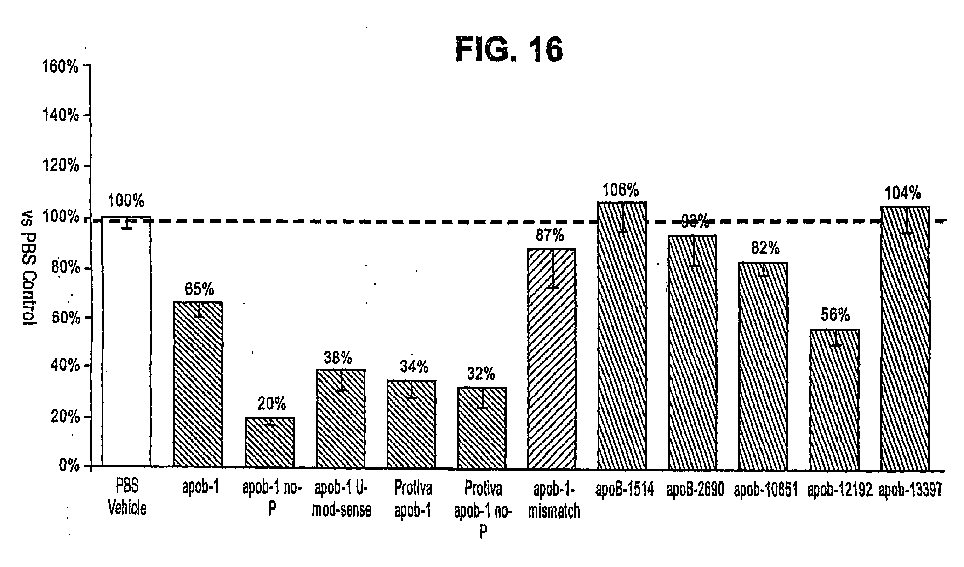

Figure 16 depicts data demonstrating *in vivo* silencing of ApoB gene expression following single-dose IV administration of a panel of SNALP encapsulating ApoB siRNA. Values describe measured ApoB:GAPDH mRNA ratios as a percentage of the ratio found in control PBS-treated liver. Each column represents the mean of 4 animals and error bars denote the standard deviation.

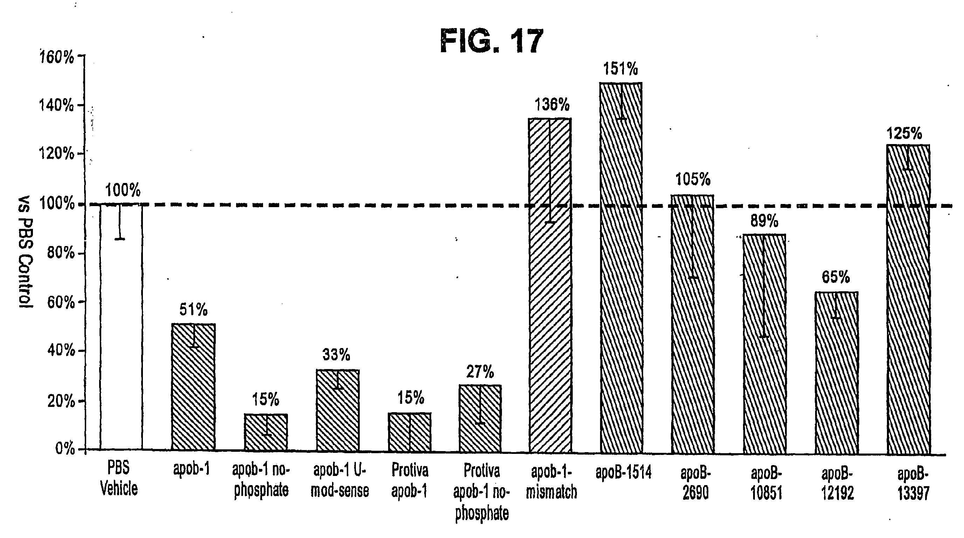

Figure 17 depicts data demonstrating *in vivo* silencing of ApoB gene expression following single-dose IV administration of a panel of SNALP encapsulating ApoB siRNA. Values describe measured apolipoprotein B protein levels in plasma a percentage of the apoB levels found in control PBS-treated plasma. Each column represents the mean of 4 animals and error bars denote the propagated standard deviation.

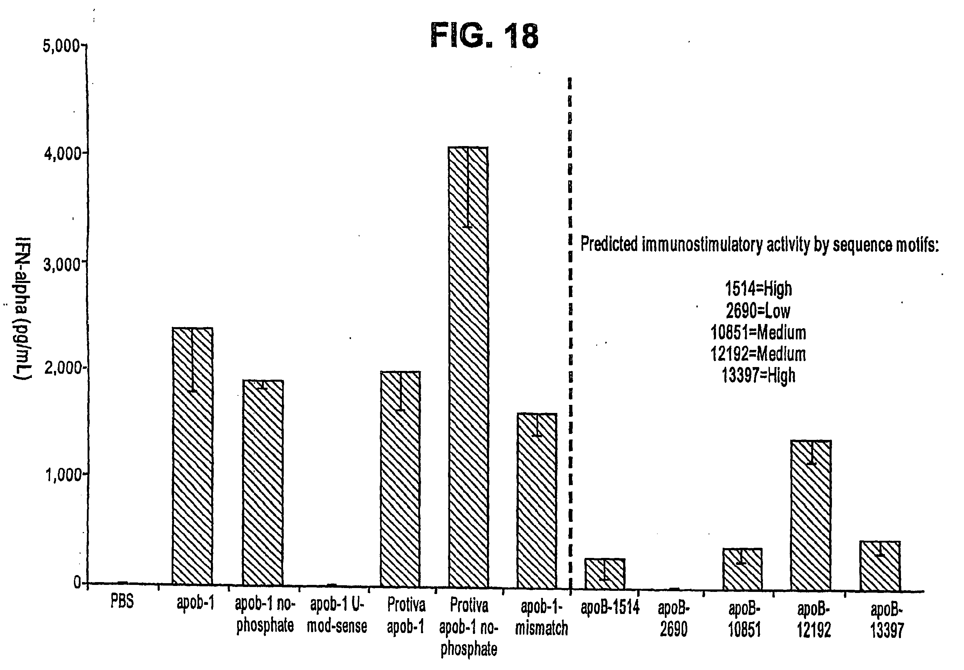

Figure 18 depicts data reflecting plasma interferon-α levels following single-dose IV administration of a panel of SNALP encapsulating ApoB siRNA. Values describe measured interferon-alpha levels in plasma at 6h after dosing. Each column represents the mean of 4 animals and error bars denote the standard deviation.

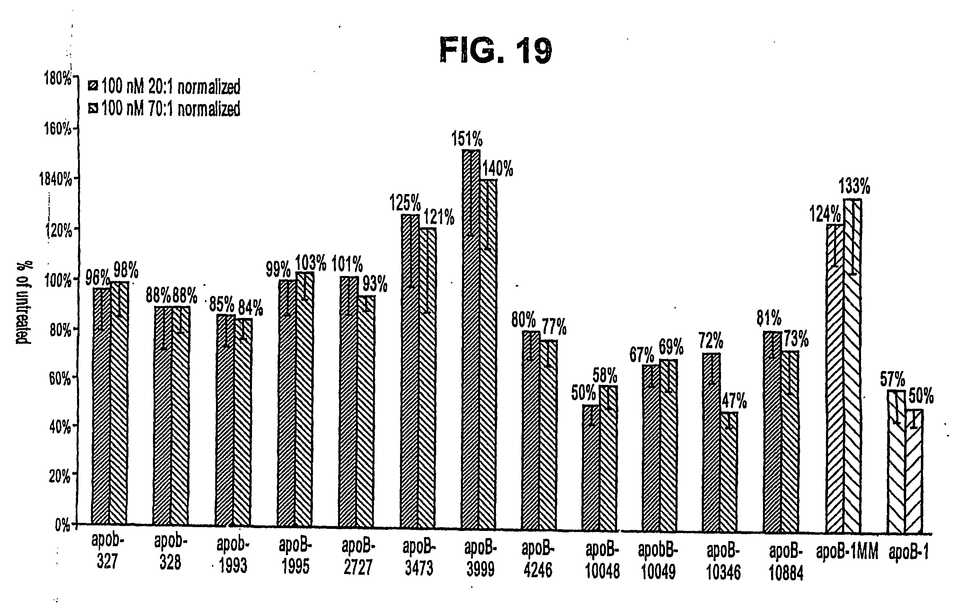

Figure 19 depicts data data demonstrating *in vivo* silencing of ApoB gene expression following single-dose IV administration of a panel of SNALP encapsulating ApoB siRNA. Relative ApoB silencing from 100 nM dosage of apoB siRNA. Values describe measured apolipoprotein B protein levels in HepG2 cell supernatants as a percentage of the apob levels found in untreated cell supernatants. Each column represents the mean of 3 replicates normalized to total protein levels in cell lysates, and error bars denote the propagated standard deviation.

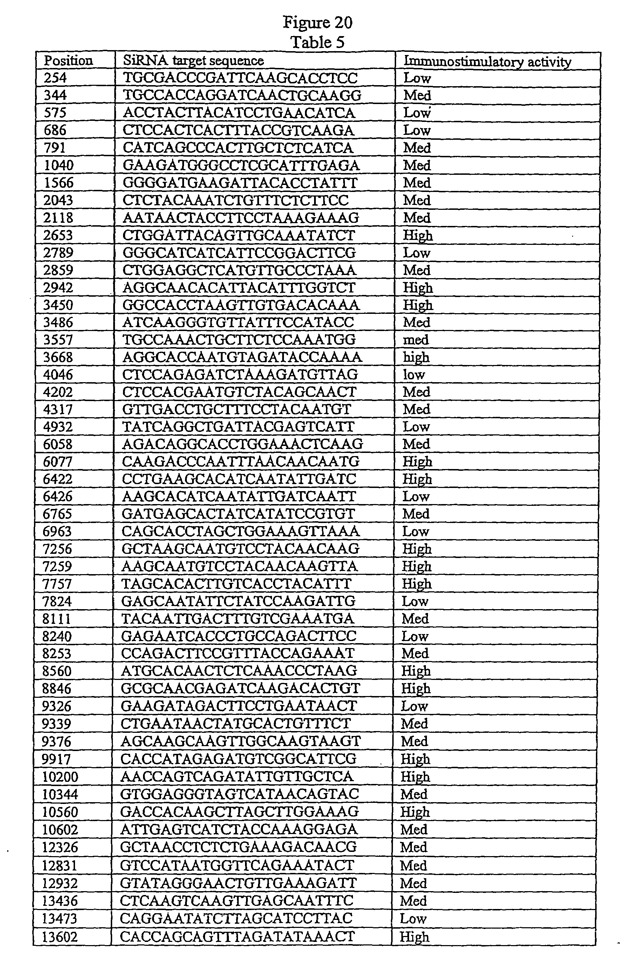

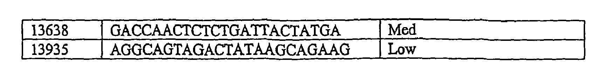

Figure 20 is Table 5 which sets forth siRNA sequences that target human ApoB and are derived from GenBank Accession No. NM_000384. The potential immunostimulatory activity of each siRNA is indicated.

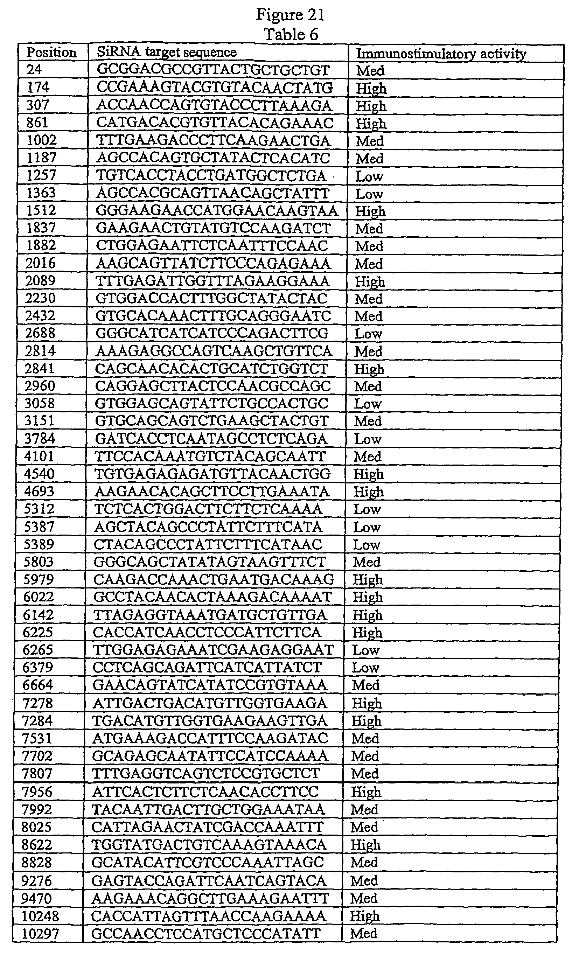

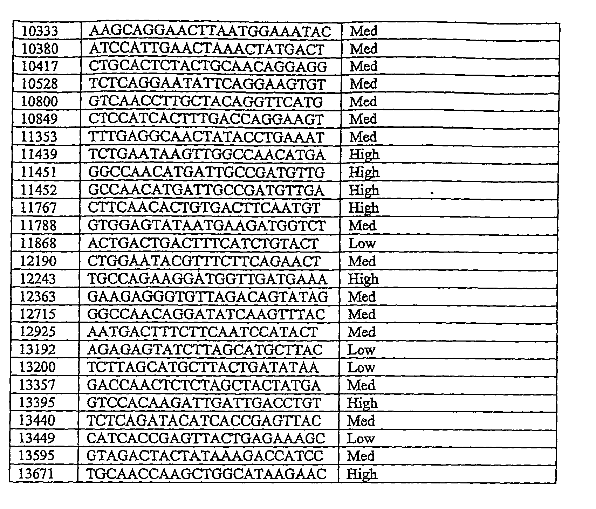

Figure 21 is Table 6 which sets forth siRNA sequences that target murine ApoB and are derived from GenBank Accession No. XM_137955. The potential immunostimulatory activity of each siRNA is indicated.

Figure 22 is Table 7 which sets forth additional siRNA sequences that target human ApoB and are derived from GenBank Accession No. NM_000384.

### DETAILED DESCRIPTION OF THE INVENTION

### I. Introduction

The present invention provides nucleic acid-lipid particles that target ApoB expression comprising an an siRNA that silences ApoB expression; a cationic lipid and a non-cationic lipid. In certain instances, the nucleic acid-lipid particle can further comprise a conjugated lipid that inhibits aggregation of particles. The siRNA sequence can be modified or unmodified.

In certain embodiments, the nucleic acid-lipid particles described herein ar particularly useful for silencing ApoB expression to treat diseases or disorders associated with expression or overexpression of ApoB. Such diseases include, *e.g.*, atherosclerosis, angina pectoris, high blood pressure, diabetes, hypothyroidism, and hypercholesterolemia. For example, administration of nucleic acid-lipid particles comprising the siRNA sequences described herein can be used to lower serum cholesterol levels.

One embodiment of the present invention is based on the surprising discovery that siRNA molecules have immunostimulatory effects that can be modulated.

Without being bound to any particular theory, it is postulated that the siRNA molecules' immunostimulatory activity is mediated by Toll-Like Receptor mediated signaling. These findings have significant implications for the clinical development of RNAi as a novel therapeutic approach and in the interpretation of specific gene silencing effects using siRNA. For example, immunostimulatory siRNA can be modified to disrupt a GU-rich (e.g., a 5'-GU-3', 5'-UGU-3', 5'-GUGU-3', or a 5'-UGUGU-3' motif), thus reducing their immunostimulatory properties while retaining their ability to silence a target gene (*i.e*., ApoB). The GU-rich motif may be disrupted by substitution of a nucleotide in the motif or by chemically modifying a nucleotide in the motif. Alternatively, the immunostimulatory siRNA can be used to generate controlled, transient cytokine production; activated T cell and NK cell proliferation, tumor-specific CTL responses, non-gene specific tumor regression, and B cell activation (*i.e.,* antibody production). In addition, non-immunostimulatory siRNA can be modified to to comprise a GU-rich motif, thus enhancing their immunostimulatory properties while retaining their ability to silence a target gene (*i.e*., ApoB).

### II. Definitions

The term "Apolipoprotein B" or "ApoB" refers to is the main apolipoprotein of chylomicrons and low density lipoproteins (LDL). Mutations in ApoB are associated with hypercholesterolemia. ApoB occurs in the plasma in 2 main forms: apoB48 and apoB 100 which are synthesized in the intestine and liver, respectively, due to an organ-specific stop codon. ApoB48 contains 2,152 residues compared to 4,535 residues in apoB100. Cloning and characterization of ApoB is described by *e.g*., Glickman et al., PNAS USA 83:5296-5300 (1986); Chen et al., . J. Biol. Chem. 261: 2918-12921 (1986); and Hospattankar et al., J. Biol. Chem. 261:9102-9104 (1986). ApoB sequences are set forth in, *e.g.*, Genbank Accession Nos. NM_000384 and BC051278. siRNA sequences that target ApoB are set forth in Tables 1-7 and in Soutschek et al., Nature 432:173-178 (2004).

The term "interfering RNA" or "RNAi" or "interfering RNA sequence" refers to double-stranded RNA (*i.e*., duplex RNA) that targets (*i.e.,* silences, reduces, or inhibits) expression of a target gene (*i.e*., by mediating the degradation of mRNAs which are complementary to the sequence of the interfering RNA) when the interfering RNA is in the same cell as the target gene. Interfering RNA thus refers to the double stranded RNA formed by two complementary strands or by a single, self-complementary strand. Interfering RNA typically has substantial or complete identity to the target gene. The sequence of the interfering RNA can correspond to the full length target gene, or a subsequence thereof.

Interfering RNA includes small-interfering RNA" or "siRNA," *i.e*., interfering RNA of about 15-60, 15-50, 15-50, or 15-40 (duplex) nucleotides in length, more typically about, 15-30, 15-25 or 19-25 (duplex) nucleotides in length, and is preferably about 20-24 or about 21-22 or 21-23 (duplex) nucleotides in length (*e.g.*, each complementary sequence of the double stranded siRNA is 15-60, 15-50, 15-50, 15-40, 15-30, 15-25 or 19-25 nucleotides in length, preferably about 20-24 or about 21-22 or 21-23 nucleotides in length, and the double stranded siRNA is about 15-60,15-50,15-50,15-40,15-30,15-25 or 19-25 preferably about 20-24 or about 21-22 or 21-23 base pairs in length). siRNA duplexes may comprise 3' overhangs of about 1 to about 4 nucleotides, preferably of about 2 to about 3 nucleotides and 5' phosphate termini. In some embodiments, the siRNA lacks a terminal phosphate. Examples of siRNA include, without limitation, a double-stranded polynucleotide molecule assembled from two separate oligonucleotides, wherein one strand is the sense strand and the other is the complementary antisense strand; a double-stranded polynucleotide molecule assembled from a single oligonucleotide, where the sense and antisense regions are linked by a nucleic acid-based or non-nucleic acid-based linker; a double-stranded polynucleotide molecule with a hairpin secondary structure having self-complementary sense and antisense regions; and a circular single-stranded polynucleotide molecule with two or more loop structures and a stem having self-complementary sense and antisense regions, where the circular polynucleotide can be processed *in vivo* or *in vitro* to generate an active double-stranded siRNA molecule.

The siRNA can be chemically synthesized or may be encoded by a plasmid (*e.g.*, transcribed as sequences that automatically fold into duplexes with hairpin loops). siRNA can also be generated by cleavage of longer dsRNA (*e.g.*, dsRNA greater than about 25 nucleotides in length) with the *E coli* RNase III or Dicer. These enzymes process the dsRNA into biologically active siRNA (*see, e.g.,* Yang et al., PNAS USA 99: 9942-7 (2002); Calegari et al., PNAS USA 99: 14236 (2002); Byrom et al., Ambion TechNotes 10(1): 4-6 (2003); Kawasaki et al., Nucleic Acids Res. 31: 981-7 (2003); Knight and Bass, Science 293: 2269-71 (2001); and Robertson et al., J. Biol. Chem. 243: 82 (1968)). Preferably, dsRNA are at least 50 nucleotides to about 100, 200, 300, 400 or 500 nucleotides in length. A dsRNA may be as long as 1000, 1500, 2000, 5000 nucleotides in length, or longer. The dsRNA can encode for an entire gene transcript or a partial gene transcript.

"Substantial identity" refers to a sequence that hybridizes to a reference sequence under stringent conditions, or to a sequence that has a specified percent identity over a specified region of a reference sequence.

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions may also be achieved with the addition of destabilizing agents such as formamide. For selective or specific hybridization, a positive signal is at least two times background, preferably 10 times background hybridization.

Exemplary stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C. For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec - 2 min., an annealing phase lasting 30 sec. - 2 min., and an extension phase of about 72°C for 1 - 2 min. Protocols and guidelines for low and high stringency amplification reactions are provided, *e.g.*, in Innis et al. (1990) PCR Protocols, A Guide to Methods and Applications, Academic Press, Inc. N.Y.).

Nucleic acids that do not hybridize to each other under stringent conditions are still substantially identical if the polypeptides which they encode are substantially identical. This occurs, for example, when a copy of a nucleic acid is created using the maximum codon degeneracy permitted by the genetic code. In such cases, the nucleic acids typically hybridize under moderately stringent hybridization conditions. Exemplary "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, *e.g.*, and Current Protocols in Molecular Biology, ed. Ausubel, *et al.*

The terms "substantially identical" or "substantial identity," in the context of two or more nucleic acids, refer to two or more sequences or subsequences that are the same or have a specified percentage of nucleotides that are the same (i.e., at least about 60%, preferably 65%, 70%, 75%, preferably 80%, 85%, 90%, or 95% identity over a specified region), when compared and aligned for maximum correspondence over a comparison window, or designated region as measured using one of the following sequence comparison algorithms or by manual alignment and visual inspection. This definition, when the context indicates, also refers analogously to the complement of a sequence. Preferably, the substantial identity exists over a region that is at least about 5, 10, 15, 20, 25, 30, 35, 40, 45, 50, 75, or 100 nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, *e.g.*, by the local homology algorithm of Smith & Waterman, *Adv. Appl. Math.* 2:482 (1981), by the homology alignment algorithm of Needleman & Wunsch, J. Mol. Biol. 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (*see* Henikoff & Henikoff, PNAS USA 89:10915 (1989)) alignments (B) of 50, expectation (E) of 10, M=5, N=-4, and a comparison of both strands.

The BLAST algorithm also performs a statistical analysis of the similarity between two sequences (*see, e.g.,* Karlin & Altschul, PNAS USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The phrase "inhibiting expression of a target gene" refers to the ability of a siRNA of the invention to silence, reduce, or inhibit expression of a target gene (*e.g.*, ApoB). To examine the extent of gene silencing, a test sample (*e.g*., a biological sample from organism of interest expressing the target gene or a sample of cells in culture expressing the target gene) is contacted with an siRNA that silences, reduces, or inhibits expression of the target gene. Expression of the target gene in the test sample is compared to expression of the target gene in a control sample (*e.g.*, a biological sample from organism of interest expressing the target gene or a sample of cells in culture expressing the target gene) that is not contacted with the siRNA. Control samples (*i.e*., samples expressing the target gene) are assigned a value of 100%. Silencing, inhibition, or reduction of expression of a target gene is achieved when the value of test the test sample relative to the control sample is about 95%, 90%, 85%, 80%, 75%, 70%, 65%, 60%, 55%, 50%, 45%, 40%, 35%, 30%, 25%, 20%, or 10%. Suitable assays include, *e.g.*, examination of protein or mRNA levels using techniques known to those of skill in the art such as dot blots, northern blots, in situ hybridization, ELISA, immunoprecipitation, enzyme function, as well as phenotypic assays known to those of skill in the art.

An "effective amount" or "therapeutically effective amount" of an siRNA is an amount sufficient to produce the desired effect, *e.g*., inhibition of expression of a target sequence in comparison to the normal expression level detected in the absence of the siRNA. Inhibition of expression of a target gene or target sequence is achieved when the value obtained with the siRNA relative to the control is about 90%, 80%, 70%, 60%, 50%, 40%, 30%, 25%, 20%, 15%, 10%, 5%, or 0%.

By "enhance," enhancement," or "enhancing" of an immune response by a siRNA is intended to mean a detectable enhancement of an immune response, typically measured by an increase in cytokine production (*e.g.,* IFNγ, IFNα, TNFα, IL-6, or IL-12) by a responder cell *in vitro* or an increase in cytokine production in the sera of a mammalian subject after administration of the siRNA. The amount of increase is determined relative to the normal level that is detected in the absence of the siRNA or other nucleic acid sequence. A detectable increase can be as small as about 5% or 10%, or as great as about 80%, 90% or 100%. More typically, a detectable increase is about 20%, 30%, 40%, 50%, 60%, or 70%.

By "decrease" or "decreasing" of an immune response by a siRNA is intended to mean a detectable decrease of an immune response, typically measured by an decrease in cytokine production (*e.g.,* IFNγ, IFNα, TNFα, IL-6, or IL-12) by a responder cell *in vitro* or an decrease in cytokine production in the sera of a mammalian subject after administration of the siRNA. The amount of decrease is determined relative to the normal level that is detected in the absence of the siRNA or other nucleic acid sequence. A detectable decrease can be as small as about 5% or 10%, or as great as about 80%, 90% or 100%. More typically, a detectable decrease is about 20%, 30%, 40%, 50%, 60%, or 70%.

As used herein, the term "responder cell" refers to a cell, preferably a mammalian cell that produces a detectable immune response when contacted with an immunostimulatory double stranded RNA. Exemplary responder cells include, *e.g.*, dendritic cells, macrophages, peripheral blood mononuclear cells ("PBMC"), splenocytes, and the like. Detectable immune responses include, *e.g*., production of cytokines such as IFN-α, IFN-γ, TNF-α, IL-1, IL-2, IL-3, Il-4, IL-5, IL-6, IL-10, IL-12, IL-13, and TGF.

The term "lipid" refers to a group of organic compounds that include, but are not limited to, esters of fatty acids and are characterized by being insoluble in water, but soluble in many organic solvents. They are usually divided into at least three classes: (1) "simple lipids' which include fats and oils as well as waxes; (2) "compound lipids" which include phospholipids and glycolipids; and (3) "derived lipids" such as steroids.

"Lipid vesicle" refers to any lipid composition that can be used to deliver a compound including, but not limited to, liposomes, wherein an aqueous volume is encapsulated by an amphipathic lipid bilayer; or wherein the lipids coat an interior comprising a large molecular component, such as a plasmid comprising an interfering RNA sequence, with a reduced aqueous interior; or lipid aggregates or micelles, wherein the encapsulated component is contained within a relatively disordered lipid mixture.

As used herein, "lipid encapsulated" can refer to a lipid formulation that provides a compound with full encapsulation, partial encapsulation, or both. In some embodiments, the nucleic acid is fully encapsulated in the lipid formulation (*e.g*., to form an SPLP, pSPLP, SNALP, or other nucleic acid-lipid particle).

The nucleic acid-lipid particles of the present invention typically have a mean diameter of less than about 150 nm and are substantially nontoxic. In addition, the nucleic acids when present in the nucleic acid-lipid particles of the present invention are resistant in aqueous solution to degradation with a nuclease. Nucleic acid-lipid particles and their method of preparation are disclosed in U.S. Patent Nos. 5,976,567 and 5,981,501 and PCT Patent Publication No. WO 96/40964.

Various suitable cationic lipids may be used in the present invention, either alone or in combination with one or more other cationic lipid species or non-cationic lipid species.

The cationic lipids of Formula I and Formula II described herein typically carry a net positive charge at a selected pH, such as physiological pH. It has been surprisingly found that cationic lipids comprising alkyl chains with multiple sites of unsaturation, *e.g*., at least two or three sites of unsaturation, are particularly useful for forming lipid-nucleic acid particles with increased membrane fluidity. A number of cationic lipids and related analogs, which are also useful in the present invention, have been described in co-pending USSN 08/316,399; U.S. Patent Nos. 5,208,036, 5,264,618, 5,279,833 and 5,283,185, and WO 96/10390.

The non-cationic lipids used in the present invention can be any of a variety of neutral uncharged, zwitterionic or anionic lipids capable of producing a stable complex. They are preferably neutral, although they can alternatively be positively or negatively charged. Examples of non-cationic lipids useful in the present invention include:
phospholipid-related materials, such as lecithin, phosphatidylethanolamine, lysolecithin, lysophosphatidylethanolamine, phosphatidylserine, phosphatidylinositol, sphingomyelin, cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidylcholine (POPC), palmitoyloleoyl- phosphatidylethanolamine (POPE) and dioleoyl- phosphatidylethanolamine 4-(N-maleimidomethyl)-cyclohexane-1-carboxylate (DOPE-mal). Non-cationic lipids or sterols such as cholesterol may be present. Additional nonphosphorous containing lipids are, *e.g*., stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide and the like, diacylphosphatidylcholine, diacylphosphatidylethanolamine, ceramide, sphingomyelin, cephalin, and cerebrosides. Other lipids such as lysophosphatidylcholine and lysophosphatidylethanolamine may be present. Non-cationic lipids also include polyethylene glycol-based polymers such as PEG 2000, PEG 5000 and polyethylene glycol conjugated to phospholipids or to ceramides (referred to as PEG-Cer), as described in co-pending USSN 08/316,429, incorporated herein by reference.

In preferred embodiments, the non-cationic lipids are diacylphosphatidylcholine *(e.g.*, distearoylphosphatidylcholine, dioleoylphosphatidylcholine, dipalmitoylphosphatidylcholine and dilinoleoylphosphatidylcholine), diacylphosphatidylethanolamine (*e.g*., dioleoylphosphatidylethanolamine and palmitoyloleoylphosphatidylethanolamine), ceramide or sphingomyelin. The acyl groups in these lipids are preferably acyl groups derived from fatty acids having C₁₀-C₂₄ carbon chains. More preferably the acyl groups are lauroyl, myristoyl, palmitoyl, stearoyl or oleoyl. In particularly preferred embodiments, the non-cationic lipid can be cholesterol, 1,2-*sn-*dioleoylphosphatidylethanolamine, or egg sphingomyelin (ESM).

In addition to cationic and non-cationic lipids, the nucleic acid-lipid particles (*e.g.,* SPLPs and SNALPs of the present invention can further comprise a bilayer stabilizing component (BSC) such as an ATTA-lipid or a PEG-lipid, such as PEG coupled to dialkyloxypropyls (PEG-DAA) (*see,* U.S. Patent Publication No. 2005017682), PEG coupled to diacylglycerol (PEG-DAG) (*see,* U.S. Patent Publication No. 2003077829), PEG coupled to phosphatidylethanolamine (PE) (PEG-PE), or PEG conjugated to ceramides, or a mixture thereof (see, U.S. Patent No. 5,885,613). In one preferred embodiment, the BSC is a conjugated lipid that inhibits aggregation of the nucleic acid-lipid particles. Suitable conjugated lipids include, but are not limited to PEG-lipid conjugates, ATTA-lipid conjugates, cationic-polymer-lipid conjugates (CPLs) or mixtures thereof. In one preferred embodiment, the nucleic acid-lipid particles comprise either a PEG-lipid conjugate or an ATTA-lipid conjugate together with a CPL.

PEG is a polyethylene glycol, a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, for example, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), and monomethoxypolyethylene glycol-imidazolyl-carbonyl (MePEG-IM). In addition, monomethoxypolyethyleneglycol-acetic acid (MePEG-CH₂COOH), is particularly useful for preparing the PEG-lipid conjugates including, *e.g.*, PEG-DAA conjugates.

In some embodiments, the PEG has an average molecular weight of from about 1000 to about 5000 daltons, more preferably, from about 1,000 to about 3,000 daltons and, even more preferably, of about 2,000 daltons. The PEG can be optionally substituted by an alkyl, alkoxy, acyl or aryl group. PEG can be conjugated directly to the lipid or may be linked to the lipid via a linker moiety. Any linker moiety suitable for coupling the PEG to a lipid can be used including, *e.g*., non-ester containing linker moieties and ester-containing linker moieties.

As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, etc. as well as combinations thereof (such as a linker containing both a carbamate linker moiety and an amido linker moiety). In some embodiments, a carbamate linker is used to couple the PEG to the lipid.

In other embodiments, an ester containing linker moiety is used to couple the PEG to the lipid. Suitable ester containing linker moieties include, *e.g.*, carbonate (-OC(O)O-), succinoyl, phosphate esters (-O-(O)POH-O-), sulfonate esters, and combinations thereof.

As used herein, the term "SNALP" refers to a stable nucleic acid lipid particle, including SPLP. A SNALP represents a vesicle of lipids coating a reduced aqueous interior comprising a nucleic acid (*e.g*., ssDNA, dsDNA, ssRNA, dsRNA, siRNA, or a plasmid, including plasmids from which an interfering RNA is transcribed). As used herein, the term "SPLP" refers to a nucleic acid lipid particle comprising a nucleic acid (*e.g*., a plasmid) encapsulated within a lipid vesicle. SNALPs and SPLPs typically contain a cationic lipid, a non-cationic lipid, and a lipid that prevents aggregation of the particle (*e.g.,* a PEG-lipid conjugate). SNALPs and SPLPs have systemic application as they exhibit extended circulation lifetimes following intravenous (i.v.) injection, accumulate at distal sites (*e.g.,* sites physically separated from the administration site and can mediate expression of the transfected gene at these distal sites. SPLPs include "pSPLP" which comprise an encapsulated condensing agent-nucleic acid complex as set forth in WO 00/03683.

The term "vesicle-forming lipid" is intended to include any amphipathic lipid having a hydrophobic moiety and a polar head group, and which by itself can form spontaneously into bilayer vesicles in water, as exemplified by most phospholipids.

The term "vesicle-adopting lipid" is intended to include any amphipathic lipid that is stably incorporated into lipid bilayers in combination with other amphipathic lipids, with its hydrophobic moiety in contact with the interior, hydrophobic region of the bilayer membrane, and its polar head group moiety oriented toward the exterior, polar surface of the membrane. Vesicle-adopting lipids include lipids that on their own tend to adopt a nonlamellar phase, yet which are capable of assuming a bilayer structure in the presence of a bilayer-stabilizing component. A typical example is DOPE (dioleoylphosphatidylethanolamine). Bilayer stabilizing components include, but are not limited to, conjugated lipids that inhibit aggregation of the SNALPs, polyamide oligomers (*e.g*., ATTA-lipid derivatives), peptides, proteins, detergents, lipid-derivatives, PEG-lipid derivatives such as PEG coupled to dialkyloxypropyls, PEG coupled to diacylglycerols, PEG coupled to phosphatidylethanolamines, and PEG conjugated to ceramides (see, U.S. Pat. No. 5,885,613).

The term "amphipathic lipid" refers, in part, to any suitable material wherein the hydrophobic portion of the lipid material orients into a hydrophobic phase, while the hydrophilic portion orients toward the aqueous phase. Amphipathic lipids are usually the major component of a lipid vesicle. Hydrophilic characteristics derive from the presence of polar or charged groups such as carbohydrates, phosphate, carboxylic, sulfato, amino, sulfhydryl, nitro, hydroxyl and other like groups. Hydrophobicity can be conferred by the inclusion of apolar groups that include, but are not limited to, long chain saturated and unsaturated aliphatic hydrocarbon groups and such groups substituted by one or more aromatic, cycloaliphatic or heterocyclic group(s). Examples of amphipathic compounds include, but are not limited to, phospholipids, aminolipids and sphingolipids. Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine and dilinoleoylphosphatidylcholine. Other compounds lacking in phosphorus, such as sphingolipid, glycosphingolipid families, diacylglycerols and β-acyloxyacids, are also within the group designated as amphipathic lipids. Additionally, the amphipathic lipid described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides and diacylglycerols.

The term "non-cationic lipid" refers to any neutral lipid as described above as well as anionic lipids.

The term "anionic lipid" refers to any lipid that is negatively charged at physiological pH. These lipids include, but are not limited to, phosphatidylglycerol, cardiolipin, diacylphosphatidylserine, diacylphosphatidic acid, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

The term "cationic lipid" refers to any of a number of lipid species that carry a net positive charge at a selected pH, such as physiological pH (*e.g.,* pH of about 7.0). As used herein, physiological pH refers to the pH of a biological fluid such as blood or lymph as well as the pH of a cellular compartment such as an endosome, an acidic endosome, or a lysosome). Such lipids include, but are not limited to, N,N-dioleyl-N,N-dimethylammonium chloride ("DODAC"); N-(1-(2,3-dioleyloxy)propyl)-N,N,N-trimethylammonium chloride ("DOTMA"); N,N-dimethyl-(2,3-dioleloxy)propylamine ("DODMA"); N,N-distearyl-N,N-dimethylammonium bromide ("DDAB "); N-(2,3-dioleoyloxy)propyl)-N,N,N-trimethylammonium chloride ("DOTAP"); 3 -(N-(N',N'-dimethylaminoethane)-carbamoyl)cholesterol ("DC-Chol"); N-(1,2-dimyristyloxyprop-3-yl)-N,N-dimethyl-N-hydroxyethyl ammonium bromide ("DMRIE"); 1,2-Dilinoleyloxy-N,N-dimethylaminopropane (DLinDMA); and 1,2-Dilinolenyloxy-N,N-dimethylamminopropane (DLenDMA). The following lipids are cationic and have a positive charge at below physiological pH: DODAP, DODMA, DMDMA and the like.

The term "hydrophobic lipid" refers to compounds having apolar groups that include, but are not limited to, long chain saturated and unsaturated aliphatic hydrocarbon groups and such groups optionally substituted by one or more aromatic, cycloaliphatic or heterocyclic group(s). Suitable examples include, but are not limited to, diacylglycerol, dialkylglycerol, N-N-dialkylamino, 1,2-diacyloxy-3-aminopropane and 1,2-dialkyl-3-aminopropane.

The term "fusogenic" refers to the ability of a liposome, an SNALP or other drug delivery system to fuse with membranes of a cell. The membranes can be either the plasma membrane or membranes surrounding organelles, *e.g*., endosome, nucleus, etc.

The term "diacylglycerol" refers to a compound having 2-fatty acyl chains, R¹ and R², both of which have independently between 2 and 30 carbons bonded to the 1- and 2-position of glycerol by ester linkages. The acyl groups can be saturated or have varying degrees of unsaturation. Diacylglycerols have the following general formula:

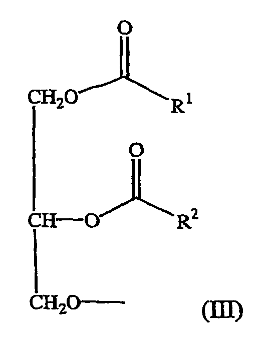

The term "dialkyloxypropyl" refers to a compound having 2-alkyl chains, R¹ and R², both of which have independently between 2 and 30 carbons. The alkyl groups can be saturated or have varying degrees of unsaturation. Dialkyloxypropyls have the following general formula:

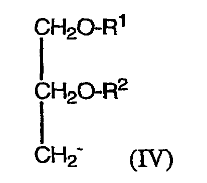

The term "ATTA" or "polyamide" refers to, but is not limited to, compounds disclosed in U.S. Patent Nos. 6,320,017 and 6,586,559, both of which are incorporated herein by reference. These compounds include a compound having the formula 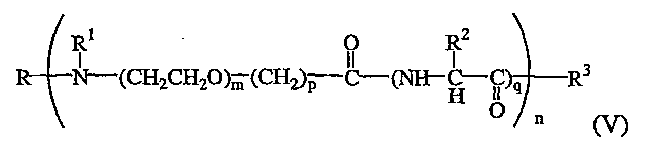 wherein: R is a member selected from the group consisting of hydrogen, alkyl and acyl; R¹ is a member selected from the group consisting of hydrogen and alkyl; or optionally, R and R¹ and the nitrogen to which they are bound form an azido moiety; R² is a member of the group selected from hydrogen, optionally substituted alkyl, optionally substituted aryl and a side chain of an amino acid; R³ is a member selected from the group consisting of hydrogen, halogen, hydroxy, alkoxy, mercapto, hydrazino, amino and NR⁴R⁵, wherein R⁴ and R⁵ are independently hydrogen or alkyl; n is 4 to 80; m is 2 to 6; p is 1 to 4; and q is 0 or 1. It will be apparent to those of skill in the art that other polyamides can be used in the compounds of the present invention.

The term "nucleic acid" or "polynucleotide" refers to a polymer containing at least two deoxyribonucleotides or ribonucleotides in either single- or double-stranded form. Unless specifically limited, the terms encompass nucleic acids containing known analogs of natural nucleotides that have similar binding properties as the reference nucleic acid and are metabolized in a manner similar to naturally occurring nucleotides. Examples of such analogs include, without limitation phosphorothioates, phosphoramidates, methyl phosphonates, chiral-methyl phosphonates, 2')-methyl ribonucleotides, and peptide nucleic acids (PNA's). Unless otherwise indicated, a particular nucleic acid sequence also implicitly encompasses conservatively modified variants thereof (*e.g.*, degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (B atzer et al., Nucleic Acid Res. 19:5081 (1991); Ohtsuka et al., J. Biol. Chem. 260:2605-2608 (1985); and Cassol *et al.* (1992);. Rossolini et al., Mol. Cell. Probes 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides. DNA may be in the form of antisense, plasmid DNA, parts of a plasmid DNA, pre-condensed DNA, product of a polymerase chain reaction (PCR), vectors (P1, PAC, BAC, YAC, artificial chromosomes), expression cassettes, chimeric sequences, chromosomal DNA, or derivatives of these groups. The term nucleic acid is used interchangeably with gene, cDNA, mRNA encoded by a gene, and an interfering RNA molecule.

The term "gene" refers to a nucleic acid (*e.g.*, DNA or RNA) sequence that comprises partial length or entire length coding sequences necessary for the production of a polypeptide or precursor (*e.g*., ApoB).

"Gene product," as used herein, refers to a product of a gene such as an RNA transcript.

As used herein, the term "aqueous solution" refers to a composition comprising in whole, or in part, water.

As used herein, the term "organic lipid solution" refers to a composition comprising in whole, or in part, an organic solvent having a lipid.

"Distal site," as used herein, refers to a physically separated site, which is not limited to an adjacent capillary bed, but includes sites broadly distributed throughout an organism.

"Serum-stable" in relation to nucleic acid-lipid particles means that the particle is not significantly degraded after exposure to a serum or nuclease assay that would significantly degrade free DNA. Suitable assays include, for example, a standard serum assay or a DNAse assay such as those described in the Examples below.

"Systemic delivery," as used herein, refers to delivery that leads to a broad biodistribution of a compound within an organism. Some techniques of administration can lead to the systemic delivery of certain compounds, but not others. Systemic delivery means that a useful, preferably therapeutic, amount of a compound is exposed to most parts of the body. To obtain broad biodistribution generally requires a blood lifetime such that the compound is not rapidly degraded or cleared (such as by first pass organs (liver, lung, etc.) or by rapid, nonspecific cell binding) before reaching a disease site distal to the site of administration. Systemic delivery of nucleic acid-lipid particules can be by any means known in the art including, for example, intravenous, subcutaneous, intraperitoneal, In some embodiments, systemic delivery of nucleic acid-lipid particles is by intravenous delivery.

"Local delivery" as used herein refers to delivery of a compound directly to a target site within an organism. For example, a compound can be locally delivered by direct injection into a disease site such as a tumor or other target site such as a site of inflammation or a target organ such as the liver, heart, pancreas, kidney, and the like.

### III. siRNAs

The nucleic acid component of the nucleic acid-lipid particles of the present invention comprises an interfering RNA that silences (*e.g.*, partially or completely inhibits) expression of a gene of interest (*i.e.,* ApoB). An interfering RNA can be provided in several forms. For example, an interfering RNA can be provided as one or more isolated small-interfering RNA (siRNA) duplexes, longer double-stranded RNA (dsRNA), or as siRNA or dsRNA transcribed from a transcriptional cassette in a DNA plasmid. The interfering RNA may also be chemically synthesized. The interfering RNA can be administered alone or co-administered (*i.e.,* concurrently or consecutively) with conventional agents used to treat , *e.g.*, a disease or disorder involving hypercholesterolemia. Such agents include statins such as, *e.g.*, Lipitor®, Mevacor®, Zocor®, Lescol®, Crestor®, and Advicor®).

In preferred embodiments, the interfering RNA is an siRNA molecule that is capable of silencing expression of a target gene (*i.e*., ApoB). The siRNA is typically from about 15 to about 30 nucleotides in length. The synthesized or transcribed siRNA can have 3' overhangs of about 1-4 nucleotides, preferably of about 2-3 nucleotides, and 5' phosphate termini. In some embodiments, the siRNA lacks terminal phosphates. For example, siRNA targeting the sequences set forth in Tables 1-5 can be used to silence ApoB expression.

In some embodiments, the siRNA molecules described herein comprise at least one region of mismatch with its target sequence. As used herein, the term "region of mismatch" refers to a region of an siRNA that does not have 100 % complementarity to its target sequence. An siRNA may have at least one, two, or three regions of mismatch. The regions of mismatch may be contiguous or may be separated by one or more nucleotides. The regions of mismatch may comprise a single nucleotide or may comprise two, three, four, or more nucleotides.

### A. Selection of siRNA sequences

Suitable siRNA sequences that target a gene of interest (*i.e.,* ApoB) can be identified using any means known in the art. Typically, the methods described in Elbashir et al., Nature 411:494-498 (2001) and Elbashir et al., EMBO J 20: 6877-6888 (2001) are combined with rational design rules set forth in Reynolds et al., Nature Biotech. 22:326-330 (2004).

Typically, the sequence within about 50 to about 100 nucleotide 3' of the AUG start codon of a transcript from the target gene of interest is scanned for dinucleotide sequences *(e.g.,* AA, CC, GG, or UU) (*see, e.g*., Elbashir, et al., EMBO J 20: 6877-6888 (2001)). The nucleotides immediately 3' to the dinucleotide sequences are identified as potential siRNA target sequences. Typically, the 19, 21, 23, 25, 27, 29, 31, 33, 35, or more nucleotides immediately 3' to the dinucleotide sequences are identified as potential siRNA target sites. In some embodiments, the dinucleotide sequence is an AA sequence and the 19 nucleotides immediately 3' to the AA dinucleotide are identified as a potential siRNA target site. Typically, siRNA target sites are spaced at different postitions along the length of the target gene. To further enhance silencing efficiency of the siRNA sequences, potential siRNA target sites may be further analyzed to identify sites that do not contain regions of homology to other coding sequences. For example, a suitable siRNA target site of about 21 base pairs typically will not have more than 16-17 contiguous base pairs of homology to other coding sequences. If the siRNA sequences are to be expressed from an RNA Pol III promoter, siRNA target sequences lacking more than 4 contiguous A's or T's are selected.

Once a potential siRNA sequence has been identified, the sequence can be analyzed using a variety of criteria known in the art. For example, , to enhance their silencing efficiency, the siRNA sequences may be analyzed by a rational design algorithm to identify sequences that have one or more of the following features: (1) G/C content of about 25% to about 60% G/C; (2) at least 3 A/Us at positions 15-19 of the sense strand; (3) no internal repeats; (4) an A at position 19 of the sense strand; (5) an A at position 3 of the sense strand; (6) a U at position 10 of the sense strand; (7) no G/C at position 19 of the sense strand; and (8) no G at position 13 of the sense strand. siRNA design tools that incorporate algorithms that assign suitable values of each of these features and are useful for selection of siRNA can be found at, *e.g*., http://boz094.ust.hk/RNAi/siRNA. One of skill in the art will appreciate that sequences with one or more of the foregoing characteristics may be selected for further analysis and testing as potential siRNA sequences. siRNA sequences complementary to the siRNA target sites may also be designed.

Additionally, potential siRNA target sequences with one or more of the following criteria can often be eliminated as siRNA: (1) sequences comprising a stretch of 4 or more of the same base in a row; (2) sequences comprising homopolymers of Gs (*i.e*., to reduce possible non-specific effects due to structural characteristics of these polymers; (3) sequences comprising triple base motifs (*e.g*., GGG, CCC, AAA, or TTT); (4) sequences comprising stretches of 7 or more G/Cs in a row; and (5) sequence comprising direct repeats of 4 or more bases within the candidates resulting in internal fold-back structures. However, one of skill in the art will appreciate that sequences with one or more of the foregoing characteristics may still be selected for further analysis and testing as potential siRNA sequences.

Once a potential siRNA sequence has been identified, the sequence can be analyzed for the presence of any immunostimulatory properties, *e.g.*, using an in *vitro* cytokine assay or an *in vivo* animal model. Motifs in the sense and/or antisense strand of the siRNA sequence such as GU-rich motifs can also provide an indication of whether the sequence may be immunostimulatory. Once an siRNA molecule is found to be immunostimulatory, it can then be modified to decrease its immunostimulatory properties. As a non-limiting example, an siRNA sequence can be contacted with a mammalian responder cell under conditions such that the cell produces a detectable immune response to determine whether the siRNA is an immunostimulatory or a non-immunostimulatory siRNA. The mammalian responder cell may be from a naïve mammal (*i.e*., a mammal that has not previously been in contact with the gene product of the siRNA sequence). The mammalian responder cell may be, *e.g*., a peripheral blood mononuclear cell (PBMC), a macrophage, and the like. The detectable immune response may comprise production of a cytokine or growth factor such as, *e.g.*, TNF-α, TNF-β, IFN-α, IFN-γ, IL-6, IL-12, or a combination thereof. An siRNA molecule identified as being immunostimulatory can then be modified to decrease its immunostimulatory properties by replacing at least one of the nucleotides on the sense and/or antisense strand with modified nucleotides such as 2'OMe nucleotides (*e.g*., 2'OMe-guanosine, 2'OMe-uridine, 2'OMe-cytosine, and/or 2'OMe-adenosine). The modified siRNA can then be contacted with a mammalian responder cell as described above to confirm that its immunostimulatory properties have been reduced or abrogated.

Suitable *in vitro* assays for detecting an immune response include, but are not limited to, the double monoclonal antibody sandwich immunoassay technique of David et al. (U.S. Patent No. 4,376,110); monoclonal-polyclonal antibody sandwich assays (Wide et al., in Kirkham and Hunter, eds., Radioimmunoassay Methods, E. and S. Livingstone, Edinburgh (1970)); the "Western blot" method of Gordon et al. (U.S. Patent No. 4,452,901); immunoprecipitation of labeled ligand (Brown et al., J. Biol. Chem. 255:4980-4983 (1980)); enzyme-linked immunosorbent assays (ELISA) as described, for example, by Raines et al., J. Biol. Chem. 257:5154-5160 (1982); immunocytochemical techniques, including the use of fluorochromes (Brooks et al., Clin. Exp. Immunol. 39:477 (1980)); and neutralization of activity (Bowen-Pope et al., Proc. Natl. Acad. Sci. USA 81:2396-2400 (1984)). In addition to the immunoassays described above, a number of other immunoassays are available, including those described in U.S. Patent Nos. 3,817,827; 3,850,752; 3,901,654; 3,935,074; 3,984,533; 3,996,345; 4,034,074; and 4,098,876.

A non-limiting example of an *in vivo* model for detecting an immune response includes an *in vivo* mouse cytokine induction assay that can be performed as follows: (1) siRNA can be administered by standard intravenous injection in the lateral tail vein; (2) blood can be collected by cardiac puncture about 6 hours after administration and processed as plasma for cytokine analysis; and (3) cytokines can be quantified using sandwich ELISA kits according to the manufacturers' instructions (*e.g*., mouse and human IFN-α (PBL Biomedical; Piscataway, NJ); human IL-6 and TNF-α (eBioscience; San Diego, CA); and mouse IL-6, TNF-α, and IFN-γ (BD Biosciences; San Diego, CA)).

Monoclonal antibodies that specifically bind cytokines and growth factors are commercially available from multiple sources and can be generated using methods known in the art (*see, e.g*., Kohler and Milstein, Nature 256: 495-497 (1975) and Harlow and Lane, ANTIBODIES, A LABORATORY MANUAL, Cold Spring Harbor Publication, New York (1999)). Generation of monoclonal antibodies has been previously described and can be accomplished by any means known in the art (Buhring et al. in Hybridoma, Vol. 10, No.1, pp. 77-78 (1991)). In some methods, the monoclonal antibody is labeled (*e.g*., with any composition detectable by spectroscopic, photochemical, biochemical, electrical, optical, or chemical means) to facilitate detection.

### B. Generating siRNA

siRNA can be provided in several forms including, e.g. as one or more isolated siRNA duplexes, longer double-stranded RNA (dsRNA) or as siRNA or dsRNA transcribed from a transcriptional cassette in a DNA plasmid. siRNA may also be chemically synthesized. The siRNA sequences may have overhangs (*e.g*., 3' or 5' overhangs as described in Elbashir et al., Genes Dev. 15:188 (2001) or Nykänen et al., Cell 107:309 (2001), or may lack overhangs (*i.e.,* to have blunt ends).

An RNA population can be used to provide long precursor RNAs, or long precursor RNAs that have substantial or complete identity to a selected target sequence can be used to make the siRNA. The RNAs can be isolated from cells or tissue, synthesized, and/or cloned according to methods well known to those of skill in the art. The RNA can be a mixed population (obtained from cells or tissue, transcribed from cDNA, subtracted, selected etc.), or can represent a single target sequence. RNA can be naturally occurring, (e.g., isolated from tissue or cell samples), synthesized *in vitro* (*e.g.,* using T7 or SP6 polymerase and PCR products or a cloned cDNA), or chemically synthesized.

To form a long dsRNA, for synthetic RNAs, the complement is also transcribed *in vitro* and hybridized to form a dsRNA. If a naturally occuring RNA population is used, the RNA complements are also provided (*e.g.,* to form dsRNA for digestion by *E*. *coli* RNAse III or Dicer), *e.g.,* by transcribing cDNAs corresponding to the RNA population, or by using RNA polymerases. The precursor RNAs are then hybridized to form double stranded RNAs for digestion. The dsRNAs can be directly administered to a subject or can be digested *in vitro* prior to administration.

Alternatively, one or more DNA plasmids encoding one or more siRNA templates are used to provide siRNA. siRNA can be transcribed as sequences that automatically fold into duplexes with hairpin loops from DNA templates in plasmids having RNA polymerase III transcriptional units, for example, based on the naturally occurring transcription units for small nuclear RNA U6 or human RNase P RNA H1 *(see,* Brummelkamp, et al., Science 296:550 (2002); Donzé, et al., Nucleic Acids Res. 30:e46 (2002); Paddison, et al., Genes Dev. 16:948 (2002); Yu, et al., PNAS USA 99:6047 (2002); Lee, et al., Nat. Biotech. 20:500 (2002); Miyagishi, et al., Nat. Biotech. 20:497 (2002); Paul, et al., Nat. Biotech. 20:505 (2002); and Sui, et al., PNAS USA 99:5515 (2002)). Typically, a transcriptional unit or cassette will contain an RNA transcript promoter sequence, such as an H1-RNA or a U6 promoter, operably linked to a template for transcription of a desired siRNA sequence and a termination sequence, comprised of 2-3 uridine residues and a polythymidine (T5) sequence (polyadenylation signal) (Brummelkamp, *Science, supra*). The selected promoter can provide for constitutive or inducible transcription. Compositions and methods for DNA-directed transcription of RNA interference molecules is described in detail in U.S. Patent No. 6,573,099. The transcriptional unit is incorporated into a plasmid or DNA vector from which the interfering RNA is transcribed. Plasmids suitable for *in vivo* delivery of genetic material for therapeutic purposes are described in detail in U.S. Patent Nos. 5,962,428 and 5,910,488. The selected plasmid can provide for transient or stable delivery of a target cell. It will be apparent to those of skill in the art that plasmids originally designed to express desired gene sequences can be modified to contain a transcriptional unit cassette for transcription of siRNA.

Methods for isolating RNA, synthesizing RNA, hybridizing nucleic acids, making and screening cDNA libraries, and performing PCR are well known in the art (*see, e.g*., Gubler and Hoffman, Gene 25:263-269 (1983); Sambrook *et al*., *supra;* Ausubel *et al*., *supra*), as are PCR methods (*see* U.S. Patents 4,683,195 and 4,683,202; *PCR Protocols: A Guide to Methods and Applications* (Innis *et al*., eds, 1990)). Expression libraries are also well known to those of skill in the art. Additional basic texts disclosing the general methods of use in this invention include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994)).

The siRNA component of the SNALP can also be chemically synthesized. The oligonucleotides that comprise the modified siRNA molecule can be synthesized using any of a variety of techniques known in the art, such as those described in Usman et al., J. Am. Chem. Soc. 109:7845 (1987); Scaringe et al., Nucl. Acids Res. 18:5433 (1990); Wincott et al., Nucl. Acids Res. 23:2677-2684 (1995); and Wincott et al., Methods Mol. Bio. 74:59 (1997). The synthesis of oligonucleotides makes use of common nucleic acid protecting and coupling groups, such as dimethoxytrityl at the 5'-end and phosphoramidites at the 3'-end. As a non-limiting example, small scale syntheses can be conducted on an Applied Biosystems synthesizer using a 0.2 µmol scale protocol with a 2.5 min. coupling step for 2'-O-methylated nucleotides. Alternatively, syntheses at the 0.2 µmol scale can be performed on a 96-well plate synthesizer from Protogene (Palo Alto, CA). However, a larger or smaller scale of synthesis is also within the scope of the present invention. Suitable reagents for oligonucleotide synthesis, methods for RNA deprotection, and methods for RNA purification are known to those of skill in the art.

Modified siRNA molecules can also be synthesized via a tandem synthesis technique, wherein both strands are synthesized as a single continuous oligonucleotide fragment or strand separated by a cleavable linker that is subsequently cleaved to provide separate fragments or strands that hybridize to form the siRNA duplex. The linker can be a polynucleotide linker or a non-nucleotide linker. The tandem synthesis of modified siRNA can be readily adapted to both multiwell/multiplate synthesis platforms as well as large scale synthesis platforms employing batch reactors, synthesis columns, and the like. Alternatively, the modified siRNA molecule can be assembled from two distinct oligonucleotides, wherein one oligonucleotide comprises the sense strand and the other comprises the antisense strand of the siRNA. For example, each strand can be synthesized separately and joined together by hybridization or ligation following synthesis and/or deprotection. In certain other instances, the modified siRNA molecule can be synthesized as a single continuous oligonucleotide fragment, wherein the self-complementary sense and antisense regions hybridize to form an siRNA duplex having hairpin secondary structure.

### C. Modifying siRNA Sequences

The anti-ApoB siRNA molecules described herein can comprise at least one modified nucleotide in the sense and/or antisense strand (*see, e.g*., U.S. Provisional Patent Application No. 60/711,494). Examples of modified nucleotides suitable for use in the present invention include, but are not limited to, ribonucleotides having a 2'-O-methyl (2'OMe), 2'-deoxy-2'-fluoro, 2'-deoxy, 5-C-methyl, 2'-methoxyethyl, 4'-thio, 2'-amino, or 2'-C-allyl group. Modified nucleotides having a Northern conformation such as those described in, *e.g*., Saenger, Principles of Nucleic Acid Structure, Springer-Verlag Ed. (1984), are also suitable for use in the siRNA molecules of the present invention. Such modified nucleotides include, without limitation, locked nucleic acid (LNA) nucleotides (*e.g*., 2'-O, 4'-C-methylene-(D-ribofuranosyl) nucleotides), 2'-methoxyethoxy (MOE) nucleotides, 2'-methyl-thio-ethyl nucleotides, 2'-deoxy-2'-fluoro nucleotides, 2'-deoxy-2'-chloro nucleotides, and 2'-azido nucleotides. In certain instances, the siRNA molecule includes one or more G-clamp nucleotides. A G-clamp nucleotide refers to a modified cytosine analog wherein the modifications confer the ability to hydrogen bond both Watson-Crick and Hoogsteen faces of a complementary guanine nucleotide within a duplex (*see, e.g.,* Lin et al., J. Am. Chem. Soc. 120:8531-8532 (1998)). In addition, nucleotides having a nucleotide base analog such as, for example, C-phenyl, C-naphthyl, other aromatic derivatives, inosine, azole carboxamides, and nitroazole derivatives such as 3-nitropyrrole, 4-nitroindole, 5-nitroindole, and 6-nitroindole (*see, e.g.,* Loakes, Nucl. Acids Res. 29:2437-2447 (2001)) can be incorporated into the siRNA molecule.

In certain embodiments, the siRNA molecule can further comprise one or more chemical modifications such as terminal cap moieties, phosphate backbone modifications, and the like. Examples of terminal cap moieties include, without limitation, inverted deoxy abasic residues, glyceryl modifications, 4',5'-methylene nucleotides, 1-(β-D-erythrofuranosyl) nucleotides, 4'-thio nucleotides, carbocyclic nucleotides, 1,5-anhydrohexitol nucleotides, L-nucleotides, α-nucleotides, modified base nucleotides, threo-pentofuranosyl nucleotides, acyclic 3',4'-seco nucleotides, acyclic 3,4-dihydroxybutyl nucleotides, acyclic 3,5-dihydroxypentyl nucleotides, 3'-3'-inverted nucleotide moieties, 3'-3'-inverted abasic moieties, 3'-2'-inverted nucleotide moieties, 3'-2'-inverted abasic moieties, 5'-5'-inverted nucleotide moieties, 5'-5'-inverted abasic moieties, 3'-5'-inverted deoxy abasic moieties, 5'-amino-alkyl phosphate, 1,3-diamino-2-propyl phosphate, 3-aminopropyl phosphate, 6-aminohexyl phosphate, 1,2-aminododecyl phosphate, hydroxypropyl phosphate, 1,4-butanediol phosphate, 3'-phosphoramidate, 5'-phosphoramidate, hexylphosphate, aminohexyl phosphate, 3'-phosphate, 5'-amino, 3'-phosphorothioate, 5'-phosphorothioate, phosphorodithioate, and bridging or non-bridging methylphosphonate or 5'-mercapto moieties (*see, e.g.,* U.S. Patent No. 5,998,203; Beaucage et al., Tetrahedron 49:1925 (1993)). Non-limiting examples of phosphate backbone modifications (*i.e.,* resulting in modified internucleotide linkages) include phosphorothioate, phosphorodithioate, methylphosphonate, phosphotriester, morpholino, amidate, carbamate, carboxymethyl, acetamidate, polyamide, sulfonate, sulfonamide, sulfamate, formacetal, thioformacetal, and alkylsilyl substitutions (*see, e.g.,* Hunziker et al., Nucleic Acid Analogues: Synthesis and Properties, in Modem Synthetic Methods, VCH, 331-417 (1995); Mesmaeker et al., Novel Backbone Replacements for Oligonucleotides, in Carbohydrate Modifications in Antisense Research, ACS, 24-39 (1994)). Such chemical modifications can occur at the 5'-end and/or 3'-end of the sense strand, antisense strand, or both strands of the siRNA.

In some embodiments, the sense and/or antisense strand can further comprise a 3'-terminal overhang having about 1 to about 4 (*e*.*g*., 1, 2, 3, or 4) 2'-deoxy ribonucleotides and/or any combination of modified and unmodified nucleotides. Additional examples of modified nucleotides and types of chemical modifications that can be introduced into the modified siRNA molecule are described, *e*.*g*., in UK Patent No. GB 2,397,818 B.

The modified siRNA molecules described herein can optionally comprise one or more non-nucleotides in one or both strands of the siRNA. As used herein, the term "non-nucleotide" refers to any group or compound that can be incorporated into a nucleic acid chain in the place of one or more nucleotide units, including sugar and/or phosphate substitutions, and allows the remaining bases to exhibit their activity. The group or compound is abasic in that it does not contain a commonly recognized nucleotide base such as adenosine, guanine, cytosine, uracil, or thymine and therefore lacks a base at the 1'-position.

In other embodiments, chemical modification of the siRNA comprises attaching a conjugate to the chemically-modified siRNA molecule. The conjugate can be attached at the 5' and/or 3'-end of the sense and/or antisense strand of the chemically-modified siRNA via a covalent attachment such as, *e*.*g*., a biodegradable linker. The conjugate can also be attached to the chemically-modified siRNA, *e*.*g*., through a carbamate group or other linking group (*see, e.g*., U.S. Patent Publication Nos. 20050074771, 20050043219, and 20050158727). In certain instances, the conjugate is a molecule that facilitates the delivery of the chemically-modified siRNA into a cell. Examples of conjugate molecules suitable for attachment to a chemically-modified siRNA include, without limitation, steroids such as cholesterol, glycols such as polyethylene glycol (PEG), human serum albumin (HSA), fatty acids, carotenoids, terpenes, bile acids, folates (*e.g*., folic acid, folate analogs and derivatives thereof), sugars (*e.g.,* galactose, galactosamine, N-acetyl galactosamine, glucose, mannose, fructose, fucose, *etc*.), phospholipids, peptides, ligands for cellular receptors capable of mediating cellular uptake, and combinations thereof *(see, e.g.,* U.S. Patent Publication Nos. 20030130186, 20040110296, and 20040249178; U.S. Patent No. 6,753,423). Other examples include the lipophilic moiety, vitamin, polymer, peptide, protein, nucleic acid, small molecule, oligosaccharide, carbohydrate cluster, intercalator, minor groove binder, cleaving agent, and cross-linking agent conjugate molecules described in U.S. Patent Publication Nos. 20050119470 and 20050107325. Yet other examples include the 2'-O-alkyl amine, 2'-O-alkoxyalkyl amine, polyamine, C5-cationic modified pyrimidine, cationic peptide, guanidinium group, amidininium group, cationic amino acid conjugate molecules described in U.S. Patent Publication No. 20050153337. Additional examples include the hydrophobic group, membrane active compound, cell penetrating compound, cell targeting signal, interaction modifier, and steric stabilizer conjugate molecules described in U.S. Patent Publication No. 20040167090. Further examples include the conjugate molecules described in U.S. Patent Publication No. 20050239739. The type of conjugate used and the extent of conjugation to the chemically-modified siRNA molecule can be evaluated for improved pharmacokinetic profiles, bioavailability, and/or stability of the siRNA. As such, one skilled in the art can screen chemically-modified siRNA molecules having various conjugates attached thereto to identify ones having improved properties using any of a variety of well-known *in vitro* cell culture or *in vivo* animal models.

### C. In Vitro Methods Using siRNA

In addition silencing ApoB gene expression, the siRNA sequences described herein can be used in a variety of *in vitro* diagnostic and screening methods. For example, the siRNA sequences can be used as probes, *e*.*g*., to detect ApoB sequences. The siRNA sequences can also be used in screening assays, including high throughput assays to detect the effects of compounds that modulate lipid metabolism on ApoB expression.

In one exemplary embodiment, the siRNA sequences can be used in high density oligonucleotide array technology (e.g., GeneChip™) to identify ApoB protein, orthologs, alleles, conservatively modified variants, and polymorphic variants in this invention. In some cases, the siRNA can be used with GeneChip™ as a diagnostic tool in detecting a disease or disorder associated with ApoB expression or overexpression (*e.g*., hypercholesterolemia) in a biological sample, see, e.g., Gunthand et al., AIDS Res. Hum. Retroviruses 14: 869-876 (1998); Kozal et al., Nat. Med. 2:753-759 (1996); Matson et al., Anal. Biochem. 224:110-106 (1995); Lockhart et al., Nat. Biotechnol. 14:1675-1680 (1996); Gingeras et al., Genome Res. 8:435-448 (1998); Hacia et al., Nucleic Acids Res. 26:3865-3866 (1998).

In another exemplary embodiment, the siRNA sequences can be used in an *in vitro* diagnostic assay to determine the effects of a potential modulator of lipid metabolism (*i.e*., by determining the effects of the potential modulator on ApoB expression). A liver biopsy is taken from a subject undergoing treatment with the lipid metabolism modulator (*e.g.,* a statin such as Lipitor®, Mevacor®, Zocor®, Lescol®, Crestor®, or Advicor®) and the siRNA sequences are used to detect ApoB expression, thereby determining the effect of the modulator on ApoB expression.

In yet another exemplary embodiment, the siRNA sequences can be inserted into an expression vector and transfected into cells for use in a variety of *in vitro* diagnostic assays. Typically the expression vector contains a strong promoter to direct transcription and a transcription/translation terminator. Suitable bacterial promoters are well known in the art and described, e.g., in Sambrook *et al.,* and Ausubel *et al supra.* Bacterial expression systems for expressing the protein are available in, e.g., *E. coli, Bacillus sp.,* and *Salmonella* (Palva et al., Gene 22:229-235 (1983); Mosbach et al., Nature 302:543-545 (1983). Kits for such expression systems are commercially available. Eukaryotic expression systems for mammalian cells, yeast, and insect cells are well known in the art and are also commercially available.

Selection of the promoter used to direct expression of a heterologous nucleic acid depends on the particular application. The promoter is preferably positioned about the same distance from the heterologous transcription start site as it is from the transcription start site in its natural setting. As is known in the art, however, some variation in this distance can be accommodated without loss of promoter function.

In addition to the promoter, the expression vector typically contains a transcription unit or expression cassette that contains all the additional elements required for the expression of the nucleic acid in host cells. A typical expression cassette thus contains a promoter operably linked to the nucleic acid sequence and signals required for efficient polyadenylation of the transcript, ribosome binding sites, and translation termination. Additional elements of the cassette may include enhancers.

In addition to a promoter sequence, the expression cassette should also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic or prokaryotic cells may be used. Standard bacterial expression vectors include plasmids such as pBR322 based plasmids, pSKF, pET23D, and fusion expression systems such as MBP, GST, and LacZ. Epitope tags can also be added to recombinant proteins to provide convenient methods of isolation, e.g., c-myc.

Expression vectors containing regulatory elements from eukaryotic viruses are typically used in eukaryotic expression vectors, e.g., SV40 vectors, papilloma virus vectors, retroviral vectors, and vectors derived from Epstein-Barr virus. Other exemplary eukaryotic vectors include pMSG, pAV009/A.sup.+, pMTO10/A.sup.+, pMAMneo-5, baculovirus pDSVE, and any other vector allowing expression of proteins under the direction of the CMV promoter, SV40 early promoter, SV40 later promoter, metallothionein promoter, murine mammary tumor virus promoter, Rous sarcoma virus promoter, polyhedrin promoter, or other promoters shown effective for expression in eukaryotic cells.

The vector may further comprise a reporter gene. The siRNA sequence is operably linked to a reporter gene such as chloramphenicol acetyltransferase, firefly luciferase, bacterial luciferase, β-galactosidase and alkaline phosphatase. The reporter construct is typically transfected into a cell. After treatment with a potential modulator, the amount of reporter gene transcription, translation, or activity is measured according to standard techniques known to those of skill in the art.

The elements that are typically included in expression vectors also include a replicon that functions in *E*. *coli,* a gene encoding antibiotic resistance to permit selection of bacteria that harbor recombinant plasmids, and unique restriction sites in nonessential regions of the plasmid to allow insertion of eukaryotic sequences. The particular antibiotic resistance gene chosen is not critical, any of the many resistance genes known in the art are suitable. The prokaryotic sequences are preferably chosen such that they do not interfere with the replication of the DNA in eukaryotic cells, if necessary.

Transformation of eukaryotic and prokaryotic cells are performed according to standard techniques (see, e.g., Morrison, J. Bact. 132:349-351 (1977); Clark-Curtiss & Curtiss, Methods in Enzymology 101:347-362 (Wu et al., eds, 1983). Any of the well-known procedures for introducing foreign nucleotide sequences into host cells may be used. These include the use of calcium phosphate transfection, polybrene, protoplast fusion, electroporation, biolistics, liposomes, microinjection, plasma vectors, viral vectors and any of the other well known methods for introducing cloned genomic DNA, cDNA, synthetic DNA or other foreign genetic material into a host cell (see, e.g., Sambrook et al., supra). It is only necessary that the particular genetic engineering procedure used be capable of successfully introducing at least one gene into the host cell capable of expressing the nucleotide sequence of interest. Suitable cell include for such cell based assays include both primary hepatocytes and hepatocyte cell lines, as described herein, e.g., Hep G2 cells, Hep 2 cells, HEP-3B cells, McArdle RH7777 cells, BRL3A cells, and NRL clone 9 cells.

After the expression vector is introduced into the cells, the transfected cells are cultured under conditions favoring expression of the siRNA sequence. The transfected cells can be used in high throughput assays to identify compounds that directly modulate ApoB expression as well as compounds that modulate expression of genes upstream and downstream of ApoB, thereby mapping genes involved in lipid metabolism pathways. The transfected cells can also be used to determine the effects of silencing ApoB expression on other components of the lipid metabolism pathway. For example, following expression of the siRNA in the cells, expression of other genes (*e.g.,* ApoE, ApoA-I, ApoE, and ApoAV) in the lipid metabolism pathway can be detected to determine the effect of silencing ApoB expression.

### IV. Lipid-Based Carrier Systems Containing siRNA

In one aspect, the present invention provides stabilized nucleic acid-lipid particles (SPLPs or SNALPs) and other lipid-based carrier systems containing the siRNA described herein. Preferably, the lipid-based carrier system is a SNALP. Alternatively, the lipid-based carrier system is a liposome, micelle, virosome, nucleic acid complex, or mixtures thereof.

Non-limiting examples of alternative lipid-based carrier systems suitable for use in the present invention include polycationic polymer/nucleic acid complexes (*see, e.g*., U.S. Patent Publication Nos. 20050222064 and 20030185890), cyclodextrin-polymer/nucleic acid complexes (*see, e.g.,* U.S. Patent Publication No. 20040087024), biodegradable poly(β-amino ester) polymer/nucleic acid complexes (*see, e.g.,* U.S. Patent Publication No. 20040071654), pH-sensitive liposomes (*see, e.g*., U.S. Patent Publication No. 20020192274; AU 2003210303), anionic liposomes (*see, e.g.,* U.S. Patent Publication No. 20030026831), cationic liposomes *(see, e.g.,* U.S. Patent Publication Nos. 20030229040, 20020160038, and 20020012998; U.S. Patent No. 5,908,635; PCT Publication No. WO 01/72283), antibody-coated. liposomes (*see*, *e*.*g*., U.S. Patent Publication No. 20030108597; PCT Publication No. WO 00/50008), reversibly masked lipoplexes (*see, e.g*., U.S. Patent Publication Nos. 20030180950), cell-type specific liposomes (*see*, *e.g.,* U.S. Patent Publication No. 20030198664), liposomes containing nucleic acid and peptides (*see, e.g.,* U.S. Patent No. 6,207,456), microparticles containing polymeric matrices (*see, e.g*., U.S. Patent Publication No. 20040142475), pH-sensitive lipoplexes (*see, e.g*., U.S. Patent Publication No. 20020192275), liposomes containing lipids derivatized with releasable hydrophilic polymers (*see, e.g*., U.S. Patent Publication No. 20030031704), lipid-entrapped nucleic acid (*see, e.g.,* PCT Publication Nos. WO 03/057190 and WO 03/059322), lipid-encapsulated nucleic acid (*see, e.g.,* U.S. Patent Publication No. 20030129221; U.S. Patent No. 5,756,122), polycationic sterol derivative/nucleic acid complexes (*see, e.g.,* U.S. Patent No. 6,756,054), other liposomal compositions (*see, e.g.,* U.S. Patent Publication Nos. 20030035829 and 20030072794; U.S. Patent No. 6,200,599), other microparticle compositions *(see, e.g.,* U.S. Patent Publication No. 20030157030), polyplexes (*see, e*.*g*., PCT Publication No. WO 03/066069), emulsion compositions (*see, e.g.,* U.S. Patent No. 6,747,014), condensed nucleic acid complexes (*see, e.g.,* U.S. Patent Publication No. 20050123600), other polycationic/nucleic acid complexes (*see, e.g.,* U.S. Patent Publication No. 20030125281), polyvinylether/nucleic acid complexes (*see, e.g.,* U.S. Patent Publication No. 20040156909), polycyclic amidinium/nucleic acid complexes *(see, e.g.,* U.S. Patent Publication No. 20030220289), nanocapsule and microcapsule compositions (*see, e.g.,* AU 2002358514; PCT Publication No. WO 02/096551), stabilized mixtures of liposomes and emulsions (*see, e.g.,* EP1304160), porphyrin/nucleic acid complexes (*see, e.g.,* U.S. Patent No. 6,620,805), lipid-nucleic acid complexes (*see, e.g.,* U.S. Patent Publication No. 20030203865), nucleic acid micro-emulsions (*see, e.g.,* U.S. Patent Publication No. 20050037086), and cationic lipid-based compositions (*see, e.g.,* U.S. Patent Publication No. 20050234232). One skilled in the art will appreciate that the anti-ApoB siRNA of the present invention can also be delivered as a naked siRNA molecule.

### V. Stable Nucleic Acid-Lipid Particles (SNALPs) and Properties Thereof

The stable nucleic acid-lipid particles or, alternatively, SNALPs typically comprise an siRNA molecule that targets ApoB expression, a cationic lipid (*e.g.,* a cationic lipid of Formula I or II) and a non-cationic lipid. The SNALP can further comprise a bilayer stabilizing component (*i*.*e*., a conjugated lipid that inhibits aggregation of the SNALPs). Preferably the SNALP comprises an siRNA molecule that targets ApoB expression, a cationic lipid, a non-cationic lipid, and a conjugated lipid that inhibits aggregation of the SNALPs. The nucleic acid-lipid particles may comprise at least 1, 2, 3, 4, 5, or more siRNA molecules comprising the sequences set forth in Table 1, rows A-F of Table 2, and Tables 3-7. In some embodiments, the nucleic acid-lipid particles comprise an siRNA molecule that targets ApoB and an siRNA molecules that targets another gene of interest (*e.g*., microsomal triglyceride transfer protein (MTP), acyl-CoA cholesterol acyl transferase (ACAT), farnesoid X receptor (FXR), 5-hydroxy-3-methylglutaryl-coenzyme A reductase (HMGR)).

The SNALPs of the present invention typically have a mean diameter of about 50 nm to about 150 nm, more typically about 60 nm to about 130 nm, more typically about 70 nm to about 110 nm, most typically about 70 to about 90 nm, and are substantially nontoxic. In addition, the nucleic acids present in the SNALPs of the present invention are resistant in aqueous solution to degradation with a nuclease.

The lipid-nucleic acid particles of the present invention typically comprise a nucleic acid, a cationic lipid, a non-cationic lipid, and can further comprise a PEG-lipid conjugate. The cationic lipid typically comprises from about 2 mol % to about 60 mol %, from about 5 mol % to about 50 mol %, from about 10 mol % to about 45 mol %, from about 20 mol % to about 40 mol %, or from about 30 mol % to about 40 mol % of the total lipid present in said particle. The non-cationic lipid typically comprises from about 5 mol % to about 90 mol %, from about 10 mol % to about 85 mol %, from about 20 mol % to about 80 mol %, from about 30 mol % to about 70 mol %, from about 40 mol % to about 60 mol % or about 48 mol % of the total lipid present in said particle. The PEG-lipid conjugate typically comprises from about 0.5 mol % to about 20 mol %, from about 1.5 mol % to about 18 mol %, from about 4 mol % to about 15 mol %, from about 5 mol % to about 12 mol %, or about 2 mol % of the total lipid present in said particle. The nucleic acid-lipid particles of the present invention may further comprise cholesterol. If present, the cholesterol typically comprises from about 0 mol % to about 10 mol %, about 2 mol % to about 10 mol %, about 10 mol % to about 60 mol %, from about 12 mol % to about 58 mol %, from about 20 mol % to about 55 mol %, or about 48 mol % of the total lipid present in said particle. It will be readily apparent to one of skill in the art that the proportions of the components of the nucleic acid-lipid particles may be varied. For example for systemic delivery, the cationic lipid may comprise from about 5 mol % to about 15 mol % of the total lipid present in said particle and for local or regional delivery, the cationic lipid may comprise from about 30 mol % to about 50 mol %, or about 40 mol % of the total lipid present in the particle.

### A. Cationic Lipids

Various suitable cationic lipids may be used in the present invention, either alone or in combination with one or more other cationic lipid species or neutral lipid species.

Suitable cationic lipids include, for example, DLinDMA, DLenDMA, DODAC, DOTMA, DDAB, DOTAP, DOSPA, DOGS, DC-Chol and DMRIE, or combinations thereof. A number of these lipids and related analogs, which are also useful in the present invention, have been described in U.S. Patent Nos. 5,208,036, 5,264,618, 5,279,833, 5,283,185, 5,753,613 and 5,785,992. Additionally, a number of commercial preparations of cationic lipids are available and can be used in the present invention. These include, for example, LIPOFECTIN^{®} (commercially available cationic liposomes comprising DOTMA and DOPE, from GIBCO/BRL, Grand Island, New York, USA); LIPOFECTAMINE^{®} (commercially available cationic liposomes comprising DOSPA and DOPE, from GIBCO/BRL); and TRANSFECTAM® (commercially available cationic liposomes comprising DOGS from Promega Corp., Madison, Wisconsin, USA). In addition, cationic lipids of Formula I and Formula II can be used in the present invention. Cationic lipids of Formula I and II have the following structures: 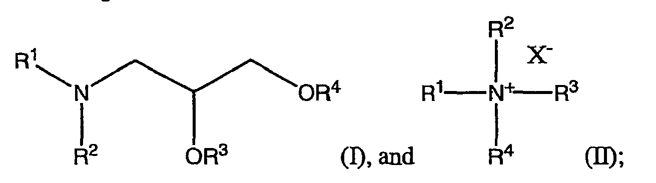 wherein R¹ and R² are independently selected and are H or C₁-C₃ alkyls. R³ and R⁴ are independently selected and are alkyl groups having from about 10 to about 20 carbon atoms; at least one of R³ and R⁴ comprises at least two sites of unsaturation. In one embodiment, R³ and R⁴ are both the same, *i.e*., R³ and R⁴ are both linoleyl (C18), *etc.* In another embodiment, R³ and R⁴ are different, *i*.*e*., R³ is myristyl (C14) and R⁴ is linoleyl (C18). In some embodiments, the cationic lipids of the present invention are symmetrical, *i.e*., R³ and R⁴ are both the same. In another preferred embodiment, both R³ and R⁴ comprise at least two sites of unsaturation. In some embodiments, R³ and R⁴ are independently selected from dodecadienyl, tetradecadienyl, hexadecadienyl, linoleyl, and icosadienyl. In some embodiments, R³ and R⁴ are both linoleyl. In some embodiments, R³ and R⁴comprise at least three sites of unsaturation and are independently selected from, *e.g*., dodecatrienyl, tetradectrienyl, hexadecatrienyl, linolenyl, and icosatrienyl.

The cationic lipids of Formula I and Formula II described herein typically carry a net positive charge at a selected pH, such as physiological pH. It has been surprisingly found that cationic lipids comprising alkyl chains with multiple sites of unsaturation, *e.g*., at least two or three sites of unsaturation, are particularly useful for forming lipid-nucleic acid particles with increased membrane fluidity. A number of cationic lipids and related analogs, which are also useful in the present invention, have been described in co-pending USSN 08/316,399; U.S. Patent Nos. 5,208,036, 5,264,618, 5,279,833 and 5,283,185, and WO 96/10390.

Additional suitable cationic lipids include, *e*.*g*., dioctadecyldimethylammonium ("DODMA"), Distearyldimethylammonium ("DSDMA"), N,N-dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 3 -(N-(N',N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). A number of these lipids and related analogs, which are also useful in the present invention, have been described in U.S. Patent Nos. 5,208,036, 5,264,618, 5,279,833, 5,283,185, 5,753,613 and 5,785,992.

### B. Non-cationic Lipids

The non-cationic lipids used in the present invention can be any of a variety of neutral uncharged, zwitterionic or anionic lipids capable of producing a stable complex. They are preferably neutral, although they can alternatively be positively or negatively charged. Examples of non-cationic lipids useful in the present invention include:
phospholipid-related materials, such as lecithin, phosphatidylethanolamine, lysolecithin, lysophosphatidylethanolamine, phosphatidylserine, phosphatidylinositol, sphingomyelin, cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoyl-phosphatidylethanolamine (DOPE), palmitoyloleoylphosphatidyleholine (POPC), palmitoyloleoyl- phosphatidylethanolamine (POPE) and dioleoyl- phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, 1-stearoyl-2-oleoyl-phosphatidyethanolamine (SOPE), and 1,2-dielaidoyl-sn-glycero-3-phophoethanolamine (transDOPE). Non-cationic lipids or sterols such as cholesterol may be present. Additional nonphosphorous containing lipids are, *e*.*g*., stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide and the like, diacylphosphatidylcholine, diacylphosphatidylethanolamine, ceramide, sphingomyelin, cephalin, and cerebrosides. Other lipids such as lysophosphatidylcholine and lysophosphatidylethanolamine may be present. Non-cationic lipids also include polyethylene glycol-based polymers such as PEG 2000, PEG 5000 and polyethylene glycol conjugated to phospholipids or to ceramides (referred to as PEG-Cer), as described in co-pending USSN 08/316,429.

In preferred embodiments, the non-cationic lipids are diacylphosphatidylcholine (*e.g.,* distearoylphosphatidylcholine, dioleoylphosphatidylcholine, dipalmitoylphosphatidylcholine and dilinoleoylphosphatidylcholine), diacylphosphatidylethanolamine (*e*.*g*., dioleoylphosphatidylethanolamine and palmitoyloleoylphosphatidylethanolamine), ceramide or sphingomyelin. The acyl groups in these lipids are preferably acyl groups derived from fatty acids having C₁₀-C₂₄ carbon chains. More preferably the acyl groups are lauroyl, myristoyl, palmitoyl, stearoyl or oleoyl. In particularly preferred embodiments, the non-cationic lipid can be cholesterol, 1,2-*sn-*dioleoylphosphatidylethanolamine, or egg sphingomyelin (ESM).

### C. Bilayer Stabilizing Component

In addition to cationic and non-cationic lipids, the nucleic acid-lipid particles (*e.g.,* SNALPs and SPLPs) of the present invention can further comprise a bilayer stabilizing component (BSC) such as an ATTA-lipid or a PEG-lipid, such as PEG coupled to dialkyloxypropyls (PEG-DAA) as described in, *e*.*g*., WO 05/026372, PEG coupled to diacylglycerol (PEG-DAG) as described in, *e*.*g*., U.S. Patent Publication Nos. 20030077829 and 2005008689), PEG coupled to phosphatidylethanolamine (PE) (PEG-PE), or PEG conjugated to ceramides, or a mixture thereof *(see,* U.S. Patent No. 5,885,613). In one preferred embodiment, the BSC is a conjugated lipid that inhibits aggregation of the nucleic acid-lipid particles. Suitable conjugated lipids include, but are not limited to PEG-lipid conjugates, ATTA-lipid conjugates, cationic-polymer-lipid conjugates (CPLs) or mixtures thereof. In one preferred embodiment, the nucleic acid-lipid particles comprise either a PEG-lipid conjugate or an ATTA-lipid conjugate together with a CPL.

PEG is a polyethylene glycol, a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, for example, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), and monomethoxypolyethylene glycol-imidazolyl-carbonyl (MePEG-IM). In addition, monomethoxypolyethyleneglycol-acetic acid (MePEG-CH₂COOH), is particularly useful for preparing the PEG-lipid conjugates including, *e*.*g*., PEG-DAA conjugates.

In some embodiments, the PEG has an average molecular weight of from about 550 daltons to about 10,000 daltons, more preferably of about 750 daltons to about 5,000 daltons, more preferably of about 1,000 daltons to about 5,000 daltons, more preferably of about 1,500 daltons to about 3,000 daltons and, even more preferably, of about 2,000 daltons, or about 750 daltons. The PEG can be optionally substituted by an alkyl, alkoxy, acyl or aryl group. PEG can be conjugated directly to the lipid or may be linked to the lipid via a linker moiety. Any linker moiety suitable for coupling the PEG to a lipid can be used including, *e*.*g*., non-ester containing linker moieties and ester-containing linker moieties. In some embodiments, the linker moiety is a non-ester containing linker moiety. As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, *etc*. as well as combinations thereof (such as a linker containing both a carbamate linker moiety and an amido linker moiety). In some embodiments, a carbamate linker is used to couple the PEG to the lipid.

In other embodiments, an ester containing linker moiety is used to couple the PEG to the lipid. Suitable ester containing linker moieties include, *e*.*g*., carbonate (-OC(O)O-), succinoyl, phosphate esters (-O-(O)POH-O-), sulfonate esters, and combinations thereof.

Phosphatidylethanolamines having a variety of acyl chain groups of varying chain lengths and degrees of saturation can be conjugated to polyethyleneglycol to form the bilayer stabilizing component. Such phosphatidylethanolamines are commercially available, or can be isolated or synthesized using conventional techniques known to those of skilled in the art. Phosphatidylethanolamines containing saturated or unsaturated fatty acids with carbon chain lengths in the range of C₁₀ to C₂₀ are preferred. Phosphatidylethanolamines with mono- or diunsaturated fatty acids and mixtures of saturated and unsaturated fatty acids can also be used. Suitable phosphatidylethanolamines include, but are not limited to, the following:
dimyristoylphosphatidylethanolamine (DMPE), dipalmitoylphosphatidylethanolamine (DPPE), dioleoylphosphatidylethanolamine (DOPE) and distearoylphosphatidylethanolamine (DSPE).

In some embodiments, the PEG-lipid is a PEG-DAA conjugate has the following formula:

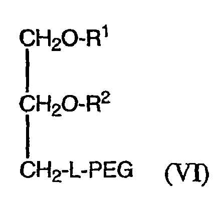

In Formula VI, R¹ and R² are independently selected and are alkyl groups having from about 10 to about 22 carbon atoms. The long-chain alkyl groups can be saturated or unsaturated. Suitable alkyl groups include, but are not limited to, lauryl (C12), myristyl (C14), palmityl (C16), stearyl (C18) and icosyl (C20). In some embodiments, R¹ and R² are the same, *i.e*., R¹ and R² are both myristyl (*i.e*., dimyristyl), R¹ and R² are both stearyl (*i.e*., distearyl), *etc.* In some embodiments, the alkyl groups are saturated.

In Formula VI above, "PEG" is a polyethylene glycol having an average molecular weight ranging of about 550 daltons to about 10,000 daltons, about 750 daltons to about 5,000 daltons, about 1,000 daltons to about 5,000 daltons, about 1,500 daltons to about 3,000 daltons, about 2,000 daltons, or about 750 daltons. The PEG can be optionally substituted with alkyl, alkoxy, acyl or aryl. In some embodiments, the terminal hydroxyl group is substituted with a methoxy or methyl group.

In Formula VI, above, "L" is a non-ester containing linker moiety or an ester containing linker moiety. In some embodiments, L is a non-ester containing linker moiety. Suitable non-ester containing linkers include, but are not limited to, an amido linker moiety, an amino linker moiety, a carbonyl linker moiety, a carbamate linker moiety, a urea linker moiety, an ether linker moiety, a disulphide linker moiety, a succinamidyl linker moiety and combinations thereof. In some embodiments, the non-ester containing linker moiety is a carbamate linker moiety (*i.e*., a PEG-C-DAA conjugate), an amido linker moiety (*i.e*., a PEG-A-DAA conjugate), or a succinamidyl linker moiety (*i.e*., a PEG-S-DAA conjugate).

The PEG-DAA conjugates are synthesized using standard techniques and reagents known to those of skill in the art. It will be recognized that the PEG-DAA conjugates will contain various amide, amine, ether, thio, carbamate and urea linkages. T hose of skill in the art will recognize that methods and reagents for forming these bonds are well known and readily available. *See, e.g*., March, ADVANCED ORGANIC CHEMISTRY (Wiley 1992), Larock, COMPREHENSIVE ORGANIC TRANSFORMATIONS (VCH 1989); and Furniss, VOGEL'S TEXTBOOK OF PRACTICAL ORGANIC CHEMISTRY 5th ed. (Longman 1989). It will also be appreciated that any functional groups present may require protection and deprotection at different points in the synthesis of the PEG-DAA conjugates. Those of skill in the art will recognize that such techniques are well known. *See, e.g*., Green and Wuts, PROTECTIVE GROUPS IN ORGANIC SYNTHESIS (Wiley 1991).

In some embodiments, the PEG-DAA conjugate is a dilauryloxypropyl (C12)-PEG conjugate, dimyristyloxypropyl (C14)-PEG conjugate, a dipalmitoyloxypropyl (C16)-PEG conjugate or a disteryloxypropyl (C18)-PEG conjugate. Those of skill in the art will readily appreciate that other dialkyloxypropyls can be used in the PEG-DAA conjugates of the present invention.

In addition to the foregoing, it will be readily apparent to those of skill in the art that other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses, such as hydroxymethylcellulose or hydroxyethylcellulose.

In addition to the foregoing components, the SNALPs and SPLPs of the present invention can further comprise cationic poly(ethylene glycol) (PEG) lipids, or CPLs, that have been designed for insertion into lipid bilayers to impart a positive charge(*see*, Chen, et al., Bioconj. Chem. 11:433-437 (2000)). Suitable SPLPs and SPLP-CPLs for use in the present invention, and methods of making and using SPLPs and SPLP-CPLs, are disclosed, *e*.*g*., in U.S. Patent No. 6,852,334 and WO 00/62813. Cationic polymer lipids (CPLs) useful in the present invention have the following architectural features: (1) a lipid anchor, such as a hydrophobic lipid, for incorporating the CPLs into the lipid bilayer; (2) a hydrophilic spacer, such as a polyethylene glycol, for linking the lipid anchor to a cationic head group; and (3) a polycationic moiety, such as a naturally occurring amino acid, to produce a protonizable cationic head group.

Suitable CPL include compounds of Formula VII:

A-W-Y (VII)

wherein A, W and Y are as described below.

With reference to Formula VII, "A" is a lipid moiety such as an amphipathic lipid, a neutral lipid or a hydrophobic lipid that acts as a lipid anchor. Suitable lipid examples include vesicle-forming lipids or vesicle adopting lipids and include, but are not limited to, diacylglycerolyls, dialkylglycerolyls, N-N-dialkylaminos, 1,2-diacyloxy-3-aminopropanes and 1,2-dialkyl-3-aminopropanes.

"W" is a polymer or an oligomer, such as a hydrophilic polymer or oligomer. Typically, the hydrophilic polymer is a biocompatable polymer that is nonimmunogenic or possesses low inherent immunogenicity. Alternatively, the hydrophilic polymer can be weakly antigenic if used with appropriate adjuvants. Suitable nonimmunogenic polymers include, but are not limited to, PEG, polyamides, polylactic acid, polyglycolic acid, polylactic acid/polyglycolic acid copolymers and combinations thereof. In some embodiments, the polymer has a molecular weight of from about 250 to about 7000 daltons.

"Y" is a polycationic moiety. The term polycationic moiety refers to a compound, derivative, or functional group having a positive charge, typically at least 2 positive charges at a selected pH, typically physiological pH. Suitable polycationic moieties include basic amino acids and their derivatives such as arginine, asparagine, glutamine, lysine and histidine; spermine; spermidine; cationic dendrimers; polyamines; polyamine sugars; and amino polysaccharides. The polycationic moieties can be linear, such as linear tetralysine, branched or dendrimeric in structure. Polycationic moieties have between about 2 to about 15 positive charges, between about 2 to about 12 positive charges, or between about 2 to about 8 positive charges at selected pH values. The selection of which polycationic moiety to employ may be determined by the type of liposome application which is desired.

The charges on the polycationic moieties can be either distributed around the entire liposome moiety, or alternatively, they can be a discrete concentration of charge density in one particular area of the liposome moiety *e.g*., a charge spike. If the charge density is distributed on the liposome, the charge density can be equally distributed or unequally distributed. All variations of charge distribution of the polycationic moiety are encompassed by the present invention.

The lipid "A," and the nonimmunogenic polymer "W," can be attached by various methods and preferably, by covalent attachment. Methods known to those of skill in the art can be used for the covalent attachment of "A" and "W." Suitable linkages include, but are not limited to, amide, amine, carboxyl, carbonate, carbamate, ester and hydrazone linkages. It will be apparent to those skilled in the art that "A" and "W" must have complementary functional groups to effectuate the linkage. The reaction of these two groups, one on the lipid and the other on the polymer, will provide the desired linkage. For example, when the lipid is a diacylglycerol and the terminal hydroxyl is activated, for instance with NHS and DCC, to form an active ester, and is then reacted with a polymer which contains an amino group, such as with a polyamide (see, U.S. Patent Nos. 6,320,017 and 6,586,559), an amide bond will form between the two groups.

In certain instances, the polycationic moiety can have a ligand attached, such as a targeting ligand or a chelating moiety for complexing calcium. Preferably, after the ligand is attached, the cationic moiety maintains a positive charge. In certain instances, the ligand that is attached has a positive charge. Suitable ligands include, but are not limited to, a compound or device with a reactive functional group and include lipids, amphipathic lipids, carrier compounds, bioaffinity compounds, biomaterials, biopolymers, biomedical devices, analytically detectable compounds, therapeutically active compounds, enzymes, peptides, proteins, antibodies, immune stimulators, radiolabels, fluorogens, biotin, drugs, haptens, DNA, RNA, polysaccharides, liposomes, virosomes, micelles, immunoglobulins, functional groups, other targeting moieties, or toxins.

### VI. Preparation of SNALPs

The present invention provides a method of preparing serum-stable nucleic acid-lipid particles in which an interfering RNA (*e.g*., an anti-ApoB siRNA) is encapsulated in a lipid bilayer and is protected from degradation. The particles made by the methods of this invention typically have a size of about 50 nm to about 150 nm, about 60 nm to about 130 nm, about 70 nm to about 110 nm, or about 70 nm to about 90 nm. The particles can be formed by any method known in the art including, but not limited to: a continuous mixing method, a direct dilution process, a detergent dialysis method, or a modification of a reverse-phase method which utilizes organic solvents to provide a single phase during mixing of the components.

In preferred embodiments, the cationic lipids are lipids of Formula I and II or combinations thereof. In other preferred embodiments, the non-cationic lipids are ESM, DOPE, DOPC, DPPE, DMPE, 16:0 Monomethyl Phosphatidylethanolamine, 16:0 Dimethyl Phosphatidylethanolamine, 18:1 Trans Phosphatidylethanolamine, 18:0 18:1 Phosphatidylethanolamine (SOPE), 1,2-dielaidoyl-sn-glycero-3-phophoethanolamine (transDOPE), 16:0 18:1 Phosphatidylethanolamine, DSPE, polyethylene glycol-based polymers (*e.g.,* PEG 2000, PEG 5000, PEG-modified diacylglycerols, or PEG-modified dialkyloxypropyls), distearoylphosphatidylcholine (DSPC), cholesterol, or combinations thereof. In still other preferred embodiments, the organic solvents are methanol, chloroform, methylene chloride, ethanol, diethyl ether or combinations thereof.

In a particularly preferred embodiment, the present invention provides for nucleic acid-lipid particles produced via a continuous mixing method, *e.g*., process that includes providing an aqueous solution comprising a nucleic acid such as an siRNA, in a first reservoir, and providing an organic lipid solution in a second reservoir, and mixing the aqueous solution with the organic lipid solution such that the organic lipid solution mixes with the aqueous solution so as to substantially instantaneously produce a liposome encapsulating the nucleic acid (*e.g*., siRNA). This process and the apparatus for carrying this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a liposome substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution *(i.e.,* aqueous solution) to produce a nucleic acid-lipid particle.

The serum-stable nucleic acid-lipid particles formed using the continuous mixing method typically have a size of from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

In another embodiment, the present invention provides for nucleic acid-lipid particles produced via a direct dilution process that includes forming a liposome solution and immediately and directly introducing the liposome solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of liposome solution introduced thereto. As a non-limiting example, liposome solution in 45% ethanol when introduced into the collection vessel containing an equal volume of ethanol will advantageously yield smaller particles in about 22.5%, about 20%, or about 15% ethanol.

In even another embodiment, the present invention provides for nucleic acid-lipid particles produced via a direct dilution process in which a third reservoir containing dilution buffer is fluidly coupled to a second mixing region. In this embodiment, the liposome solution formed in a first mixing region is immediately and directly mixed with dilution buffer in the second mixing region. In preferred aspects, the second mixing region includes a T-connector arranged so that the liposome solution and the dilution buffer flows meet as opposing 180° flows, however, connectors providing shallower angles can be used, *e*.*g*., from about 27° to about 180°. A pump mechanism delivers a controllable flow of buffer to the second mixing region. In one aspect, the flow rate of dilution buffer provided to the second mixing region is controlled to be substantially equal to the flow rate of liposome solution introduced thereto from the first mixing region. This embodiment advantageously allows for more control of the flow of dilution buffer mixing with the liposome solution in the second mixing region, and therefore also the concentration of liposome solution in buffer throughout the second mixing process. Such control of the dilution buffer flow rate advantageously allows for small particle size formation at reduced concentrations.

These processes and the apparati for carrying out these direct dilution processes is described in detail in U.S. Provisional Patent Application No. 60/703,380 filed July 27, 2005.

The serum-stable nucleic acid-lipid particles formed using the direct dilution process typically have a size of from about of from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

In some embodiments, the particles are formed using detergent dialysis. Without intending to be bound by any particular mechanism of formation, a plasmid or other nucleic acid (*e.g*., siRNA) is contacted with a detergent solution of cationic lipids to form a coated nucleic acid complex. These coated nucleic acids can aggregate and precipitate. However, the presence of a detergent reduces this aggregation and allows the coated nucleic acids to react with excess lipids (typically, non-cationic lipids) to form particles in which the plasmid or other nucleic acid is encapsulated in a lipid bilayer. Thus, serum-stable nucleic acid-lipid particles can be prepared as follows:
(a) combining a nucleic acid with cationic lipids in a detergent solution to form a coated nucleic acid-lipid complex;
(b) contacting non-cationic lipids with the coated nucleic acid-lipid complex to form a detergent solution comprising a nucleic acid-lipid complex and non-cationic lipids; and
(c) dialyzing the detergent solution of step (b) to provide a solution of serum-stable nucleic acid-lipid particles, wherein the nucleic acid is encapsulated in a lipid bilayer and the particles are serum-stable and have a size of from about 50 to about 150 nm.

An initial solution of coated nucleic acid-lipid complexes is formed by combining the nucleic acid with the cationic lipids in a detergent solution.

In these embodiments, the detergent solution is preferably an aqueous solution of a neutral detergent having a critical micelle concentration of 15-300 mM, more preferably 20-50 mM. Examples of suitable detergents include, for example, N,N'-((octanoylimino)-bis-(trimethylene))-bis-(D-gluconamide) (BIGCHAP); BRIJ 35; Deoxy-BIGCHAP; dodecylpoly(ethylene glycol) ether; Tween 20; Tween 40; Tween 60; Tween 80; Tween 85; Mega 8; Mega 9; Zwittergent® 3-08; Zwittergent® 3-10; Triton X-405; hexyl-, heptyl-, octyl- and nonyl-β-D-glucopyranoside; and heptylthioglucopyranoside; with octyl β-D-glucopyranoside and Tween-20 being the most preferred. The concentration of detergent in the detergent solution is typically about 100 mM to about 2 M, preferably from about 200 mM to about 1.5 M.

The cationic lipids and nucleic acids will typically be combined to produce a charge ratio (+/-) of about 1:1 to about 20:1, in a ratio of about 1:1 to about 12:1, or in a ratio of about 2:1 to about 6:1. Additionally, the overall concentration of nucleic acid in solution will typically be from about 25 µg/mL to about 1 mg/mL, from about 25 µg/mL to about 200 µg/mL, or from about 50 µg/mL to about 100 µg/mL. The combination of nucleic acids and cationic lipids in detergent solution is kept, typically at room temperature, for a period of time which is sufficient for the coated complexes to form. Alternatively, the nucleic acids and cationic lipids can be combined in the detergent solution and warmed to temperatures of up to about 37°C, about 50°C, about 60°C, or about 70°C. For nucleic acids which are particularly sensitive to temperature, the coated complexes can be formed at lower temperatures, typically down to about 4°C.

In some embodiments, the nucleic acid to lipid ratios (mass/mass ratios) in a formed nucleic acid-lipid particle will range from about 0.01 to about 0.2, from about 0.03 to about 0.01 or from about 0.01 to about 0.08. The ratio of the starting materials also falls within this range. In other embodiments, the nucleic acid-lipid particle preparation uses about 400 µg nucleic acid per 10 mg total lipid or a nucleic acid to lipid ratio (mg:mg) of about 0.01 to about 0.08 and, more preferably, about 0.04, which corresponds to 1.25 mg of total lipid per 50 µg of nucleic acid.

The detergent solution of the coated nucleic acid-lipid complexes is then contacted with non-cationic lipids to provide a detergent solution of nucleic acid-lipid complexes and non-cationic lipids. The non-cationic lipids which are useful in this step include, diacylphosphatidylcholine, diacylphosphatidylethanolamine, ceramide, sphingomyelin, cephalin, cardiolipin, and cerebrosides. In preferred embodiments, the non-cationic lipids are diacylphosphatidylcholine, diacylphosphatidylethanolamine, ceramide or sphingomyelin. The acyl groups in these lipids are preferably acyl groups derived from fatty acids having C₁₀-C₂₄ carbon chains. More preferably the acyl groups are lauroyl, myristoyl, palmitoyl, stearoyl or oleoyl. In particularly preferred embodiments, the non-cationic lipid will be 1,2-sn-dioleoylphosphatidylethanolamine (DOPE), palmitoyl oleoyl phosphatidylcholine (POPC), egg phosphatidylcholine (EPC), distearoylphosphatidylcholine (DSPC), cholesterol, or a mixture thereof. In the most preferred embodiments, the nucleic acid-lipid particles will be fusogenic particles with enhanced properties *in vivo* and the non-cationic lipid will be DSPC or DOPE. In addition, the nucleic acid-lipid particles of the present invention may further comprise cholesterol. In other preferred embodiments, the non-cationic lipids will further comprise polyethylene glycol-based polymers such as PEG 2000, PEG 5000 and polyethylene glycol conjugated to a diacylglycerol, a ceramide or a phospholipid, as described in U.S. Patent No. 5,820,873 and U.S. Patent Publication No. 20030077829. In further preferred embodiments, the non-cationic lipids will further comprise polyethylene glycol-based polymers such as PEG 2000, PEG 5000 and polyethylene glycol conjugated to a dialkyloxypropyl.

The amount of non-cationic lipid which is used in the present methods is typically about 2 to about 20 mg of total lipids to 50 µg of nucleic acid. Preferably, the amount of total lipid is from about 5 to about 10 mg per 50 µg of nucleic acid.

Following formation of the detergent solution of nucleic acid-lipid complexes and non-cationic lipids, the detergent is removed, preferably by dialysis. The removal of the detergent results in the formation of a lipid-bilayer which surrounds the nucleic acid providing serum-stable nucleic acid-lipid particles which have a size of from about 50 nm to about 150 nm, more typically about 100 nm to about 130 nm, more typically about 110 nm to about 115 nm, most typically about 65 to 95 nm. The particles thus formed do not aggregate and are optionally sized to achieve a uniform particle size.

The serum-stable nucleic acid-lipid particles can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles is described in U.S. Patent No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In another group of embodiments, the present invention provides a method for the preparation of serum-stable nucleic acid-lipid particles, comprising:
(a) preparing a mixture comprising cationic lipids and non-cationic lipids in an organic solvent;
(b) contacting an aqueous solution of nucleic acid with the mixture in step (a) to provide a clear single phase; and
(c) removing the organic solvent to provide a suspension of nucleic acid-lipid particles, wherein the nucleic acid is encapsulated in a lipid bilayer, and the particles are stable in serum and have a size of from about 50 to about 150 nm.

The nucleic acids (*e.g.,* siRNA), cationic lipids and non-cationic lipids which are useful in this group of embodiments are as described for the detergent dialysis methods above.

The selection of an organic solvent will typically involve consideration of solvent polarity and the ease with which the solvent can be removed at the later stages of particle formation. The organic solvent, which is also used as a solubilizing agent, is in an amount sufficient to provide a clear single phase mixture of nucleic acid and lipids. Suitable solvents include, but are not limited to, chloroform, dichloromethane, diethylether, cyclohexane, cyclopentane, benzene, toluene, methanol, or other aliphatic alcohols such as propanol, isopropanol, butanol, tert-butanol, iso-butanol, pentanol and hexanol. Combinations of two or more solvents may also be used in the present invention.

Contacting the nucleic acid with the organic solution of cationic and non-cationic lipids is accomplished by mixing together a first solution of nucleic acid, which is typically an aqueous solution, and a second organic solution of the lipids. One of skill in the art will understand that this mixing can take place by any number of methods, for example by mechanical means such as by using vortex mixers.

After the nucleic acid has been contacted with the organic solution of lipids, the organic solvent is removed, thus forming an aqueous suspension of serum-stable nucleic acid-lipid particles. The methods used to remove the organic solvent will typically involve evaporation at reduced pressures or blowing a stream of inert gas (*e.g*., nitrogen or argon) across the mixture.

The serum-stable nucleic acid-lipid particles thus formed will typically be sized from about 50 nm to about 150 nm, more typically about 100 nm to about 130 nm, most typically about 110 nm to about 115 nm. To achieve further size reduction or homogeneity of size in the particles, sizing can be conducted as described above.

In other embodiments, the methods will further comprise adding non-lipid polycations which are useful to effect the delivery to cells using the present compositions. Examples of suitable non-lipid polycations include, but are limited to, hexadimethrine bromide (sold under the brand name POLYBRENE®, from Aldrich Chemical Co., Milwaukee, Wisconsin, USA) or other salts of heaxadimethrine. Other suitable polycations include, for example, salts of poly-L-orniithine, poly-L-arginine, poly-L-lysine, poly-D-lysine, polyallylamine and polyethyleneimine.

In certain embodiments, the formation of the nucleic acid-lipid particles can be carried out either in a mono-phase system (*e.g.,* a Bligh and Dyer monophase or similar mixture of aqueous and organic solvents) or in a two-phase system with suitable mixing.

When formation of the complexes is carried out in a mono-phase system, the cationic lipids and nucleic acids are each dissolved in a volume of the mono-phase mixture. Combination of the two solutions provides a single mixture in which the complexes form. Alternatively, the complexes can form in two-phase mixtures in which the cationic lipids bind to the nucleic acid (which is present in the aqueous phase), and "pull" it into the organic phase.

In another embodiment, serum-stable nucleic acid-lipid particles can be prepared as follows:
(a) contacting nucleic acids with a solution comprising non-cationic lipids and a detergent to form a nucleic acid-lipid mixture;
(b) contacting cationic lipids with the nucleic acid-lipid mixture to neutralize a portion of the negative charge of the nucleic acids and form a charge-neutralized mixture of nucleic acids and lipids; and
(c) removing the detergent from the charge-neutralized mixture to provide the nucleic acid-lipid particles in which the nucleic acids are protected from degradation.

In one group of embodiments, the solution of non-cationic lipids and detergent is an aqueous solution. Contacting the nucleic acids with the solution of non-cationic lipids and detergent is typically accomplished by mixing together a first solution of nucleic acids and a second solution of the lipids and detergent. One of skill in the art will understand that this mixing can take place by any number of methods, for example, by mechanical means such as by using vortex mixers. Preferably, the nucleic acid solution is also a detergent solution. The amount of non-cationic lipid which is used in the present method is typically determined based on the amount of cationic lipid used, and is typically of from about 0.2 to 5 times the amount of cationic lipid, preferably from about 0.5 to about 2 times the amount of cationic lipid used.

In some embodiments, the nucleic acids are precondensed as described in, *e.g*., U.S. Patent Application No. 09/744,103.

The nucleic acid-lipid mixture thus formed is contacted with cationic lipids to neutralize a portion of the negative charge which is associated with the nucleic acids (or other polyanionic materials) present. The amount of cationic lipids used will typically be sufficient to neutralize at least 50 % of the negative charge of the nucleic acid. Preferably, the negative charge will be at least 70 % neutralized, more preferably at least 90 % neutralized. Cationic lipids which are useful in the present invention, include, for example, DLinDMA and, DLenDMA. These lipids and related analogs have been described in U.S. Provisional Patent Application Nos. 60/578,075, filed June 7, 2004; 60/610,746, filed September 17, 2004; and 60/679,427, filed May 9, 2005.

Contacting the cationic lipids with the nucleic acid-lipid mixture can be accomplished by any of a number of techniques, preferably by mixing together a solution of the cationic lipid and a solution containing the nucleic acid-lipid mixture. Upon mixing the two solutions (or contacting in any other manner), a portion of the negative charge associated with the nucleic acid is neutralized. Nevertheless, the nucleic acid remains in an uncondensed state and acquires hydrophilic characteristics.

After the cationic lipids have been contacted with the nucleic acid-lipid mixture, the detergent (or combination of detergent and organic solvent) is removed, thus forming the nucleic acid-lipid particles. The methods used to remove the detergent will typically involve dialysis. When organic solvents are present, removal is typically accomplished by evaporation at reduced pressures or by blowing a stream of inert gas (*e.g.,* nitrogen or argon) across the mixture.

The particles thus formed will typically be sized from about 50 nm to several microns, more typically about 50 nm to about 150 nm, even more typically about 100 nm to about 130 nm, most typically about 110 nm to about 115 nm. To achieve further size reduction or homogeneity of size in the particles, the nucleic acid-lipid particles can be sonicated, filtered or subjected to other sizing techniques which are used in liposomal formulations and are known to those-of-skill in the art.

In other embodiments, the methods will further comprise adding non-lipid polycations which are useful to effect the lipofection of cells using the present compositions. Examples of suitable non-lipid polycations include, hexadimethrine bromide (sold under the brand name POLYBRENE®, from Aldrich Chemical Co., Milwaukee, Wisconsin, USA) or other salts of hexadimethrine. Other suitable polycations include, for example, salts of poly-L-ornithine, poly-L-arginitne, poly-L-lysine, poly-D-lysine, polyallylamine and polyethyleneimine. Addition of these salts is preferably after the particles have been formed.

In another aspect, the serum-stable nucleic acid-lipid particles can be prepared as follows:
(a) contacting an amount of cationic lipids with nucleic acids in a solution; the solution comprising from about 15-35 % water and about 65-85 % organic solvent and the amount of cationic lipids being sufficient to produce a +/- charge ratio of from about 0.85 to about 2.0, to provide a hydrophobic nucleic acid-lipid complex;
(b)contacting the hydrophobic, nucleic acid-lipid complex in solution with non-cationic lipids, to provide a nucleic acid-lipid mixture; and
(c)removing the organic solvents from the nucleic acid-lipid mixture to provide nucleic acid-lipid particles in which the nucleic acids are protected from degradation.

The nucleic acids, non-cationic lipids, cationic lipids and organic solvents which are useful in this aspect of the invention are the same as those described for the methods above which used detergents. In one group of embodiments, the solution of step (a) is a mono-phase. In another group of embodiments, the solution of step (a) is two-phase.

In preferred embodiments, the non-cationic lipids are ESM, DOPE, DOPC, polyethylene glycol-based polymers (*e.g*., PEG 2000, PEG 5000, PEG-modified diacylglycerols, or PEG-modified dialkyloxypropyls), distearoylphosphatidylcholine (DSPC), DPPE, DMPE, 16:0 Monomethyl Phosphatidylethanolamine, 16:0 Dimethyl Phosphatidylethanolamine, 18:1 Trans Phosphatidylethanolamine, 18:0 18:1 Phosphatidylethanolamine (SOPE), 1,2-dielaidoyl-sn-glycero-3-phophoethanolamine (transDOPE),16:0 18:1 Phosphatidylethanolamine, DSPE, cholesterol, or combinations thereof. In still other preferred embodiments, the organic solvents are methanol, chloroform, methylene chloride, ethanol, diethyl ether or combinations thereof.

In one embodiment, the nucleic acid an interfering RNA (*i.e*., and anti-ApoB siRNA); the cationic lipid is DLindMA, DLenDMA, DODAC, DDAB, DOTMA, DOSPA, DMRIE, DOGS or combinations thereof; the non-cationic lipid is ESM, DOPE, DAG-PEGs, distearoylphosphatidylcholine (DSPC), DPPE, DMPE, 16:0 Monomethyl Phosphatidylethanolamine, 16:0 Dimethyl Phosphatidylethanolamine, 18:1 Trans Phosphatidylethanolamine, 18:0 18:1 Phosphatidylethanolamine (SOPE), 16:018:1 Phosphatidylethanolamine DSPE, cholesterol, or combinations thereof (*e.g*. DSPC and PEG-DAA); and the organic solvent is methanol, chloroform, methylene chloride, ethanol, diethyl ether or combinations thereof.

As above, contacting the nucleic acids with the cationic lipids is typically accomplished by mixing together a first solution of nucleic acids and a second solution of the lipids, preferably by mechanical means such as by using vortex mixers. The resulting mixture contains complexes as described above. These complexes are then converted to particles by the addition of non-cationic lipids and the removal of the organic solvent. The addition of the non-cationic lipids is typically accomplished by simply adding a solution of the non-cationic lipids to the mixture containing the complexes. A reverse addition can also be used. Subsequent removal of organic solvents can be accomplished by methods known to those of skill in the art and also described above.

The amount of non-cationic lipids which is used in this aspect of the invention is typically an amount of from about 0.2 to about 15 times the amount (on a mole basis) of cationic lipids which was used to provide the charge-neutralized nucleic acid-lipid complex. Preferably, the amount is from about 0.5 to about 9 times the amount of cationic lipids used.

In yet another embodiment, the nucleic acid-lipid particles prepared by the methods described above are either net charge neutral or carry an overall charge which provides the particles with greater transfection activity. Preferably, the nucleic acid component of the particles is a nucleic acid which interferes with the production of an undesired protein. In some embodiments, the nucleic acid comprises an interfering RNA (*i*.*e*., an anti-ApoB siRNA), the non-cationic lipid is egg sphingomyelin and the cationic lipid is DLinDMA or DLenDMA. In some embodiments, the nucleic acid comprises an interfering RNA, the non-cationic lipid is a mixture of DSPC and cholesterol, and the cationic lipid is DLinDMA or DLenDMA. In other preferred embodiments, the non-cationic lipid may further comprise cholesterol.

A variety of general methods for making SNALP-CPLs (CPL-containing SNALPs) are discussed herein. Two general techniques include "post-insertion" technique, that is, insertion of a CPL into for example, a pre-formed SNALP, and the "standard" technique, wherein the CPL is included in the lipid mixture during for example, the SNALP formation steps. The post-insertion technique results in SNALPs having CPLs mainly in the external face of the SNALP bilayer membrane, whereas standard techniques provide SNALPs having CPLs on both internal and external faces. The method is especially useful for vesicles made from phospholipids (which can contain cholesterol) and also for vesicles containing PEG-lipids (such as PEG-DAAs and PEG-DAGs). Methods of making SNALP-CPL, are taught, for example, in U.S. Patent Nos. 5,705,385, 6,586,410, 5,981,501 6,534,484; 6,852,334; U.S. Patent Publication No. 20020072121; and WO 00/62813.

### VII. Kits

The present invention also provides nucleic acid-lipid particles in kit form. The kit will typically be comprised of a one or more containers containing the compositions of the present inventions, preferably in dehydrated form, with instructions for their rehydration and administration. For example, one container of a kit may hold the dehydrated nucleic acid-lipid particles and another container of the kit may hold a buffer suitable for rehydrating the particles.

### VIII. Administration of Nucleic Acid-Lipid Particles

Once formed, the serum-stable nucleic acid-lipid particles of the present invention are useful for the introduction of nucleic acids (*i.e*., siRNA that silences expression of ApoB) into cells (*e.g*., a hepatocyte). Accordingly, the present invention also provides methods for introducing a nucleic acids (*e.g.,* a plasmid or and siRNA) into a cell. The methods are carried out *in vitro* or *in vivo* by first forming the particles as described above and then contacting the particles with the cells for a period of time sufficient for delivery of the nucleic acid to the cell to occur.

The nucleic acid-lipid particles of the present invention can be adsorbed to almost any cell type with which they are mixed or contacted. Once adsorbed, the particles can either be endocytosed by a portion of the cells, exchange lipids with cell membranes, or fuse with the cells. Transfer or incorporation of the nucleic acid portion of the particle can take place via any one of these pathways. In particular, when fusion takes place, the particle membrane is integrated into the cell membrane and the contents of the particle combine with the intracellular fluid.

The nucleic acid-lipid particles of the present invention can be administered either alone or in mixture with a physiologically-acceptable carrier (such as physiological saline or phosphate buffer) selected in accordance with the route of administration and standard pharmaceutical practice. Generally, normal saline will be employed as the pharmaceutically acceptable carrier. Other suitable carriers include, *e.g*., water, buffered water, 0.4% saline, 0.3% glycine, and the like, including glycoproteins for enhanced stability, such as albumin, lipoprotein, globulin, etc.

The pharmaceutical carrier is generally added following particle formation. Thus, after the particle is formed, the particle can be diluted into pharmaceutically acceptable carriers such as normal saline.

The concentration of particles in the pharmaceutical formulations can vary widely, *i*.*e*., from less than about 0.05%, usually at or at least about 2-5% to as much as 10 to 30% by weight and will be selected primarily by fluid volumes, viscosities, etc., in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well known sterilization techniques. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol and water-soluble iron-specific chelators, such as ferrioxamine, are suitable.

The nucleic acid-lipid particles can be incorporated into a broad range of topical dosage forms including, but not limited to, gels, oils, emulsions, topical creams, pastes, ointments, lotions, foams, and the like.

### A. In vivo administration

Systemic delivery for *in vivo* gene therapy, *i.e*., delivery of a therapeutic nucleic acid to a distal target cell via body systems such as the circulation, has been achieved using nucleic acid-lipid particles such as those disclosed in WO 96/40964, U.S. Patent Nos. 5,705,385, 5,976,567, 5,981,501, and 6,410,328. This latter format provides a fully encapsulated nucleic acid-lipid particle that protects the nucleic acid from nuclease degradation in serum, is nonimmunogenic, is small in size and is suitable for repeat dosing.

For *in vivo* administration, administration can be in any manner known in the art, *e.g.,* by injection, oral administration, inhalation (*e.g.,* intransal or intratracheal), transdermal application, or rectal administration. Administration can be accomplished via single or divided doses. The pharmaceutical compositions can be administered parenterally, *i.e*., intraarticularly, intravenously, intraperitoneally, subcutaneously, or intramuscularly. In some embodiments, the pharmaceutical compositions are administered intravenously or intraperitoneally by a bolus injection (*see, e.g.,* Stadler, et al., U.S. Patent No. 5,286,634). Intracellular nucleic acid delivery has also been discussed in Straubringer, et al., Methods Enzymol, Academic Press, New York. 101:512 (1983); Mannino, et al., Biotechniques 6:682 (1988); Nicolau, et al., Crit. Rev. Ther. Drug Carrier Syst. 6:239 (1989), and Behr, Acc. Chem. Res. 26:274 (1993). Still other methods of administering lipid based therapeutics are described in, for example, Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179; Lenk et al., U.S. Patent No. 4,522,803; and Fountain et al., U.S. Patent No. 4,588,578. The lipid nucleic acid particles can be administered by direct injection at the site of disease or by injection at a site distal from the site of disease (*see, e.g.,* Culver, HUMAN GENE THERAPY, MaryAnn Liebert, Inc., Publishers, New York. pp.70-71(1994)).

The compositions of the present invention, either alone or in combination with other suitable components, can be made into aerosol formulations (*i.e*., they can be "nebulized") to be administered via inhalation (*e.g.,* intranasally or intratracheally) (*see,* Brigham, et al., Am. J. Sci. 298(4):278 (1989)). Aerosol formulations can be placed into pressurized acceptable propellants, such as dichlorodifluoromethane, propane, nitrogen, and the like.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, *e.g.,* sucrose, as well as pastilles comprising the active ingredient in an inert base, such as gelatin and glycerin or sucrose and acacia emulsions, gels, and the like containing, in addition to the active ingredient, carriers known in the art.

Generally, when administered intravenously, the nucleic acid-lipid formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, PA, 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, etc. Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

When preparing pharmaceutical preparations of the nucleic acid-lipid particles of the invention, it is preferable to use quantities of the particles which have been purified to reduce or eliminate empty particles or particles with nucleic acid associated with the external surface.

The methods of the present invention may be practiced in a variety of hosts. Preferred hosts include mammalian species, such as avian (*e.g.,* ducks), primates (*e.g.,* humans and chimpanzees as well as other nonhuman primates), canines, felines, equines, bovines, ovines, caprines, rodents (*e.g.,* rats and mice), lagomorphs, and swine.

The amount of particles administered will depend upon the ratio of nucleic acid to lipid; the particular nucleic acid used, the disease state being diagnosed; the age, weight, and condition of the patient and the judgment of the clinician; but will generally be between about 0.01 and about 50 mg per kilogram of body weight; preferably between about 0.1 and about 5 mg/kg of body weight or about 10⁸-10¹⁰ particles per injection.

### B. Cells for delivery of interfering RNA

The compositions and methods of the present invention are used to treat a wide variety of cell types, *in vivo* and *in vitro.* Suitable cells include, *e.g*., hematopoietic precursor (stem) cells, fibroblasts, keratinocytes, hepatocytes, endothelial cells, skeletal and smooth muscle cells, osteoblasts, neurons, quiescent lymphocytes, terminally differentiated cells, slow or noncycling primary cells, parenchymal cells, lymphoid cells, epithelial cells, bone cells, and the like.

*In vivo* delivery of nucleic acid lipid particles encapsulating an interfering RNA is suited for targeting cells of any type. The methods and compositions can be employed with cells of a wide variety of vertebrates, including mammals, such as, e.g, canines, felines, equines, bovines, ovines, caprines, rodents (*e.g.,* mice, rats and guinea pigs), swine, and primates (*e.g*. monkeys, chimpanzees, and humans).

To the extent that tissue culture of cells may be required, it is well known in the art. Freshney (1994) (Culture of Animal Cells, a Manual of Basic Technique, third edition Wiley-Liss, New York), Kuchler et al. (1977) Biochemical Methods in Cell Culture and Virology, Kuchler, R.J., Dowden, Hutchinson and Ross, Inc., and the references cited therein provides a general guide to the culture of cells. Cultured cell systems often will be in the form of monolayers of cells, although cell suspensions are also used.

### C. Detection of SNALPs

In some embodiments, the nucleic acid-lipid particles are detectable in the subject at about 1, 2, 4, 6, 8, 12, 24, 48, 60, 72, or 96 hours, 6, 8, 10,12, 14, 16, 18, 19, 22, 24, 25, or 28 days after administration of the particles. For example about 1, 2, 5, 10, 15, 20, 25, 30, 40, or 50 % of the particles may be detectable in the subject at each of these time points. The presence of the particles can be detected in the cells, tissues, or other biological samples from the subject. The particles may be detected, *e*.*g*., by direct detection of the particles, detection of the interfering RNA sequence, detection of the target sequence of interest (*i.e.,* by detecting expression or reduced expression of the ApoB sequence of interest), detection of a compound modulated by ApoB (*e*.*g*., serum cholesterol) or a combination thereof.

### 1. Detection of Particles

Nucleic acid-lipid particles are detected herein using any methods known in the art. For example, a label can be coupled directly or indirectly to a component of the SNALP or other lipid-based carrier system using methods well known in the art. A wide variety of labels can be used, with the choice of label depending on sensitivity required, ease of conjugation with the SNALP component, stability requirements, and available instrumentation and disposal provisions. Suitable labels include, but are not limited to, spectral labels, such as fluorescent dyes (*e*.*g*., fluorescein and derivatives, such as fluorescein isothiocyanate (FITC) and Oregon Green™; rhodamine and derivatives, such Texas red, tetrarhodimine isothiocynate (TRITC), etc., digoxigenin, biotin, phycoerythrin, AMCA, CyDyes™, and the like; radiolabels, such as ³H, ¹²⁵I, ³⁵S, ¹⁴C, ³²P, ³³P, etc.; enzymes, such as horse radish peroxidase, alkaline phosphatase, etc.; spectral colorimetric labels, such as colloidal gold or colored glass or plastic beads, such as polystyrene, polypropylene, latex, etc. The label can be detected using any means known in the art.

### 2. Detection of Nucleic Acids

Nucleic acids (*i.e*., siRNA that silence ApoB expression) are detected and quantified herein by any of a number of means well known to those of skill in the art. The detection of nucleic acids proceeds by well known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), hyperdiffusion chromatography, may also be employed

The selection of a nucleic acid hybridization format is not critical. A variety of nucleic acid hybridization formats are known to those skilled in the art. For example, common formats include sandwich assays and competition or displacement assays. Hybridization techniques are generally described in "Nucleic Acid Hybridization, A Practical Approach," Ed. Hames, B.D. and Higgins, S.J., IRL Press, 1985.

The sensitivity of the hybridization assays may be enhanced through use of a nucleic acid amplification system which multiplies the target nucleic acid being detected. *In vitro* amplification techniques suitable for amplifying sequences for use as molecular probes or for generating nucleic acid fragments for subsequent subcloning are known. Examples of techniques sufficient to direct persons of skill through such *in vitro* amplification methods, including the polymerase chain reaction (PCR) the ligase chain reaction (LCR), Qβ-replicase amplification and other RNA polymerase mediated techniques (*e.g*., NASBA™) are found in Sambrook, et al., In Molecular Cloning: A Laboratory Manual, Cold Spring Harbor Laboratory Press, 2000, and Ausubel et al., SHORT PROTOCOLS IN MOLECULAR BIOLOGY, eds., Current Protocols, a joint venture between Greene Publishing Associates, Inc. and John Wiley & Sons, Inc., (2002), as well as Mullis et al. (1987), U.S. Patent No. 4,683,202; PCR Protocols A Guide to Methods and Applications (Innis et al. eds) Academic Press Inc. San Diego, CA (1990) (Innis); Arnheim & Levinson (October 1, 1990), C&EN 36; The Journal Of NIH Research, 3:81 (1991); (Kwoh et al., PNAS USA 86:1173 (1989); Guatelli et al., PNAS USA 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990), and Sooknanan and Malek, Biotechnology, 13:563 (1905). Improved methods of cloning *in vitro* amplified nucleic acids are described in Wallace et al., U.S. Pat. No. 5,426,039. Other methods described in the art are the nucleic acid sequence based amplification (NASBA™, Cangene, Mississauga, Ontario) and Q Beta Replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

Oligonucleotides for use as probes, *e.g*., in *in vitro* amplification methods, for use as gene probes, or as inhibitor components are typically synthesized chemically according to the solid phase phosphoramidite triester method described by Beaucage and Caruthers, Tetrahedron Letts., 22(20):1859 1862 (1981), *e.g*., using an automated synthesizer, as described in Needham VanDevanter et al., Nucleic Acids Res., 12:6159 (1984). Purification of oligonucleotides, where necessary, is typically performed by either native acrylamide gel electrophoresis or by anion exchange HPLC as described in Pearson and Regnier, J. Chrom., 255:137 149 (1983). The sequence of the synthetic oligonucleotides can be verified using the chemical degradation method of Maxam and Gilbert (1980) in Grossman and Moldave (eds.) Academic Press, New York, Methods in Enzymology, 65:499.

An alternative means for determining the level of transcription is *in situ* hybridization. In situ hybridization assays are well known and are generally described in Angerer et al., Methods Enzymol., 152:649 (1987). In an in situ hybridization assay cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### D. Detection of an Immune Response

An immune response to induced by the siRNA (*i.e*., modified or unmodified siRNA that silence ApoB expression) described herein can be long-lived and can be detected long after administration of the siRNA or nucleic acid-lipid particles containing the siRNA. An immune response to the siRNA can be detected by using immunoassays that detect the presence or absence of cytokines and growth factors *e.g*., produced by responder cells.

Suitable immunoassays include the double monoclonal antibody sandwich immunoassay technique of David et al. (U.S. Patent No. 4,376,110); monoclonal-polyclonal antibody sandwich assays (Wide et al., in Kirkham and Hunter, eds., Radioimmunoassay Methods, E. and S. Livingstone, Edinburgh (1970)); the "Western blot" method of Gordon et al. (U.S. Patent No. 4,452,901); immunoprecipitation of labeled ligand (Brown et al. (1980) J. Biol. Chem. 255:4980-4983); enzyme-linked immunosorbent assays (ELISA) as described, for example, by Raines et al. (1982) J. Biol. Chem. 257:5154-5160; immunocytochemical techniques, including the use of fluorochromes (Brooks et al. (1980) Clin. Exp. Immunol. 39:477); and neutralization of activity (Bowen-Pope et al. (1984) PNAS USA 81:2396-2400). In addition to the immunoassays described above, a number of other immunoassays are available, including those described in U.S. Patent Nos. 3,817,827; 3,850,752; 3,901,654; 3,935,074; 3,984,533; 3,996,345; 4,034,074; and 4,098,876.

Monoclonal antibodies that specifically bind cytokines and growth factors (*e*.*g*., Il-6, IL-12, TNF-α, IFN-α, and IFN-γ can be generated using methods known in the art (*see, e*.*g*., Kohler and Milstein, Nature 256: 495-497 (1975) and Harlow and Lane, ANTIBODIES, A LABORATORY MANUAL, Cold Spring Harbor Publication, New York (1999)). Generation of monoclonal antibodies has been previously described and can be accomplished by any means known in the art. (Buhring et al. in Hybridoma 1991, Vol. 10, No. 1, pp. 77-78). For example, an animal such as a guinea pig or rat, preferably a mouse is immunized with an immunogenic polypeptide, the antibody-producing cells, preferably splenic lymphocytes, are collected and fused to a stable, immortalized cell line, preferably a myeloma cell line, to produce hybridoma cells which are then isolated and cloned. (U.S. Patent 6,156,882). In some methods, the monoclonal antibody is labeled to facilitate detection.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

### EXAMPLES

The following examples are provided to illustrate, but not to limit the claimed invention.

### Example 1: Selection of Candidate ApoB siRNA

Candidate Apolipoprotein B sequences were identified by scanning and Apolipoprotein sequence to identify AA dinucleotide motifs and the 19 nucleotides 3' of the motif. The following candidate sequences were eliminated: (1) sequences comprising a stretch of 4 or more of the same base in a row; (2) sequences comprising homopolymers of Gs; (3) sequences comprising triple base motifs (GGG, CCC, AAA, or TTT); (4) sequences comprisig stretches of 7 or more G/Cs in a row; and (5) sequences comprising direct repeats of 4 or more bases resulting in internal fold-back structures.

Reynold's Rational Design criteria was then applied to the remaining candidate sequences to identify sequences with:
1. 30%-52% GC Content;
2. At least 3 A/Us at positions 15-19 (sense);
3. Absence of internal repeats;
4. A at position 19 (sense);
5. A at position 3 (sense);
6. U at position 10 (sense);
7. No G/C at position 19 (sense); and
8. No G at position 13 (sense).

Next, the following criteria were removed to identify additional candidate sequences of interest: 30-52% GC (went higher on 1 candidate); the requirement for a AA leader sequence; (no constraints chosen to get 3 candidates) triplet motifs (found in 5 candidates)

BLASTn was used to identify sequences that don't cross-hybridize in the mouse genome. Finally, the candidate sequences were scanned to avoid or reduce GUGU, polyU or GU rich sequences. The candidate sequences and their positions are shown in Table 1 below.

**Table 1**

| Working Designation | Target Sequence (5'-3', sense strand only) | Selected as Immunostimulatory? |
|---|---|---|
| ApoB-148 | GAA GAU GCA ACU CGA UUC A | No |
| ApoB-911 | ACA GUC GCU UCU UCA GUG A | No |
| ApoB-1455 | UGA AUG CAC GGG CAA UGA A | No |
| ApoB-3050 | CGG GAG AAG UGG AGC AGU A | No |
| ApoB-3193 | AGA AGC AGG ACC UUA UCU A | No |
| ApoB-3699 | GGA CAU GGG UUC CAA AUU A | No |
| ApoB-10067 | CCA ATG CTG GAC TTT ATA A | No |
| ApoB-13205 | GCA TGC TTA CTG ATA TAA A | No |
| ApoB-309 | CAA CCA GTG TAC CCT TAA A | Yes |

### Example 2: Production of Type I Interferons and Inflammatory Cytokines Following Administration of SNALP encapsulating siRNA targeting ApoB

SNALP (30:2:20:48:DLinDMA:PEG-cDMA:DSPC:Chol) encapsulating siRNA targeting ApoB and having the sequences shown in Table 2 were administered to female Balb/C mice at 2.5 mg siRNA/kg.

**Table 2**

| **siRNA** **Identifier** | **Designation** | **Target Sequence** **(5'-3' sense strand)** | **Overhang** |
|---|---|---|---|
| A | ApoB-148 | GAA GAU GCA ACU CGA UUC A | dTdT |
| B | ApoB-911 | ACA GUC GCU UCU UCA GUG A | dTdT |
| C | ApoB-1455 | UGA AUG CAC GGG CAA UGA A | dTdT |
| D | ApoB-3050 | CGG GAG AAG UGG AGC AGU A | dTdT |
| E | ApoB-3193 | AGA AGC AGG ACC UUA UCU A | dTdT |
| F | ApoB-3699 | GGA CAU GGG UUC CAA AUU A | dTdT |
| G | ApoB-5490 | GAA UGU GGG UGG CAA CUU U | dTdT |
| H | ApoB-6134 | UUA AUG GCU UAG AGG UAA A | dTdT |

Plasma IFN-α was measured 6 hours after administration of the SNALP using methods known in the art. The results are shown in Figure 1.

### Example 3: Production of Type I Interferons and Inflammatory Cytokines Following Contact with SNALP encapsulating siRNA targeting ApoB

SNALP (30:2:20:48:DLinDMA:PEG-cDMA:DSPC:Chol) encapsulating siRNA targeting ApoB and having the sequences shown in Table 1 were incubated with naïve human PBMC. siRNA was present in the culture at either 0.3 µg/ ml or 1.0 µg/ ml. IFN-α in the culture media was measured after an overnight culture using methods known in the art. The results are shown in Figure 2.

### Example 4: In vitro silencing of ApoB Expression

SNALP (30:2:20:48::=DLinDMA:PEG-cDMA:DSPC:Chol) encapsulating 0.93 µg. ml siRNA targeting ApoB and having the sequences shown in Table 2 were incubated with human AML12 cells. ApoB expression was measured 40 hours following contacting the cells with SNALP. As shown in Figure 3, siRNA of sequence A reduced ApoB expression to 59% of the control samples, siRNA of sequence B reduced ApoB expression to 69% of the control samples, siRNA of sequence C reduced ApoB expression to 66% of the control samples, siRNA of sequence D reduced ApoB expression to 56% of the control samples, siRNA of sequence E reduced ApoB expression to 42% of the control samples, siRNA of sequence F reduced ApoB expression to 67% of the control samples, siRNA of sequence G reduced ApoB expression to 73% of the control samples, siRNA of sequence H reduced ApoB expression to 87% of the control samples.

### Example 5: In vivo silencing of ApoB Expression

SNALP (30:2:20:48:DLinDMA:PEG-cDMA:DSPC:Chol) encapsulating siRNA targeting ApoB and having Sequences D, E, and F as shown in Table 2 were administered to female Balb/C mice at 2.5 mg siRNA (0.833 mg per siRNA sequence)/kg, once daily for 3 days. ApoB expression was measured 48 hours following administration of SNALP. As shown in Figure 4, the encapsulated siRNA reduced ApoB expression by 54%.

### Example 6: In vivo silencing of ApoB Expression using multiple SNALP doses

A female Balb/c mouse model was used to demonstrate the efficacy of a SNALP formulation designed for siRNA delivery to the liver. This study demonstrated SNALP-mediated anti-ApoB activity with regards to dose response and duration of target knockdown in liver ApoB mRNA as well as biologically related parameters such as circulating ApoB protein and total cholesterol in peripheral blood.

A "2:30:20" (DSPC:Cholesterol:PEG-C-DMA:DLinDMA, 20:48:2:30 % molar composition) SNALP formulation was prepared using a Direct Dilution process. SNALP containing either apob-1 or apob-1-mismatch siRNA were prepared at 0.5, 0.25 or 0.125 mg siRNA/ml for administration.

The siRNA sequences were as follows:

| siRNA Duplex Name | Oligo Strands | Nucleotide Sequence ('5-3') |
|---|---|---|
| apob-1 | sense | GUCAUCACACUGAAUACCAAU |
| apob-1 | antisense | AUUGGUAUUCAGUGUGAUGACAC |
| apob-1-mismatch | sense | GUGAUCAGACUCAAUACGAAU |
| apob-1-mismatch | antisense | AUUCGUAUUGAGUCUGAUCACAC |

| | | |
|---|---|---|
| Note apob-1-mismatch is also referred to as "mismatch". | | |

Balb/c mice (female, 4 weeks old) were obtained from Harlan Labs. After an acclimation period (of at least 7 days), animals were administered SNALP by intravenous (IV) injection through the tail vein once daily on Study Days 0, 1 & 2 (3 doses total per animal). Dosages were 5, 2.5 or 1.25 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0, 1, 2 Drug Dose** | **Sacrifice Time Point** |
|---|---|---|---|---|---|
| 1 | 5 | PBS Vehicle | | 10 ml/kg | Day 4 (48 h) |
| 2 | | apob-1 | 2:30:20 SNALP | 5 mg/kg | |
| 3 | | | | 2.5 mg/kg | |
| 4 | | | | 1.25 mg/kg | |
| | | | | 5 mg/kg | Day 3 (24 h) |
| 6 | | | | | Day 5 (72 h) |
| 7 | | | | | Day 7 (120 h) |
| 8 | | apob-1-mismatch | | 5 mg/kg | Day 4 (48 h) |
| 9 | | | | 2.5 mg/kg (48 h) | |
| 10 | | | | 1.25 mg/kg | |
| 11 | | | | 5 mg/kg | Day 3 (24 h) |
| 12 | | | | | Day 5 (72 h) |
| 13 | | | | | Day 7 (120 h) |

Body weights were measured daily, and cageside observations of animal behaviour and/or appearance were recorded daily. Animals were sacrificed on Day 3, 4, 5 or 7 (*i.e*., 24-120 hours after the third and last administration of test article.

Animals were euthanized with a lethal dose of ketamine/xylazine and blood was collected via cardiac puncture prior to cervical dislocation. Blood was split in a lavendar EDTA microtainer (for plasma) and a SST microtainer (for serum). The liver was removed whole, weighed, and immersed in at least 5 volumes of RNAlater. One lobe of some livers (2 animals of each group) was removed before RNAlater immersion and frozen in O.C.T. (Tissue-Tek 4583) over liquid nitrogen.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Tissue sections were prepared from frozen liver lobes and stained with haematoxylin and eosin for standard histological analysis or stained with Oil-Red-O and haematoxylin for detection of lipids.

As shown in Figure 5, downregulation of ApoB mRNA in the liver was observed from a dosage level as low as 1.25 mg/kg (per injection) at 48 hours after the last injection. As shown in Figure 5, treatment with the 5 mg/kg dosage led to a decrease in ApoB expression in terms of liver mRNA of as much as 88%. This silencing was observed as soon as 24 hours and continued without much lessening of effect to 120 hours after the last SNALP administration. Reductions in ApoB protein levels in plasma (up to 91% decrease) corresponded to observed patterns of reduction in liver mRNA. Silencing of ApoB was expected to have additional biologicial consequences and these were measured in the form of lowered serum cholesterol levels (up to 64% decrease) and occurrence of fatty liver as detected by liver weight and appearance as well as Oil-Red-O staining of liver sections for lipid deposits.

### Example 7: In vivo silencing of ApoB Expression Using Multiple SNALP Doses

A female Balb/c mouse model was used to demonstrate the efficacy of a SNALP formulation designed for siRNA delivery to the liver. This study demonstrated SNALP-mediated anti-ApoB activity with regards to duration of target knockdown in circulating ApoB protein as well as biologically related parameters such as ApoB mRNA in liver and total cholesterol in peripheral blood.

A "2:30:20" (DSPC:Cholesterol:PEG-C-DMA:DLinDMA, 20:48:2:30 % molar composition) SNALP formulation was prepared using a Direct Dilution process. SNALP containing either apob-1 or apob-1-mismatch siRNA were prepared at 0.5 mg siRNA/ml for administration. Liposomes of the same lipid formulation but not containing siRNA (also referred to as "empty particles") were prepared at a lipid concentration equivalent to siRNA-containing SNALPs.

The "apob-1" and "apob-1-mismatch" (also referred to as "mismatch") siRNA sequences were as described in Example 6.

Balb/c mice (female, 6 weeks old) were obtained from Harlan Labs. After an acclimation period (of at least 7 days), animals were administered SNALP by IV injection through the tail vein once daily on Study Days 0, 1 & 2 (3 doses total per animal). Dosages were 5 mg siRNA per kg body weight, corresponding to 10 ml/kg (rounded to the nearest 10 microlitres). As a control, one group of animals was administered PBS vehicle.

| **Group** | **# Mice** | **Test Article** | **Day 0, 1, & 2** **Drug Dose** | **Sample Collection** |
|---|---|---|---|---|
| 1 | 5 | PBS Vehicle | 10 ml/kg | Tail nick on Day -4, 3, 4, 5, 7, 10,14 & 17. Euth on Day 21 for liver and blood. |
| 2 | | apob-1 2:30:20 SNALP | 5 mg/kg | |
| 3 | | mismatch 2:30:20 SNALP | | |
| 4 | | Empty particles | equiv. [lipid] | |

Body weights were measured on each day of injection and each day that samples were collected, and cageside observations of animal behaviour and/or appearance were recorded at the same time. Animals were sacrificed on Day 21, 19 days after the third and last administration of test article.

Animals were euthanized with a lethal dose of ketamine/xylazine and blood was collected via cardiac puncture prior to cervical dislocation. Blood was split in a lavendar EDTA microtainer (for plasma) and a SST microtainer (for serum). The liver was removed whole, weighed, and immersed in at least 5 volumes of RNAlater. One lobe of some livers (2 animals of each group) was removed before RNAlater immersion and frozen in O.C.T. (Tissue-Tek 4583) over liquid nitrogen.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Tissue sections were prepared from frozen liver lobes and stained with haematoxylin and eosin for standard histological analysis or stained with Oil-Red-O and haematoxylin for detection of lipids.

Serum cholesterol levels of mice given apob-1 SNALP were observed to have returned to baseline levels within 15 days of the cessation of treatment. As shown in Figure 6, decreased ApoB protein levels in plasma were detected through to 19 days after administration of the final dose of SNALP. The small measured decrease in ApoB protein (13%) at 19 days after SNALP administration was correlated to a similar small (21%) decrease in the corresponding ApoB liver mRNA.

### Example 8: In vivo silencing of ApoB Expression using SNALP prepared via a Stepwise Dilution Process

A female Balb/c mouse model was used to demonstrate the efficacy of a SNALP formulation designed for siRNA delivery to the liver. This study demonstrated SNALP-mediated anti-ApoB activity with regards to target knockdown in liver ApoB mRNA as well as biologically related parameters such as circulating ApoB protein and total cholesterol in peripheral blood.

SNALP containing apob-1 siRNA were prepared at 0.5 mg siRNA/ml for administration. A "2:30:20 +10%DODAC" (DSPC:Cholesterol:PEG-C-DMA:DLinDMA:DODAC, 20:38:2:30:10 % molar composition) SNALP formulation was prepared using a Stepwise Dilution process. Similarly, "5:30:20" (DSPC:Cholesterol:PEG-C-DMA:DLinDMA:DODAC, 20:45:5:30 % molar composition), a "2:30:20 DODMA" (DSPC:Cholesterol:PEG-C-DMA:DODMA, 20:48:2:30 % molar composition) and a "2:30:10" (DSPC:Cholesterol:PEG-C-DMA:DLinDMA, 10:58:2:30 % molar composition) SNALP formulations were prepared.

The "apob-1" siRNA sequences were as described in Example 6.

Balb/c mice (female, 4 weeks old) were obtained from Harlan Labs. After an acclimation period (of at least 7 days), animals were administered SNALP by IV injection through the tail vein once daily on Study Days 0, 1 & 2 (3 doses total per animal). Dosages were 5 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0, 1, & 2** **Drug Dose** | **Sample Collection** |
|---|---|---|---|---|---|
| 1 | 5 | PBS vehicle | | 10 mg/kg | Tail nick at Hour 6. Euth at Day 4 for blood & liver. |
| 2 | | apob-1 | 2:30:20+10%DODAC | 5 mg/kg | |
| 3 | | | 5:30:20 | | |
| 4 | | | 2:30:20 DODMA | | |
| 5 | | | 2:30:10 | | |

Body weights were measured daily, and cageside observations of animal behaviour and/or appearance were recorded at the same time. Animals were sacrificed on Day 4, 48 hours after the third and last administration of test article.

Animals were euthanized with a lethal dose of ketamine/xylazine and blood was collected via cardiac puncture prior to cervical dislocation. Blood was split in a lavendar EDTA microtainer (for plasma) and a SST microtainer (for serum). The liver was removed whole, weighed, and immersed in at least 5 volumes of RNAlater.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Interferon-α in plasma and/or serum was measured using a commercially available ELISA kit (PBL Biomedical Laboratories, USA) according to the manufacturer's instructions.

As shown in Figure 7, downregulation of ApoB mRNA in the liver was observed upon treatment with all four formulations but ranged from 99% decrease to 44% decrease. Reductions in ApoB protein levels in plasma (79, 76, 23, 75 % decrease, respectively) roughly corresponded to observed patterns of reduction in liver mRNA. Silencing of ApoB was expected to have additional biologicial consequences and these were measured in the form of lowered serum cholesterol levels (relative difference in decrease more similar to mRNA than to pattern of protein reduction).

### Example 9: In vivo silencing of ApoB Expression Using Multiple Doses of SNALP Comprising Different Cationic Lipids

A female Balb/c mouse model was used to demonstrate the efficacy of SNALP formulations, containing various cationic lipids, that are designed for siRNA delivery to the liver. This study demonstrated SNALP-mediated anti-ApoB activity with regards to target knockdown in liver ApoB mRNA as well as biologically related parameters such as circulating ApoB protein and total cholesterol in peripheral blood.

"2:30:20 DODMA" (DSPC:Cholesterol:PEG-C-DMA:DODMA, 20:48:2:30 % molar composition) and "2:30:20 DLenDMA" (DSPC:Cholesterol:PEG-C-DMA:DLenDMA, 20:48:2:30 % molar composition) SNALP formulations were prepared using a Direct Dilution process. SNALP containing either apob-1 or apob-1-mismatch siRNA were prepared at 0.5 mg siRNA/ml for administration.

The "apob-1" and "apob-1-mismatch" (also referred to as "mismatch") siRNA sequences were as described in Example 6.

Balb/c mice (female, 4 weeks old) were obtained from Harlan Labs. After an acclimation period (of at least 7 days), animals were administered SNALP by IV injection through the tail vein once daily on Study Days 0, 1 & 2 (3 doses total per animal). Dosages were 5 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0, 1, 2** **Drug Dose** | **Day 4** **Sacrifice** |
|---|---|---|---|---|---|
| 1 | | PBS vehicle | | 10 ml/kg | Collect liver & blood. |
| 2 | | apob-1 | 2:30:20 DODMA | 5 mg/kg | |
| 3 | | | 2:30:20 DLenDMA | | |
| 4 | | apob-1-mismatch | 2:30:20 DODMA, | | |
| 5 | | | 2:30:20 DLenDMA | | |

Body weights were measured every day, and cageside observations of animal behaviour and/or appearance were recorded at the same time. Animals were sacrificed on Study Day 4, 48 hours after the third and last administration of test article.

Animals were euthanized with a lethal dose of ketamine/xylazine and blood was collected via cardiac puncture prior to cervical dislocation. Blood was split in a lavender EDTA microtainer (for plasma) and a SST microtainer (for serum). The liver was removed whole, weighed, and immersed in at least 5 volumes of RNAlater.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA).

As shown in Figure 8, downregulation of ApoB mRNA in the liver was observed upon treatment with formulations containing either cationic lipid: 79% silencing with DODMA and 71% silencing with DLenDMA. Reductions in ApoB protein levels in plasma (72 and 52 % decrease, respectively) roughly corresponded to observed patterns of reduction in liver mRNA. Silencing of ApoB was expected to have additional biologicial consequences and these were measured in the form of lowered serum cholesterol levels (25 and 14% decrease, respectively).

### Example 10: In vivo silencing of ApoB Expression Using Multiple Doses of SNALP Containing Different Phospholipids

A female Balb/c mouse model was used to demonstrate the efficacy of SNALP formulations, containing various phospholipids, that are designed for siRNA delivery to the liver. This study demonstrated SNALP-mediated anti-ApoB activity with regards to target knockdown in liver ApoB mRNA as well as biologically related parameters such as circulating ApoB protein and total cholesterol in peripheral blood.

"2:30:20 DOPE" (DOPE:Cholesterol:PEG-C-DMA:DLinDMA, 20:48:2:30 % molar composition), "2:30:20 DSPE" (DSPE:Cholesterol:PEG-C-DMA:DLinDMA, 20:48:2:30 % molar composition) and "2:30:20 DPPE" (DPPE:Cholesterol:PEG-C-DMA:DLinDMA, 20:48:2:30 % molar composition) SNALP formulations were prepared using a Direct Dilution process. SNALP containing either apob-1 or apob-1-mismatch siRNA were prepared at 0.35 mg siRNA/ml for administration.

The "apob-1" and "apob-1-mismatch" (also referred to as "mismatch") siRNA sequences were as described in Example 6.

Balb/c mice (female, 4 weeks old) were obtained from Harlan Labs. After an acclimation period (of at least 7 days), animals were administered SNALP by IV injection through the tail vein once daily on Study Days 0, 1 & 2 (3 doses total per animal). Dosages were 3.5 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0, 1, 2** **Drug Dose** | **Day 3** **Collection** |
|---|---|---|---|---|---|
| 1 | 4 | PBS vehicle | | 10 ml/kg | Collect liver and blood. |
| 2 | 4 | apob-1 | 2:30:20 DOPE | 3.5 mg/kg | |
| 3 | 4 | | 2:30:20 DSPE | | |
| 4 | 4 | | 2:30:20 DPPE | | |
| 5 | 3 | apob-1-mismatch | 2:30:20 DOPE | | |
| 6 | 3 | | 2:30:20 DSPE | | |
| 7 | 3 | | 2:30:20 DPPE | | |

Body weights were measured each day, and cageside observations of animal behaviour and/or appearance were recorded at the same time. Animals were sacrificed on Study Day 3, 24 hours after the third and last administration of test article.

Animals were euthanized with a lethal dose of ketamine/xylazine and blood was collected via cardiac puncture prior to cervical dislocation. Blood from each animal was collected into a lavender EDTA microtainer (for plasma). The spleen was removed and weighed. The liver was removed whole, weighed, and immersed in at least 5 volumes of RNAlater.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA).

As shown in Figure 9, downregulation of ApoB mRNA in the liver was observed upon treatment with formulations containing any of the phospholipids: 94% silencing with DOPE, 87% silencing with DSPE and 90% silencing with DPPE. The considerable degree of 'non-specific effect', which was correlated to the SNALP dosage but not the action of the active apob-1 siRNA, was not unexpected as similar effects have been observed at this time point in other studies (*see, e.g*., Examples 21 and 22) and are known to be transient. Reductions in ApoB protein levels in plasma were not quantified as samples fell below the lower limit (13%) of the assay. Silencing of ApoB was expected to have additional biological consequences and these were measured in the form of lowered serum cholesterol levels (30, 31 and 40% decrease, respectively).

### Example 11: In vivo silencing of ApoB Expression Using a Single SNALP Dose

A female Balb/c mouse model was used to demonstrate the efficacy of a SNALP formulation designed for siRNA delivery to the liver. This study demonstrated SNALP-mediated anti-ApoB activity with regards to dose response and duration of target knockdown in in circulating ApoB protein as well as biologically related parameters such as ApoB mRNA in liver and total cholesterol in peripheral blood..

A "2:30:20" (DSPC:Cholesterol:PEG-C-DMA:DLinDMA, 20:48:2:30 % molar composition) SNALP formulation was prepared using a Direct Dilution process. SNALP containing either apob-1 or apob-1-mismatch siRNA were prepared at 0.5 mg siRNA/ml for administration.

The apob-1 and apob-1-mismatch (also referred to as "mismatch") siRNA sequences were as described in Example 6.

Balb/c mice (female, 4 weeks old) were obtained from Harlan Labs. After an acclimation period (of at least 7 days), animals were administered SNALP by IV injection through the tail vein once on Study Day 0 (1 dose total per animal). Dosage was 5 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0** **Drug** **Dose** | **Sacrifice** **Time** **Point** |
|---|---|---|---|---|---|
| 1 | 4 | PBS vehicle | | 10 ml/kg | Day 10 |
| 2 | | apoB-1 apob-1 | 2:30:20 SNALP | 5 mg/kg | Day 1 |
| 3 | | | | | Day 3 |
| 4 | | | | | Day 7 |
| 5 | | | | | Day 10 |
| 6 | | apob-1-mismatch | | | Day 1 |
| 7 | | | | | Day 3 |
| 8 | | | | | Day 7 |
| 9 | | | | | Day 10 |

Body weights were measured on the day of injection and each day that samples were collected, and cageside observations of animal behaviour and/or appearance were recorded at the same time. Animals were sacrificed 1, 3, 7 and 10 days after test article administration.

Animals were euthanized with a lethal dose of ketamine/xylazine and blood was collected via cardiac puncture prior to cervical dislocation. Blood was split in a lavender EDTA microtainer (for plasma) and a SST microtainer (for serum). The liver was removed whole, weighed, and immersed in at least 5 volumes of RNAlater. One lobe of some livers (2 animals of each group) was removed before RNAlater immersion and frozen in O.C.T. (Tissue-Tek 4583) over liquid nitrogen.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Tissue sections were prepared from frozen liver lobes and stained with haematoxylin and eosin for standard histological analysis or stained with Oil-Red-O and haematoxylin for detection of lipids.

As shown in Figure 10, SNALP-mediated downregulation of ApoB protein in plasma was observed to have the greatest effect (84% decrease) at 1 day after administration. This silencing effect gradually lessened over the duration of the study period, to as low as 13% decrease at 10 days after SNALP administration. A transient 'non-specific' effect, which was correlated to the SNALP dosage but not the action of the active apob-1 siRNA, was observed at Day 1 but this was essentially abolished by Day 3, at which time the specific activity of active SNALP resulted in a 49% decrease in the plasma ApoB protein level. Reductions in ApoB liver mRNA (up to 72% decrease) corresponded to observed patterns of reduction in plasma ApoB protein. Silencing of ApoB was expected to have additional biologicial consequences and these were measured in the form of lowered serum cholesterol levels (up to 24% decrease at Day 3, resolved by Day 10) and occurrence of fatty liver as detected by liver weight and appearance as well as Oil-Red-O staining of liver sections for lipid deposits.

### Example 12: In vivo silencing of ApoB Expression Using a Single SNALP Dose

A female Balb/c mouse model was used to demonstrate the efficacy of a SNALP formulation designed for siRNA delivery to the liver. This study was performed for method development (use of tail nicks to assay silencing at multiple time points, allowing for a decrease in the number of animals utilized) and demonstrated SNALP-mediated anti-ApoB activity with regards to duration of target knockdown in circulating ApoB protein as well as biologically related parameters such as ApoB mRNA in liver and total cholesterol in peripheral blood.

A "2:30:20" (DSPC:Cholesterol:PEG-C-DMA:DLinDMA, 20:48:2:30 % molar composition) SNALP formulation was prepared using a Direct Dilution process. SNALP containing either apob-1 or apob-1-mismatch siRNA were prepared at 0.5 mg siRNA/ml for administration.

The "apob-1" and "apob-1-mismatch" (also referred to as "mismatch") siRNA sequences were as described in Example 6.

Balb/c mice (female, 4 weeks old) were obtained from Harlan Labs. After an acclimation period (of at least 7 days), animals were administered SNALP by IV injection through the tail vein once on Study Day 0 (1 dose total per animal). Dosage was 5 mg siRNA per kg body weight, corresponding to 10 ml/kg (rounded to the nearest 10 microlitres). As a control, one group of animals was administered PBS vehicle.

| **Group** | # **Mice** | **Test Article** | | **Day 0** **Drug** **Dose** | **Sample Collection** |
|---|---|---|---|---|---|
| 1 | 4 | PBS vehicle | | 10 ml/kg | Tail nicks at Hour 6, Day 1, 2 & 3. Terminal bleed on Day 4 & collect liver. |
| 2 | | apob-1 | 2:30:20 SNALP | 5 mg/kg | |
| 3 | | apob-1-mismatch | | | |

Body weights were measured on the day of injection and each day that samples were collected, and cageside observations of animal behaviour and/or appearance were recorded at the same time. Animals were sacrificed on Day 4, 96 hours after IV administration of test article.

Animals were euthanized with a lethal dose of ketamine/xylazine and blood was collected via cardiac puncture prior to cervical dislocation. Blood was split in a lavender EDTA microtainer (for plasma) and a SST microtainer (for serum). The liver was removed whole, weighed, and immersed in at least 5 volumes of RNAlater. One lobe of some livers (2 animals of each group) was removed before RNAlater immersion and frozen in O.C.T. (Tissue-Tek 4583) over liquid nitrogen.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Tissue sections were prepared from frozen liver lobes and stained with haematoxylin and eosin for standard histological analysis or stained with Oil-Red-O and haematoxylin for detection of lipids. Interferon-alpha in plasma and/or serum was measured using a commercially available ELISA kit (PBL Biomedical Laboratories, USA) according to the manufacturer's instructions.

As shown in Figure 11, SNALP-mediated downregulation of ApoB protein in plasma was observed to have the greatest effect (53 % decrease) at Hour 24 after administration. This silencing effect gradually lessened over the duration of the study period, to as low as 27% decrease at Hour 96 after SNALP administration. A transient 'non-specific' effect, which was correlated to the SNALP dosage but not the action of the active apob-1 siRNA, was observed at Hour 24 but this was essentially abolished by Hour 48, at which time the specific activity of active SNALP resulted in a 34% decrease in the plasma ApoB protein level. At the the sacrifice time point, 96 hours after SNALP administration, reduction in ApoB liver mRNA (30% decrease) corresponded to observed reduction in plasma ApoB protein. Silencing of ApoB was expected to have additional biologicial consequences and these were measured in the form of lowered serum cholesterol levels (21% silencing at Hour 96) and occurrence of fatty liver as detected by liver weight and appearance as well as Oil-Red-O staining of liver sections for lipid deposits.

### Example 13: In vivo silencing of ApoB Expression

A diet-induced high cholesterol mouse model was used to demonstrate the efficacy of liver-targeted anti-ApoB SNALP in lowering total blood cholesterol level. This study demonstrated SNALP-mediated anti-ApoB activity with regards to the extent and duration of the effect of lowering total cholesterol in the blood. Reduction of blood cholesterol is a potentially therapeutic application of SNALP technology.

A "2:40:10" (DSPC:Cholesterol:PEG-C-DMA:DLinDMA, 10:48:2:40 % molar composition) SNALP formulation was prepared using a Direct Dilution process. SNALP containing either apob-1 or apob-1-mismatch siRNA were prepared at 0.5 mg siRNA/ml for administration.

The "apob-1" and "apob-1-mismatch" (also referred to as "mismatch") siRNA sequences were as described in Example 6.

Balb/c and C57BL/6 mice (female, 4 weeks old) were obtained from Harlan Labs. After an acclimation period (of at least 7 days), and after tail nick samples are taken on Study Day 0, animals in selected cages were switched to a high fat diet (a so-called 'Western diet', Harlan Teklad # 88137: 0.2% cholesterol, 4.5 kcal/g, 43% calories derived from fat) which will be supplied *ad libitum* in pellet form. The normal diet was Laboratory Rodent Diet (PMI Nutrition International), containing 12% calories derived from fat and 200 ppm cholesterol, which was supplied in the same manner.

Blood cholesterol levels in animals fed normal versus high fat diet were monitored for four weeks in order to establish a baseline for the hypercholesterolemia model.

Body weights were measured twice per week, and cageside observations of animal behaviour and/or appearance were recorded at the same time. Plasma was collected via tail nick once per week up to Study Day 28.

On Study Day 32, animals were administered SNALP by intravenous (IV) injection through the tail vein once Study Day 0 (1 dose total per animal). Dosage was 5 mg siRNA per kg body weight, corresponding to 10 ml/kg (rounded to the nearest 10 microlitres).

| **Cage** | **# Mice** | **Mouse Strain** | **IV Dose at 5 mg/kg** | | **Sample Collection** |
|---|---|---|---|---|---|
| 1 | 2 | Balb/c Normal Diet | apob-1 | 2:40:10 SNALP | BW 2x/week. Tail Nick 2x/week for cholesterol, ApoB protein. |
| | 2 | | mismatch | | |
| 2 | 2 | Balb/c High Fat Diet | apob-1 | | |
| | 2 | | mismatch | | |
| 3 | 2 | C57BL/6 Normal Diet | apob-1 | | |
| | 2 | | mismatch | | |
| 4 | 2 | C57BL/6 High Fat Diet | apob-1 | | |
| | 2 | | mismatch | | |

Body weights were measured twice per week, and cageside observations of animal behaviour and/or appearance were recorded at the same time. Plasma was collected via tail nick twice per week up to Study Day 39.

Total cholesterol in plasma was measured using an enzymatic method according to manufacturer's instructions (Infinity Cholesterol, Thermo Electron Corp, USA).

As shown in Figure 22, IV administration of a single dose of anti-ApoB SNALP completely abrogated the elevated cholesterol levels previously induced by a high fat 'Western' diet in female C57BL/6 mice. Similar results were obtained using female Balb/c mice

### Example 14: In vivo silencing of ApoB Expression

A female Balb/c mouse model was used to demonstrate the efficacy of a SNALP formulation designed for siRNA delivery to the liver. These studies demonstrated SNALP-mediated anti-ApoB activity with regards to dose response and duration of target knockdown in liver ApoB mRNA as well as biologically related parameters such as circulating ApoB protein and total cholesterol in peripheral blood

A "2:40:10" (DSPC:Cholesterol:PEG-C-DMA:DLinDMA, 10:48:2:40 % molar composition) SNALP formulation was prepared using a Direct Dilution process, at a nucleic acid to lipid ratio of 0.039. SNALP containing either apob-1 or apob-1-mismatch siRNA were prepared at 0.2 mg siRNA/ml for administration.

The "apob-1" and "apob-1-mismatch" (also referred to as "mismatch") siRNA sequences were as described in Example 6.

Balb/c mice (female, 4 weeks old) were obtained from Harlan Labs. After an acclimation period (of at least 7 days), animals were administered SNALP by intravenous (IV) injection through the tail vein once on Study Day 0 (1 dose total per animal). Dosage was 2 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0** **Drug Dose** | **Sample Collection** |
|---|---|---|---|---|---|
| 1 | 4 | PBS vehicle | | 10 ml/kg | Tail nick at Day 1, 2 & 3. Euth at Day 4 for blood & liver. |
| 2 | 4 | apob-1 | 2:40:10 SNALP | 2 mg/kg | |
| 3 | 3 | apob-1-mismatch | | | |

Body weights were measured daily, and cageside observations of animal behaviour and/or appearance were recorded daily. Animals were sacrificed on Day 4, 96 hours after administration of test article.

Animals were euthanized with a lethal dose of ketamine/xylazine and blood was collected via cardiac puncture prior to cervical dislocation. Blood was split in a lavendar EDTA microtainer (for plasma) and a SST microtainer (for serum). The liver was removed whole, weighed, and immersed in at least 5 volumes of RNAlater.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA).

As shown in Figure 13, downregulation of ApoB mRNA in the liver was observed at 96 hours after a single injection of SNALP at a dosage of 2 mg/kg. Silencing of ApoB was expected to have additional biologicial consequences and these were measured in the form of lowered serum cholesterol levels (up to 59% decrease) and occurrence of fatty liver as detected by liver weight.

### Example 15: In vivo silencing of ApoB Expression Following Intraperitoneal Administration of SNALP

A female Balb/c mouse model was used to demonstrate the efficacy of SNALP formulation administerered intraperitoneally.

A "2:40:10" (DSPC:Cholesterol:PEG-C-DMA:DLinDMA, 10:48:2:40 % molar composition) SNALP formulation was prepared using a Direct Dilution process, at a nucleic acid to lipid ratio of 0.0195. SNALP containing either apob-1 or apob-1-mismatch siRNA were prepared at 0.2 mg siRNA/ml for administration.

The "apob-1" and "apob-1-mismatch" (also referred to as "mismatch") siRNA sequences were as described in Example 6.

Balb/c mice (female, 4 weeks old) were obtained from Harlan Labs. After an acclimation period (of at least 7 days), animals were administered SNALP by intraperitoneal (IP) injection in the abdominal region once daily on Study Days 0,1 & 2 (3 doses total per animal). Dosage was 2 mg siRNA per kg body weight, corresponding to 10 ml/kg (rounded to the nearest 10 microlitres). As a control, one group of animals was given an intravenous (IV) injection of PBS vehicle.

| **Group** | **# Mice** | **Test Article** | | **Dose Regime** | **Day 4** **Sacrifice** |
|---|---|---|---|---|---|
| 1 | 4 | PBS vehicle | | IV Day 0 | Collect plasma & liver. |
| 2 | 4 | apob-1 | 2:40:10 SNALP 2 mg/kg per dose | IP Days 0, 1 & 2 | |
| 3 | 3 | mismatch | | | |

Body weights were measured daily, and cageside observations of animal behaviour and/or appearance were recorded daily. Animals were sacrificed on Day 4, 48 hours after the final administration of test article.

Animals were euthanized with a lethal dose of ketamine/xylazine and blood was collected via cardiac puncture prior to cervical dislocation. Blood was collected in lavendar EDTA microtainer and processed for plasma. The liver was removed whole, weighed, and immersed in at least 5 volumes of RNAlater.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84).

As shown in Figure 14, downregulation of ApoB mRNA in the liver was observed at 48 hours after the third injection of SNALP and this downregulation effect was observed in both ApoB mRNA and ApoB protein. The use of a negative control treatment, consisting of SNALP containing siRNA that do not target the ApoB gene, demonstrates that the observed downregulation effect is specific to a formulation that contains siRNA designed to act against the target gene.

### Example 16: In vivo silencing of ApoB Expression Following Subcutaneous Administration of SNALP

A female Balb/c mouse model was used to demonstrate the efficacy of SNALP administerered subcutaneously.

A "2:40:10" (DSPC:Cholesterol:PEG-C-DMA:DLinDMA, 10:48:2:40 % molar composition) SNALP formulation was prepared using a Direct Dilution process, at a nucleic acid to lipid ratio of 0.0195. SNALP containing either apob-1 or apob-1-mismatch siRNA were prepared at either 0.1, 0.3 or 1.0 mg siRNA/ml for administration.

The "apob-1" and "apob-1-mismatch" (also referred to as "mismatch") siRNA sequences were as described in Example 6, except that all uridine residues in each sense strand carried a 2'-O-methyl modification (referred to below as "UmodS").

Balb/c mice (female, 4 weeks old) were obtained from Harlan Labs. After an acclimation period (of at least 7 days), animals were administered SNALP by subcutaneous (subQ) injection in the scapular region once on Study Day 0 (1 dose total per animal). Dosage was 1, 3 or 10 mg siRNA per kg body weight, corresponding to 10 ml/kg (rounded to the nearest 10 microlitres). As a control, one group of animals was given an intravenous (IV) injection of PBS vehicle.

| **Group** | **# Mice** | **Test Article** | | **Day 0** **Dose** | **Sample** **Collection** |
|---|---|---|---|---|---|
| 1 | 4 | PBS vehicle | | IV 10 mL/kg | Euthanize on Day 2. Collect Liver. |
| 2 | 5 | 2:40:10 Direct Dilution SNALP | apob-1 UmodS | subQ 1 mg/kg | |
| 3 | 5 | | apob-1 UmodS | subQ 3 mg/kg | |
| 4 | 5 | | apob-1 UmodS | subQ 10 mg/kg | |
| 5 | 5 | | apob-1-MMUmodS | subQ 3 mg/kg | |

Body weights were measured daily, and cageside observations of animal behaviour and/or appearance were recorded daily. Animals were sacrificed on Day 2, 48 hours after administration of test article.

Animals were euthanized with a lethal dose of ketamine/xylazine and the liver was removed whole, weighed, and immersed in at least 5 volumes of RNAlater. ApoB and GAPDH mRNA levels in liver were measured using a QuantiGene assay kit (Genospectra, USA) according to the manufacturer's instructions.

As shown in Figure 15, downregulation of ApoB mRNA in the liver was observed at 48 hours after a single injection of SNALP and this downregulation effect increased with the administration of greater dosages (up to 10 mg/kg). The use of a negative control treatment, consisting of SNALP containing siRNA that do not target the ApoB gene, demonstrates that the observed downregulation effect is specific to a formulation that contains siRNA designed to act against the target gene.

### Example 17: In vivo silencing of ApoB Expression Using SNALP Encapsulating anti-APoB siRNA

A female Balb/c mouse model was used to demonstrate the relative efficacy of a panel of SNALP encapsulating anti-ApoB siRNA.

A panel of siRNA sequences was generated by scanning the murine ApoB sequence (XM 137955) using the rules described in Example 1 above. Table 3 sets forth the sequence, position, and predicted immunostimulatory activity of each identified siRNA sequence.

**Table 3**

| Position | SiRNA target sequence | Immunostimulatory activity |
|---|---|---|
| 1512 | GAAGAACCAUGGAACAAGU | High |
| 2688 | GCAUCAUCAUCCCAGACUU | Low |
| 10849 | CCAUCACUUUGACCAGGAA | Med |
| 12190 | GGAAUACGUUUCUUCAGAA | Med |
| 13395 | CCACAAGAUUGAUUGACCU | High |

A "2:40:10" (DSPC:Cholesterol:PEG-C-DMA:DLinDMA, 10:48:2:40 % molar composition) SNALP formulation was prepared using a Direct Dilution process. SNALP containing the ApoB siRNA set forth in Table 4 were prepared at 0.2 mg siRNA/ml for administration. The "apob-1" and "apob-1-mismatch" (also referred to as "mismatch") siRNA sequences were as described in Example 6. "Protiva apoB-1" and "Protiva apob-1 mismatch" have the same sequences as the siRNA sequences described in Example 6, but were produced from different manufacturing lots. UmodS was as described in Example 16 above. The notation "no phosphate" indicates that the siRNA lacks a terminal phosphate.

Balb/c mice (female, 4 weeks old) were obtained from Harlan Labs. After an acclimation period (of at least 7 days), animals were administered SNALP by intravenous (IV) injection through the tail vein once daily on Study Day 0. Dosage was 2 mg siRNA per kg body weight, corresponding to 10 ml/kg (rounded to the nearest 10 microlitres). As a control, one group of animals was administered PBS vehicle.

| **Group** | **# Mice** | **Test Article** | | **Day 0 IV** **Drug Dose** | **Sample Collection** | **Test Article Lot No.** |
|---|---|---|---|---|---|---|
| 1 | 4 | PBS vehicle | | 10 mL/kg | | N/A |
| 2 | 4 | apob-1 | :40:10 1xD:L NALP2 | 2 mg/kg | Hour 6 tail nick for plasma. Hour 48 of collection of liver in RNAlater and plasma. | 242-072005-01 |
| 3 | 4 | apob-1 no phosphate | | | | 242-080405-06 |
| 4 | 4 | apob-1 U-mod-sense | | | | 242-072505-01 |
| 5 | 4 | apoB-1514(*i.e.,* 1512) | | | | 242-080405-01 |
| 6 | 4 | apoB-2690 (*i.e.,* 2688) | | | | 242-080405-02 |
| 7 | 4 | apoB-10851 (*i.e.,* 10849) | | | | 242-080405-03 |
| 8 | 4 | apoB-12192 (*i.e.,* 12190) | | | | 242-080405-04 |
| 9 | 4 | apoB-13397 (*i.e.,* 13395) | | | | 242-080405-05 |
| 10 | 4 | apob-1-mismatch | | | | 233-061505-05 |
| 11 | 4 | Protiva apob-1 | | | | 242-080405-07 |
| 12 | 4 | Protiva apob-1 no phosphate | | | | 242-080405-08 |

Body weights were measured daily, and cageside observations of animal behaviour and/or appearance were recorded daily. Animals were sacrificed on Day 3, 48h after the single dose adminstration.

Animals were euthanized with a lethal dose of ketamine/xylazine and blood was collected via cardiac puncture prior to cervical dislocation. Blood was collected in a lavendar EDTA microtainer (for plasma). The liver was removed whole, weighed, and immersed in at least 5 volumes of RNAlater. Spleens were removed whole and weighed.

ApoB and GAPDH mRNA levels in liver were measured using a QuantiGene assay kit (Genospectra, USA) according to the manufacturer's instructions. ApoB protein levels in plasma and/or serum were measured using an ELISA method essentially as described by Zlot et al. (Journal of Lipid Research, 1999, 40:76-84). Total cholesterol in plasma and/or serum was measured using an enzymatic method according to manufacturer's instructions (Infinity Cholesterol, Thermo Electron Corp, USA). Interferon-alpha levels in plasma were measured using a sandwich ELISA method according to manufacturer's instructions (Mouse Interferon-α, PBL Biomedical, Piscataway, NJ).

*Silencing efficacy of newly designed apoB siRNA:* As shown in Figures 16 and 17, downregulation of ApoB in the mouse was observed at the 2 mg/kg dosage at 48 hours after dosing. Downregulation of apoB by the newly designed siRNA was achieved to the greatest extent with apoB-12192 (liver mRNA-54% decrease, plasma protein- 35% decrease). Silencing of ApoB was expected to have additional biologicial consequences and these were measured in the form of lowered serum cholesterol levels (15% decrease with apoB-12192).

*Immunostimulatory activity of newly designed apoB siRNA:* Scoring of the newly designed apoB siRNA for the presence or absence of putative immunostimulatory motifs indicated that an absence of any such motifs correlated with a lack of induction of interferon-α release at 6 h in mouse plasma (*see,* Figure 18).

### Example 18: In vitro silencing of ApoB Expression Using SNALP Encapsulating anti-APoB siRNA

A panel of apoB siRNA were screened in vitro using HepG2 cells to assess their efficacy in silencing ApoB gene expression. Downregulation of secreted apoB protein was demonstrated with a number of these siRNA, at levels matching or exceeding that of apoB-1.

Candidate Apolipoprotein B sequences were identified using the methods set forth in Example 1 above, by scanning and mouse ApoB (XM_137955) and human ApoB (NM_000384) sequences to identify AA dinucleotide motifs and the 21 nucleotides 3' of the motif. The sequences and their positions are set forth in Table 4 below.

**Table 4**

| Mouse apoB | Human apoB | Sense 23 bp target sequence |
|---|---|---|
| 327 | 428 | AA AGAGGUGUAUGGCUUCAAC CC |
| 328 | 429 | AA GAGGUGUAUGGCUUCAACC CU |
| 330 | 431 | GA GGUGUAUGGCUUCAACCCU GA |
| 1151 | 1252 | CA GCCCCAUCACUUUACAAGC CU |
| 1157 | 1258 | CA UCACUUUACAAGCCUUGGU UC |
| 1167 | 1268 | CA AGCCUUGGUUCAGUGUGGA CA |
| 1989 | 2090 | AA AAUAGAAGGGAAUCUUAUA UU |
| 1990 | 2091 | AA AUAGAGGGAAUCUUAUAU UU |
| 1991 | 2092 | AA UAGAAGGGAAUCUUAUAUU UG |
| 1993 | 2094 | UA GAAGGGAAUCUUAUAUUUG AU |
| 1995 | 2096 | GA AGGGAAUCUUAUAUUUGAU CC |
| 1996 | 2097 | AA GGGAAUCUUAUAUUUGAUC CA |
| 2727 | 2828 | CA GAUGAACACCAACUUCUUC CA |
| 2732 | 2833 | GA ACACCAACUUCUUCCACGA GU |
| 2733 | 2834 | AA CACCAACUUCUUCCACGAG UC |
| 3473 | 3574 | AA UGGACUCAUCUGCUACAGC UU |
| 3475 | 3576 | AA AUGGACUCAUCUGCUACAG CU |
| 3998 | 4099 | CA AGUCUGUGGGAUUCCAUCU GC |
| 3999 | 4100 | AA GUCUGUGGGAUUCCAUCUG CC |
| 4242 | 4343 | CA AGGAUCUGGAGAAACAACA UA |
| 4243 | 4344 | AA GGAUCUGGAGAAACAACAU AU |
| 4246 | 4347 | GA UCUGGAGAAACAACAUAUG AC |
| 6560 | 6664 | GA UACAAUUUGAUCAGUAUAU UA |
| 6564 | 6668 | CA AUUUGAUCAGUAUAUUAAA GA |
| 6565 | 6669 | AA UUUGAUCAGUAUAUUAAAG AU |
| 9098 | 9217 | UA UUGGAACUUUGAAAAAUUC UC |
| 10048 | 10164 | CA AGUGUCAUCACACUGAAUA CC |
| 10049 | 10165 | AA GUGUCAUCACACUGAAUAC CA |
| 10055 | 10171 | CA UCACACUGAAUACCAAUGC UG |
| 10346 | 10462 | UA AUGGAAAUACCAAGUCAAA AC |
| 10347 | 10463 | AA UGGAAAUACCAAGUCAAAA AC |
| 10886 | 11002 | UA ACACUAAGAACCAGAAGAU CA |
| 12093 | 12299 | AA UUGGGAAGAAGAGGCAGCU UC |

HepG2 cells (human hepatocellular carcinoma) were transfected with the murine siRNA sequences using Lipofectamine 2000 (Invitrogen) at a 100 nM dosage at the following ratios: 70 pmol siRNA:1 uL lipofectamine and 20 pmol siRNA:1 uL lipofectamine. Cells were plated on day 0, transfected with complexes on day 1, media was replaced with fresh media on day 2 and supernatants and cells were harvested on day 3 (48h after transfection).

ApoB expression was measured by assaying the supernatants of transfected HepG2 cells for secreted apoB protein using an ELISA method essentially as described by Soutschek et al. (Nature, 2004,432:173-78). Cell lysates were assayed for total protein using the BCA assay (BCA Micro Kit, Pierce). ApoB levels in HepG2 supernatants were normalized to total protein levels.

As shown in Figure 19, downregulation of ApoB in HepG2 cells was observed at the 100 nM dosage at both transfection ratios. Downregulation of apoB by the newly designed siRNA was achieved with a number of the newly designed siRNA at levels matching or exceeding that of apoB-1. These include apob-10048, apob-10049, apob-10346 and apob-10884.

It is to be understood that the above description is intended to be illustrative and not restrictive. Many embodiments will be apparent to those of skill in the art upon reading the above description. The scope of the invention should, therefore, be determined not with reference to the above description, but should instead be determined with reference to the appended claims, along with the full scope of equivalents to which such claims are entitled. The disclosures of all articles and references, including patent applications, patents, PCT publications, and Accession Nos. are incorporated herein by reference for all purposes.

### FURTHER EMBODIMENTS

Item 1. A nucleic acid lipid particle comprising:
   an siRNA molecule that silences Apolipoprotein B (ApoB) expression;
   a cationic lipid; and
   a non-cationic lipid.
Item 2. The nucleic acid-lipid particle in accordance with item 1, wherein said nucleic acid-lipid particle comprises an siRNA molecule comprising a sequence set forth in Table 1, rows A-F of Table 2, and Tables 3-7.
Item 3. The nucleic acid-lipid particle in accordance with item 1, wherein said nucleic acid-particle comprises at least two siRNA molecules, wherein each siRNA molecule comprises a sequence independently selected from the sequences set forth in Table 1, rows A-F of Table 2, and Tables 3-7.
Item 4. The nucleic acid-lipid particle in accordance with item 1, wherein the siRNA silences ApoB expression by at least about 2 fold more than an siRNA that is not in a nucleic acid-lipid particle.
Item 5. The nucleic acid-lipid particle in accordance with item 1, wherein the siRNA silences ApoB expression by at least about 5 fold more than an siRNA that is not in a nucleic acid-lipid particle.
Item 6. The nucleic acid-lipid particle in accordance with item 1, wherein said cationic lipid is a member selected from the group consisting of N,N-dioleyl-N,N-dimethylammonium chloride (DODAC), N,N-distearyl-N,N-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), and N,N-dimethyl-2,3-dioleyloxy)propylamine (DODMA),1,2-DiLinoleyloxy-N,N-dimethylaminopropane (DLinDMA),1,2-Dilinolenyloxy-N,N-dimethylaminopropane (DLendMA), and a mixture thereof.
Item 7. The nucleic acid-lipid particle in accordance with item 1, wherein said cationic lipid is DLinDMA.
Item 8. The nucleic acid-lipid particle in accordance with item 1, wherein said non-cationic lipid is an anionic lipid.
Item 9. The nucleic acid-lipid particle in accordance with item 1, wherein said non-cationic lipid is a neutral lipid.
Item 10. The nucleic acid-lipid particle in accordance with item 1, wherein said non-cationic lipid is a member selected from the group consisting of dioleoylphosphatidylethanolamine (DOPE), palmitoyloleoylphosphatidylcholine (POPC), egg phosphatidylcholine (EPC), distearoylphosphatidylcholine (DSPC), palmitoyloleyolphosphatidylglycerol (POPG), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, palmitoyloleoyl- phosphatidylethanolamine (POPE), 1-stearoyl-2-oleoyl-phosphatidyethanolamine (SOPE), cholesterol, and a mixture thereof.
Item 11. The nucleic acid-lipid particle in accordance with item 1, wherein said non-cationic lipid is DSPC.
Item 12. The nucleic acid-lipid particle in accordance with item 1, further comprising a conjugated lipid that inhibits aggregation of particles
Item 13. The nucleic acid-lipid particle in accordance with item 12, wherein the conjugated lipid that inhibits aggregation of particles is a member selected from the group consisting of: a polyethyleneglycol (PEG)-lipid conjugate, a polyamide (ATTA)-lipid conjugate, and a mixture thereof.
Item 14. The nucleic acid-lipid particle in accordance with item 13, wherein the PEG-lipid conjugate is member selected from the group consisting of: a PEG-- diacylglycerol (PEG-DAG), a PEG dialkyloxypropyl (PEG-DAA), a PEG-phospholipid, a PEG-ceramide (PEG-Cer), and a mixture thereof.
Item 15. The nucleic acid-lipid particle in accordance with item 13, wherein the conjugated lipid that inhibits aggregation of particles comprises a PEG-DAA conjugate.
Item 16. The nucleic acid-lipid particle in accordance with item 15, wherein the PEG-DAA conjugate is a member selected from the group consisting of a PEG-dilauryloxypropyl (C₁₂), a PEG-dimyristyloxypropyl (C₁₄), a PEG-dipalmityloxypropyl (C₁₆), and a PEG-distearyloxypropyl (C₁₈).
Item 17. The nucleic acid-lipid particle in accordance with item 15, wherein said PEG-DAA conjugate is a PEG-dimyristyloxypropyl (C₁₄).
Item 18. The nucleic acid-lipid particle in accordance with item 1, wherein said cationic lipid comprises from about 5 mol % to about 15 mol % of the total lipid present in said particle.
Item 19. The nucleic acid-lipid particle in accordance with item 1, wherein said cationic lipid comprises from about 30 mol % to about 50 mol % of the total lipid present in said particle.
Item 20. The nucleic acid-lipid particle in accordance with item 1, wherein said cationic lipid comprises from about 30 mol % to about 50 mol % of the total lipid present in said particle.
Item 21. The nucleic acid-lipid particle in accordance with item 1, wherein said cationic lipid comprises about 40 mol % of the total lipid present in said particle.
Item 22. The nucleic acid-lipid particle in accordance with item 1, wherein said non-cationic lipid comprises from about 5 mol % to about 90 mol % of the total lipid present in said particle.
Item 23. The nucleic acid-lipid particle in accordance with item 1, wherein said non-cationic lipid comprises from about 20 mol % to about 85 mol % of the total lipid present in said particle.
Item 24. The nucleic acid-lipid particle in accordance with item 15, wherein said PEG-DAA conjugate comprises from 0.5 mol % to about 20 mol % of the total lipid present in said particle.
Item 25. The nucleic acid-lipid particle in accordance with item 15, wherein said PEG-DAA conjugate comprises from 2 mol % to about 15 mol % of the total lipid present in said particle.
Item 26. The nucleic acid-lipid particle in accordance with item 15, wherein said PEG-DAA conjugate comprises about 2 mol % of the total lipid present in said particle.
Item 27. The nucleic acid-lipid particle in accordance with item 1, further comprising cholesterol.
Item 28. The nucleic acid-lipid particle in accordance with item 27, wherein the cholesterol comprises from about 0 mol % to about 10 mol % of the total lipid present in said particle.
Item 29 The nucleic acid-lipid particle in accordance with item 27, wherein the cholesterol comprises from about 10 mol % to about 60 mol % of the total lipid present in said particle.
Item 30. The nucleic acid-lipid particle in accordance with item 27, wherein the cholesterol comprises from about 20 mol % to about 45 mol % of the total lipid present in said particle.
Item 31. The nucleic acid-lipid particle in accordance with item 1, wherein the nucleic acid in said nucleic acid-lipid particle is not substantially degraded after exposure of said particle to a nuclease at 37°C for 20 minutes.
Item 32. The nucleic acid-lipid particle in accordance with item 1, wherein the nucleic acid in said nucleic acid-lipid particle is not substantially degraded after incubation of said particle in serum at 37°C for 30 minutes.
Item 33. The nucleic acid-lipid particle in accordance with item 1, wherein the nucleic acid is fully encapsulated in said nucleic acid-lipid particle.
Item 34. The nucleic acid-lipid particle in accordance with item 1, wherein said particle has a nucleic acid: lipid ratio (mg:mg) of from about 0.01 to about 0.2.
Item 35. The nucleic acid-lipid particle in accordance with item 1, wherein said particle has a nucleic acid: lipid ratio (mg:mg) of from about 0.02 to about 0.1.
Item 36. The nucleic acid-lipid particle in accordance with item 1, wherein said particle has a nucleic acid: lipid ratio (mg:mg) of about 0.04.
Item 37. The nucleic acid-lipid particle in accordance with item 1, wherein wherein said particle has a median diameter of less than about 150 nm.
Item 38. The nucleic acid-lipid particle in accordance with item 1, wherein said particle has a median diameter of less than about 100 nm.
Item 39. A pharmaceutical composition comprising a nucleic acid-lipid particle in accordance with item 1 and a pharmaceutically acceptable carrier.
Item 40. A method of introducing an siRNA that silences ApoB expression into a cell, said method comprising contacting said cell with a nucleic acid-lipid particle comprising a cationic lipid, a non-cationic lipid, and said siRNA.
Item 41. The method of item 40, wherein said nucleic acid-lipid particle further comprises a conjugated lipid that inhibits aggregation of particles.
Item 42. The method of item 40, wherein said nucleic acid-lipid particle comprises an siRNA molecule comprising any one of the sequences set forth in Table 1, rows A-F of Table 2, and Tables 3-7.
Item 43. The method of item 40, wherein said nucleic acid-lipid particle comprises at least two siRNA molecules, wherein each siRNA molecule comprises a sequence independently selected from the sequences set forth in Table 1, rows A-F of Table 2, and Tables 3-7.
Item 44. The method of item 40, wherein said siRNA in said nucleic acid-lipid particle is resistant in aqueous solution to degradation with a nuclease.
Item 45. The method of item 40, wherein said cationic lipid is a member selected from the group consisting of DODAC, DDAB, DOTAP, DOTMA, DODMA, DLinDMA, DLenDMA, and a mixture thereof.
Item 46. The method of item 40, wherein said non-cationic lipid is a member selected from the group consisting of DOPE, POPC, EPC, DSPC, POPG, DPPE, DMPE, DSPE, 16-O-monomethyl PE, 16-O-dimethyl PE, 18-1 -trans PE, POPE, SOPE, cholesterol, and a mixture thereof.
Item 47. The method of item 40, wherein the conjugated lipid that inhibits aggregation of particles comprises a PEG-lipid conjugate selected from the group consisting of: a PEG-DAG, a PEG-DAA, a PEG-phospholipid, a PEG-Cer, and a mixture thereof.
Item 48. The method of item 40, wherein said cell is in a mammal.
Item 49. The method of item 48, wherein said contacting comprises administering said nucleic acid-lipid particle via a route selected from the group consisting of: intravenous, subcutaneous, and intraperitoneal.
Item 50. The method of item 48, wherein the mammal is a human.
Item 51. The method of item 50, wherein said human has a disease or disorder associated with expression of a ApoB and wherein expression of ApoB is silenced by said siRNA.
Item 52. The method of item 50, wherein said disease or disorder is associated with overexpression of ApoB and wherein expression of ApoB is silenced by said siRNA.
Item 53. The method of item 50, wherein said human has a disease or disorder selected from the group consisting of: atherosclerosis, angina pectoris, high blood pressure, diabetes, and hypothyroidism.
Item 54. The method of item 50, wherein said human has a disease or disorder involving hypercholesterolemia and wherein serum cholesterol levels are lowered when expression of ApoB is silenced by said siRNA.
Item 55. The method of item 54, wherein said disease is a member selected from the group consisting of: atherosclerosis, angina pectoris, and high blood pressure.
Item 56. An isolated nucleic acid comprising a sequence set forth in Table 1, rows A-F of Table 2, and Tables 3-7.

## Claims

1. A nucleic acid-lipid particle comprising:
an siRNA molecule that silences Apolipoprotein B (ApoB) expression, wherein said siRNA molecule targets the sequence apoB-10048 set forth in Table 4;
a cationic lipid;
a non-cationic lipid; and
a conjugated lipid that inhibits aggregation of particles.

2. The nucleic acid-lipid particle in accordance with claim 1, wherein
(a) the siRNA molecule is a double-stranded siRNA molecule formed by two complementary strands;
(b) each strand of the siRNA molecule is 21 to 23 nucleotides in length;
(c) the siRNA molecule comprises 3' overhangs of 2 to 3 nucleotides;
(d) the siRNA molecule targets the 19 or 21 nucleotides immediately 3' to the dinucleotide sequence of apoB-10048 set forth in Table 4; and/or
(e) the siRNA molecule comprises a sense strand sequence consisting of nucleotides 3-23 of apoB-10048 set forth in Table 4.

3. The nucleic acid-lipid particle in accordance with claim 1 or 2, wherein said cationic lipid is a member selected from the group consisting of N,N-dioleyl-N,N-dimethylamrnonium chloride (DODAC), N,N-distearyl-N,N-dimethylammonium bromide (DDAB), N-(1-(2,3-dioleoyloxy)propyl)-N,N,N-trimethylammonium chloride (DOTAP), N-(1-(2,3-dioleyloxy)propyl)-N,N,N-trimethylammonium chloride (DOTMA), and N,N-dimethyl-2,3-dioleyloxy)propylamine (DODMA), 1,2-DiLinoleyloxy-N,N-dimethylaminopropane (DLinDMA), 1,2-Dilinolenyloxy-N,N-dimethylaminopropane (DLenDMA), and a mixture thereof.

4. The nucleic acid-lipid particle in accordance with any one of claims 1 to 3, wherein said non-cationic lipid is a member selected from the group consisting of dipalmitoylphosphatidylcholine (DPPC), dioleoylphosphatidylethanolamine (DOPE), palmitoyloleoylphosphatidylcholine (POPC), egg phosphatidylcholine (EPC), distearoylphosphatidylcholine (DSPC), palmitoyloleyolphosphatidylglycerol (POPG), dipalmitoyl phosphatidyl ethanolamine (DPPE), dimyristoylphosphoethanolamine (DMPE), distearoyl-phosphatidyl-ethanolamine (DSPE), 16-O-monomethyl PE, 16-O-dimethyl PE, 18-1-trans PE, palmitoyloleoyl- phosphatidylethanolamine (POPE), 1-stearoyl-2-oleoyl-phosphatidylethanolamine (SOPE), cholesterol, and a mixture thereof.

5. The nucleic acid-lipid particle in accordance with any one of the preceding claims, wherein the conjugated lipid that inhibits aggregation of particles is a member selected from the group consisting of: a polyethyleneglycol (PEG)-lipid conjugate, a polyamide (ATTA)-lipid conjugate, and a mixture thereof, and
wherein the PEG-lipid conjugate is a member selected from the group consisting of: a PEG-diacylglycerol (PEG-DAG), a PEG dialkyloxypropyl (PEG-DAA), a PEG-phospholipid, a PEG-ceramide (PEG-Cer), and a mixture thereof.

6. The nucleic acid-lipid particle in accordance with claim 5, wherein the PEG-DAA conjugate is a member selected from the group consisting of a PEG-dilauryloxypropyl (C₁₂), a PEG-dimyristyloxypropyl (C₁₄), a PEG-dipalmityloxypropyl (C₁₆), and a PEG-distearyloxypropyl (C₁₈).

7. The nucleic acid-lipid particle in accordance with any one of the preceding claims, wherein
(a) said cationic lipid comprises from about 2 mol % to about 60 mol % of the total lipid present in said particle, from about 5 mol % to about 15 mol % of the total lipid present in said particle, from about 30 mol % to about 50 mol % of the total lipid present in said particle, from about 40 mol % to about 50 mol % of the total lipid present in said particle, or about 40 mol % of the total lipid present in said particle;
(b) said non-cationic lipid comprises from about 5 mol % to about 90 mol % of the total lipid present in said particle or from about 20 mol % to about 85 mol % of the total lipid present in said particle; and/or
(c) said conjugated lipid that inhibits aggregation of particles comprises from about 0.5 mol % to about 20 mol % of the total lipid present in said particle, from about 2 mol % to about 15 mol % of the total lipid present in said particle, or about 2 mol % of the total lipid present in said particle.

8. The nucleic acid-lipid particle in accordance with any one of the preceding claims, wherein
(a) said siRNA molecule in said nucleic acid-lipid particle is not substantially degraded after exposure of said particle to a nuclease at 37° C for 20 minutes or after incubation of said particle in serum at 37° C for 30 minutes; and/or
(b) said siRNA molecule is fully encapsulated in said nucleic acid-lipid particle.

9. The nucleic acid-lipid particle in accordance with any one of the preceding claims, wherein
(a) said particle has an siRNA: lipid ratio (mg:mg) of from about 0.01 to about 0.2, of from about 0.02 to about 0.1, or of about 0.04; and/or
(b) said particle has a median diameter of less than about 150 nm or of less than about 100 nm.

10. The nucleic acid-lipid particle in accordance with any one of the preceding claims, wherein said siRNA molecule comprises at least one modified nucleotide, preferably at least one 2'-O-methyl (2'OMe) nucleotide.

11. A pharmaceutical composition comprising a nucleic acid-lipid particle in accordance with any one of the preceding claims and a pharmaceutically acceptable carrier.

12. A method of introducing an siRNA that silences apoB expression into a cell, said method comprising contacting said cell *in vitro* with a nucleic acid-lipid particle of any one of the preceding claims.

13. The nucleic acid-lipid particle in accordance with any one of the preceding claims, for use in a method of introducing the siRNA into a cell in a mammal wherein the cell is contacted with the particle.

14. The particle of claim 13, wherein said mammal is a human has having a disease or disorder associated with expression or overexpression of ApoB that is silenced by said siRNA.

15. The particle of claim 13, wherein
(a) said human has a disease or disorder selected from the group consisting of: atherosclerosis, angina pectoris, high blood pressure, diabetes, and hypothyroidism; and/or
(b) said human has a disease or disorder involving hypercholesterolemia, wherein serum cholesterol levels are lowered when expression of ApoB is silenced by said siRNA.

16. An isolated siRNA molecule
(a) comprising a sense strand sequence consisting of nucleotides 3-23 of apoB-10048 set forth in Table 4;
(b) that targets the sequence apoB-10048 set forth in Table 4; and/or
(c) that targets the 19 or 21 nucleotides immediately 3' to the dinucleotide sequence of apoB-10048 set forth in Table 4.
